# EUROPEAN PATENT APPLICATION

(11) **EP 3 992 632 A1**
(43) Date of publication of application: **04.05.2022**
(21) Application number: 21190202.8
(22) Date of filing: 27.06.2017
(51) Int. Cl.: G01N 33/68, G01N 33/566, C07K 4/00, C07K 17/00, C12N 5/0783, A61P 35/00

(54) **MHC-E RESTRICTED EPITOPES, BINDING MOLECULES AND RELATED METHODS AND USES**

(30) Priority: 27.06.2016 US 201662355236 P
(62) Divisional of application: 17740170.0
(71) Applicant: Juno Therapeutics, Inc., Seattle, WA 98109 (US)
(72) Inventor: TAREEN, Semih, U., Seattle, WA 98109 (US); SISSONS, James, Seattle, WA 98109 (US); FROHLICH, Mark, Seattle, WA 98109 (US); EBENS, Allen, Seattle, WA 98109 (US)
(74) Representative: Carpmaels & Ransford LLP

(57) **Abstract**

Provided are methods of identifying peptide epitopes of an antigen recognized by the non-classical major histocompatibility complex (MHC) molecule designated MHC-E. In some embodiments, the antigen is a tumor antigen, autoimmune antigen or pathogenic antigen. Also provided are methods of identifying peptide binding molecules that bind to a peptide in the context of an MHC-E molecule. In some embodiments, the peptide binding molecule is a T cell receptor (TCR) or antibody, including antigen-binding fragments thereof and chimeric antigen receptors (CAR) thereof. Also provided are methods of genetically engineering cells containing such MHC-E-restricted peptide binding molecules, and such genetically engineered cells, including compositions thereof and uses in adoptive cell therapy.

## Description

### Cross-Reference to Related Applications

The application claims priority from US provisional patent application 62/355,236, filed June 27, 2016, entitled "MHC-E RESTRICTED EPITOPES, BINDING MOLECULES AND RELATED METHODS AND USES," the contents of which are incorporated by reference in their entirety.

### Incorporation by Reference of Sequence Listing

The present application is being filed with a Sequence Listing in electronic format. The Sequence Listing is provided as a file entitled 735042002240seqlist.txt, created June 27, 2017, which is 39.3 kilobytes in size. The information in electronic format of the Sequence Listing is incorporated by reference in its entirety.

### Field

The present disclosure relates in some aspects to methods of identifying peptide epitopes of an antigen recognized by the non-classical major histocompatibility complex (MHC) molecule designated MHC-E. In some embodiments, the antigen is a tumor antigen, autoimmune antigen or pathogenic antigen. The present disclosure further relates to methods of identifying peptide binding molecules that bind to a peptide in the context of an MHC-E molecule. In some embodiments, the peptide binding molecule is a T cell receptor (TCR) or antibody, including antigen-binding fragments thereof and chimeric antigen receptors (CARs) thereof. The disclosure further relates to methods of genetically engineering cells containing such MHC-E-restricted peptide binding molecules, and such genetically engineered cells, including compositions and uses thereof in adoptive cell therapy.

### Background

Various strategies are available for identifying T cell epitopes of antigens, which can be used to design vaccines or to develop therapeutic binding molecules (e.g. TCRs or antibodies) against such epitopes. In most cases, existing methods used to identify peptide epitopes are limited to identifying canonical peptide epitopes of known antigens, typically based on bioinformatics analyses and/or based on presentation of epitopes on the classical MHC class I and/or MHC class II molecules. Improved strategies are needed to identify unique T cell epitopes, which can increase the targets available for the design and development of TCRs and other binding molecules for the development of therapeutic molecules, including in adoptive immunotherapy, for use in treating cancer, infectious diseases and autoimmune diseases. Provided are methods, cells and compositions that meet such needs.

### Summary

Provided herein are cells comprising an MHC-E molecule, such as MHC-E-expressing cells, and methods using such cells and other reagents for identification of peptide epitopes bound in the context of an MHC-E molecule, e.g. MHC-E restricted epitopes. Also provided are methods for identifying peptide binding molecules, such as TCRs or other recombinant antigen receptors capable of targeting or binding such epitopes. In some embodiments, the provided methods can include contacting such cells with potential peptide epitopes, such as by introduction into such cells of a protein antigen under conditions to elicit processing and presentation of potential peptide epitopes of the antigen on the surface in the context of the MHC molecule. In some embodiments, the peptide epitopes bound or in complex with an MHC-E molecule can be identified or detected. In some embodiments, cells in which peptide epitopes are bound or present in the context of an MHC-E molecule on the surface can be panned or screened against libraries (e.g. phase display libraries or other libraries as described) of candidate binding molecules, such as TCRs or CAR-like TCRs, to identify binding molecules that bind to the peptide epitope in the context of the MHC-E. In some embodiments, the methods can be used to identify antigen-binding domains that can be used, for example, in the context of adoptive cell therapy.

Provided herein is a method of identifying a peptide epitope, the method comprising or involving a) contacting an MHC-E molecule (also called HLA-E) with one or more peptides; and b) detecting or identifying peptide(s) in the context of the MHC-E molecule. In some embodiments of any of the provided methods, the MHC-E molecule is expressed on the surface of a cell.

In some embodiments of any of the provided methods, the methods further include identifying a peptide binding molecule or antigen-binding fragment thereof that binds to at least one of the one or more peptides in the context of the MHC-E molecule. In some embodiments, provided herein is a method of identifying a peptide binding molecule that binds to one or more peptides in the context of an MHC-E molecule, the method comprising or involving a) providing a cell comprising one or more peptides in the context of an MHC-E molecule on the surface of the cell; and b) identifying a peptide binding molecule or antigen-binding fragment thereof that binds to at least one of the one or more peptides in the context of the MHC-E molecule. In some embodiments, providing a cell comprising one or more peptides in the context of an MHC-E molecule comprises contacting the MHC-E molecule on the surface of the cell with the one or more peptides. In some embodiments, the method includes detecting or identifying if the peptide(s) in the context of an MHC-E molecule is present or formed on the surface of the cell prior to providing the cell in a).

In some embodiments of any of the provided methods, the one or more peptides are or include peptides of one or more protein antigens. In some embodiments, the one or more peptides in the context of the MHC-E molecule are identified as peptide epitopes of the one or more protein antigens. In some embodiments, the antigen is a tumor antigen. In some embodiments, the antigen is a pathogenic antigen. In some embodiments, the pathogenic antigen is a bacterial antigen or viral antigen.

In some embodiments of any of the provided methods, the antigen is a viral antigen and the viral antigen is from hepatitis A, hepatitis B, hepatitis C virus (HCV), human papilloma virus (HPV), hepatitis viral infections, Epstein-Barr virus (EBV), human herpes virus 8 (HHV-8), human T-cell leukemia virus-1 (HTLV-1), human T-cell leukemia virus-2 (HTLV-2), or a cytomegalovirus (CMV). In some embodiments, the antigen is an HPV antigen selected from among HPV-16, HPV-18, HPV-31, HPV-33 and HPV-35. In some embodiments, the viral antigen is an EBV antigen selected from among Epstein-Barr nuclear antigen (EBNA)-1, EBNA-2, EBNA-3A, EBNA-3B, EBNA-3C, EBNA-leader protein (EBNA-LP), latent membrane proteins LMP-1, LMP-2A and LMP-2B, EBV-EA, EBV-MA and EBV-VCA. In some embodiments, the viral antigen is an HTLV-antigen that is TAX. In some embodiments, the viral antigen is an HBV antigen that is a hepatitis B core antigen or a hepatitis B envelope antigen.

In some embodiments of any of the provided methods, the antigen is a tumor antigen. In some embodiments, the tumor antigen is selected from among glioma-associated antigen, β-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, Melanin-A/MART-1, WT-1, S-100, MBP, CD63, MUC1 (e.g. MUC1-8), p53, Ras, cyclin B1, HER-2/neu, carcinoembryonic antigen (CEA), gp100, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A11, MAGE-B1, MAGE-B2, MAGE-B3, MAGE-B4, MAGE-C1, BAGE, GAGE-1, GAGE-2, pl5, tyrosinase (e.g. tyrosinase-related protein 1 (TRP-1) or tyrosinase-related protein 2 (TRP-2)), β-catenin, NY-ESO-1, LAGE-1a, PP1, MDM2, MDM4, EGVFvIII, Tax, SSX2, telomerase, TARP, pp65, CDK4, vimentin, S100, eIF-4A1, IFN-inducible p78, melanotransferrin (p97), Uroplakin II, prostate specific antigen (PSA), human kallikrein (huK2), prostate specific membrane antigen (PSM), and prostatic acid phosphatase (PAP), neutrophil elastase, ephrin B2, BA-46, Bcr-abl, E2A-PRL, H4-RET, IGH-IGK, MYL-RAR, Caspase 8, FRa, CD24, CD44, CD133, CD 166, epCAM, CA-125, HE4, Oval, estrogen receptor, progesterone receptor, uPA, PAI-1, CD19, CD20, CD22, ROR1, CD33/IL3Ra, c-Met, PSMA, Glycolipid F77, GD-2, insulin growth factor (IGF)-I, IGF-II, IGF-I receptor and mesothelin.

In some embodiments of any one of the provided methods, the one or more peptides comprise one or more peptides having a length of from or from about 8 to 20 amino acids or 9 to 15 amino acids. In some embodiments, the one or more peptides comprises peptides having a length of or about 9 amino acids, about 10 amino acids, about 11 amino acids, about 12 amino acids, about 13 amino acids, about 14 amino acids or about 15 amino acids. In some embodiments, the one or more peptides comprise overlapping peptides of the antigen or a region of the antigen. In some embodiments, the overlapping peptides, collectively, comprise peptides that represent at least 30%, at least 40%, at least 50%, at least 60%, at least 70%, at least 80%, at least 90% or more of a contiguous sequence of amino acids of the antigen.

In some embodiments of any one of the provided methods, the peptides comprised by the one or more peptides are present in orthogonal pools, each of said orthogonal pools comprising at least two or more different peptides, wherein at least one peptide in each orthogonal pool is the same as a peptide present in at least another orthogonal pool. In some embodiments, a peptide in the context of the MHC-E molecule is detected or identified if the peptide is eluted or extracted from the MHC-E molecule or from a cell expressing the MHC-E molecule in at least two orthogonal pools comprising the same peptide.

In some embodiments of any one of the provided methods, the MHC-E-expressing cell comprises one or a combination of antigens heterologous or exogenous to the cell, in which, in some embodiments, such antigens can be a source of the one or more peptides contacted with the MHC-E. In some embodiments, the one or combination of antigens present in the cell is processed to one or more peptides, thereby contacting the MHC-E molecule with one or more peptides. In some embodiments, the heterologous or exogenous antigens are synthetic nucleic acid molecule(s) that are introduced into the cell. In some embodiments, the cell comprises one or a combination of synthetic nucleic acid molecules comprising one or more coding sequences encoding the one or combination of antigens.

Provided herein is a method of identifying a peptide epitope, the method comprising a) introducing a synthetic nucleic acid molecule or a combination of synthetic nucleic acid molecules comprising one or more coding sequences encoding an antigen or a combination of antigens into a cell expressing an MHC-E molecule; b) incubating the cell under conditions whereby the encoded antigen or antigens are processed to peptides; and c) detecting or identifying peptide(s) of the antigen in the context of the MHC-E molecule. In some embodiments, the method further includes identifying a peptide binding molecule or antigen-binding fragment thereof that binds to at least one of the one or more peptides of the antigen in the context of the MHC-E molecule. Also provided herein is a method of identifying a peptide binding molecule that binds a peptide in the context of an MHC-E molecule, comprising a) introducing a synthetic nucleic acid molecule or a combination of synthetic nucleic acid molecules comprising one or more coding sequences encoding an antigen or combination of antigens into a cell expressing an MHC-E molecule; b) incubating the cell under conditions whereby the encoded antigen or combination of antigens is processed to peptides; and c) identifying a peptide binding molecule or antigen-binding fragment thereof that binds to at least one of the one or more peptides of the antigen in the context of the MHC-E molecule. In some embodiments, the antigen or a combination of antigens can be any as described above, such as a protein antigen, including a tumor antigen or a pathogenic antigen. In some embodiments, the one or more peptides in the context of the MHC-E molecule are identified as peptide epitopes of the one or more protein antigen.

In some embodiments of any one of the provided methods, the synthetic nucleic acid is synthetic DNA. In some embodiments, the synthetic DNA is complementary DNA (cDNA). In some embodiments, the MHC-E-expressing cell comprises a synthetic nucleic acid comprising a coding sequence encoding the antigen. In some embodiments, the MHC-E-expressing cell comprises a combination of synthetic nucleic acids each individually comprising a coding sequence of one of the combination of antigens. In some embodiments, the combination of synthetic nucleic acids comprises one or more nucleic acid molecules of a cDNA library. In some embodiments, the cDNA library is a tumor-derived cDNA library. In some embodiments, the tumor is a melanoma, sarcoma, breast carcinoma, renal carcinoma, lung carcinoma, ovarian carcinoma, prostate carcinoma, colorectal carcinoma, pancreatic carcinoma, squamous tumor of the head and neck, or squamous carcinoma of the lung. In some embodiments of any one of the provided methods, the combination of synthetic nucleic acid molecules and/or combination of encoded antigens is present in orthogonal pools, each of said orthogonal pools comprising at least two or more different synthetic nucleic acid molecules and/or encoded antigens, wherein at least one synthetic nucleic acid molecule and/or encoded antigen is the same in at least two orthogonal pools. In some embodiments, a peptide in the context of an MHC-E molecule is detected or identified if the peptide is eluted or extracted from an MHC-E molecule or from a cell expressing an MHC-E molecule in at least two orthogonal pools comprising the same peptide.

In some embodiments of any one of the provided methods, the method is performed in an array. In some embodiments, the array is an addressable or spatial array.

In some embodiments of any one of the provided methods, the MHC-E molecule is an HLAE^{∗}01:01 or HLA E^{∗}01:03.

In some embodiments of any one of the provided methods, the cell is a primary cell or is a cell line. In some embodiments, the cell is a human cell. In some embodiments, the cell is selected from among a fibroblast, a B cell, a dendritic cell and a macrophage. In some embodiments, the cell is a cell line and the cell line is or is derived from a cell selected from among K562, C1R, KerTr, HCT-15, DLD-1, Daudi, 221, 721.221, BLS-1, BLS-2, JEG-3 and JAR. In some embodiments, the cell is or is derived from a 221 or K562 cell line. In some embodiments of any one of the provided methods, the MHC-E-expressing cell is an artificial antigen presenting cell. In some embodiments, the artificial antigen presenting cell expresses the MHC-E molecule and one or more of a stimulatory or costimulatory molecule(s), an Fc receptor, an adhesion molecule(s) or a cytokine. In some embodiments, the cell is genetically or recombinantly engineered to express the MHC-E molecule.

In some embodiments of any one of the provided methods, the cell is activated or stimulated. In some embodiments, the cell has been or is incubated with an activating or stimulating agent prior to or simultaneously with contacting the MHC-E molecule with the one or more peptides. In some embodiments, the method further comprises incubating the cell with an activating or stimulating agent prior to or simultaneously with contacting the MHC-E molecule with the one or more peptides. In some embodiments, the cell has been or is incubated with an activating or stimulating agent prior to or simultaneously with introducing the synthetic nucleic acid molecule or combination of synthetic nucleic acid molecules into the cell. In some embodiments, the method further comprises incubating the cell with an activating or stimulating agent prior to or simultaneously with introducing the synthetic nucleic acid molecule or combination of synthetic nucleic acid molecules into the cell. In some embodiments, the incubating with the activating or stimulating agent increases expression of the MHC-E molecule on the surface of the cell compared to expression of the MHC-E molecule in the absence of said activating or stimulating. In some embodiments, expression of the MHC-E molecule is increased at least 1.2-fold, 1.5-fold, 2-fold, 3-fold, 4-fold, 5-fold, 6-fold, 7-fold, 8-fold, 9-fold or 10-fold. In some embodiments, the activating or stimulating is effected or carried out in the presence of interferon gamma. In some embodiments, the activating or stimulating comprises incubating the cell with a virus or viral particle. In some embodiments, the virus or viral particle is a cytomegalovirus (CMV).

In some embodiments of any one of the provided methods, the MHC-E-expressing cell is repressed and/or disrupted in a gene encoding a classical MHC class I molecule and/or does not express a classical MHC class I molecule. In some embodiments, the repression is effected by an inhibitory nucleic acid molecule. In some embodiments, the inhibitory nucleic acid molecule comprises an RNA interfering agent. In some embodiments, the inhibitory nucleic acid is or comprises or encodes a small interfering RNA (siRNA), a microRNA-adapted shRNA, a short hairpin RNA (shRNA), a hairpin siRNA, a microRNA (miRNA-precursor) or a microRNA (miRNA). In some embodiment, the MHC-expressing cell is disrupted in a gene encoding a classical MHC class I or comprises a disruption in a gene encoding MHC class I and the disruption of the gene is mediated by a gene editing nuclease, a zinc finger nuclease (ZFN), a clustered regularly interspaced short palindromic nucleic acid (CRISPR) /Cas9, and/or a TAL-effector nuclease (TALEN). In some embodiments, expression of the classical MHC class I molecule in the cell is reduced by at least 50%, 60%, 70%, 80%, 90%, or 95% as compared to the expression in the cell in the absence of the repression or gene disruption. In some embodiments, the classical MHC class I molecule is an HLA-A, HLA-B or HLA-C molecule or is a combination thereof.

In some embodiments of any one of the provided methods, detecting or identifying the one or more peptide(s) in the context of an MHC-E molecule comprises extracting peptides from a lysate of the cell, eluting peptides from the cell surface or isolating the MHC-E molecule or molecules and eluting the one or more peptides from the MHC-E molecule. In some embodiments, the MHC-E is isolated, which comprises or involves solubilizing the cell and selecting the MHC-E molecule by immunoprecipitation or immunoaffinity chromatography. In some embodiments, eluting peptides from an MHC-E molecule is effected in the presence of a mild acid or a diluted acid. In some embodiments of any of the provided methods, the method includes fractionating, separating or purifying the identified or detected peptide(s). In some embodiments of any of the provided methods, the method includes sequencing the identified or detected peptide(s).

In some embodiments of any one of the provided methods, the method further includes determining if the identified or detected peptide(s) elicit an antigen-specific immune response. In embodiments, the antigen-specific immune response is a humoral T cell response. In some embodiments, the antigen-specific immune response is a cytotoxic T lymphocyte response.

In some embodiments of any one of the provided methods, the MHC-expressing cell is a test cell and the method further includes assessment or comparison of one or more peptide epitopes present or found in the context of an MHC-E molecule or other MHC molecule on the surface of a control cell. In some embodiments, the method further comprises detecting or identifying peptides in the context of an MHC-E molecule on the surface of a control cell, said control cell having not been contacted with the one or more peptides, such as one or more peptides of the protein antigen and identifying peptide(s) in the context of an MHC-E molecule that is unique to the test cell compared to the control cell, thereby identifying the one or more peptides of the antigen in the context of an MHC-E molecule.

In some embodiments of any one of the provided methods, the metod is performed in vitro.

In some embodiments of any one of the provided methods, the peptide(s) identified in the context of the MHC-E molecule comprise a length of from or from about 8 to 13 amino acids. In some embodiments, the peptide(s) identified in the context of the MHC-E molecule comprise a length of or about 8 amino acids, 9 amino acids, 10 amino acids or 11 amino acids.

In some embodiments of any one of the provided methods, the peptide(s) in the context of the MHC-E molecule have a binding affinity with an IC50 for the MHC-E molecule of greater than 200 nM, 300 nM, 400 nM, 500 nM, 600 nM, 700 nM, 800 nM, 900 nM, or 1000 nM. In some embodiments, the peptide(s) in the context of the MHC-E molecule have a binding affinity with an IC50 for the MHC-E molecule of less than 500 nm, 400 nM, 300 nM, 200 nM, 100 nM, or 50 nM.

In some embodiments of any one of the provided methods, the peptide(s) in the context of the MHC-E molecule are capable of inducing a CD8+ immune response in a subject. In some embodiments, the peptide(s) in the context of the MHC-E molecule are capable of generating a universal immune response in a majority of subjects in a population. In some embodiments, the universal immune response is elicited in greater than 50%, 60%, 70%, 80%, or 90% of subjects in a population. In some embodiments, the subjects are human subjects.

Provided herein is a peptide epitope identified by any of the provided methods. Provided herein is a stable MHC-E-peptide complex comprising any of the provided peptide epitopes. In some embodiments, the stable MHC-E-peptide complex is present on the surface of a cell. Also provided is a cell expressing any of the provided stable MHC-E peptide complexes.

In some embodiments of any of the provided methods of identifying a peptide binding molecule or antigen-binding fragment thereof that binds a peptide in the context of an MHC-E molecule, the method can include a) assessing binding of a plurality of candidate peptide binding molecules or antigen-binding fragments thereof to any of the provied stable MHC-E-peptide complex; and b) identifying from among the plurality one or more peptide binding molecules that bind to the peptide in the context of an MHC-E molecule. Provided herein is a method of identifying a peptide binding molecule or antigen-binding fragment thereof that binds an MHC-E-restricted peptide, in which the method comprises a) identifying a peptide epitope in accord with any one of the provided methods, b) assessing binding of a plurality of candidate peptide binding molecules or antigen-binding fragments thereof to an MHC-E molecule comprising the peptide of a) bound thereto; and c) identifying from among the plurality one or more peptide binding molecules that bind to the peptide in the context of the MHC-E molecule.

In some embodiments, the plurality of candidate peptide binding molecules comprises one or more T cell receptors (TCRs), antigen-binding fragments of a TCR, antibodies or antigen-binding fragments thereof. In some embodiments, the plurality of candidate peptide binding molecules comprises at least 2, 5, 10, 100, 10³, 10⁴, 10⁵, 10⁶, 10⁷, 10⁸, 10⁹, or more different molecules. In some embodiments, the plurality of candidate peptide binding molecules comprise peptide binding molecules comprising one or more mutations compared to a parent or scaffold peptide binding molecule, wherein individual candidate peptide binding molecules comprise one or more different mutations compared to other candidate peptide binding molecules in the plurality. In some embodiments, the one or more amino acid mutations comprise a mutation or mutations in a complementarity determining region (CDR) or CDRs of the molecule.

In some embodiments of any of the provided methods of identifying a peptide binding molecule or antigen-binding fragment thereof, the candidate peptide binding molecules are obtained from a sample from a subject or a population of subjects. In some embodiments, the subject or population of subjects comprises normal or healthy subjects or diseased subjects. In some embodiments, the diseased subjects are tumor-bearing subjects. In some embodiments, the subject has been vaccinated with the peptide epitope of the antigen. In some embodiments, the subject is a human or non-human mammal, such as a rodent. In some embodiments, the subject is an HLA-transgenic mouse and/or is a human TCR transgenic mouse.

In some embodiments of any one of the provided methods of identifying a peptide binding molecule or antigen-binding fragment thereof, the sample from which the candidate peptide binding molecules are obtained comprises T cells. In some embodiments, the sample comprises peripheral blood mononuclear cells (PBMCs) or tumor-infiltrating lymphocytes (TIL). In some embodiments of any one of the provided methods of identifying a candidate peptide binding molecule or antigen-binding fragment thereof, the candidate peptide binding molecule comprises a T cell receptor (TCR) or an antigen-binding fragment of a TCR. In some embodiments, the antigen-binding fragment of a TCR is a single chain TCR (scTCR).

In some embodiments of any one of the provided methods of identifying a peptide binding molecule or antigen-binding fragment thereof, the sample from which the candidate peptide binding molecules are obtained comprises B cells. In some embodiments, the sample is selected from among blood, bone marrow and spleen and/or the sample comprises PBMCs, splenocytes or bone marrow cells. In some embodiments of any one of the provided methods of identifying a peptide binding molecule or antigen-binding fragment thereof, the candidate peptide binding molecules comprise antibodies or antigen-binding fragments thereof. In some embodiments, the candidate peptide binding molecules comprise IgM-derived antibodies or antigen-binding fragments and/or are naive. In some embodiments, the antibodies or antigen-binding fragments thereof are produced by immunizing a host with an immunogen comprising the MHC-peptide complex. In some embodiments, the candidate peptide binding molecule is a single chain variable fragment (scFv).

In some embodiments of any one of the provided methods of identifying a peptide binding molecule or antigen-binding fragment thereof, the candidate peptide binding molecules are present in a display library. In some embodiments, the display library is selected from among a cell surface display library, a phage display library, a ribosome display library, an mRNA display library, and a dsDNA display library.

In some embodiments, of any one of the provided methods of identifying a peptide binding molecule or antigen-binding fragment thereof, the identified peptide binding molecule exhibits binding affinity for the peptide in the context of an MHC-E molecule with a dissociation constant (K_{D}) of from or from about 10⁻⁵ M to 10⁻¹³ M, 10⁻⁵ M to 10⁻⁹ or 10⁻⁷ M to 10⁻¹². In some embodiments, the identified peptide binding molecule exhibits binding affinity for the peptide in the context of an MHC-E molecule with a K_{D} of less than or less than about 10⁻⁵ M, 10⁻⁶ M, 10⁻⁷ M, 10⁻⁸ M, 10⁻⁹ M, 10⁻¹⁰ M, 10⁻¹¹ M or less.

Provided herein is a peptide binding molecule identified by any of the provided methods. In some embodiments, the peptide binding molecule is a TCR or antigen-binding fragment thereof. In some embodiments, the peptide binding molecule is an antibody or antigen-binding fragment thereof.

Provided herein is a recombinant antigen receptor containing an extracellular antigen-binding domain that is or comprising any one of the provided peptide binding molecules. In some embodiments, the recombinant antigen receptor is a chimeric antigen receptor (CAR).

Provided herein is a genetically engineered cell that comprises any of the provided peptide binding molecules or any of the provided recombinant receptors. In some embodiments, the peptide binding molecule and/or recombinant receptor is expressed on the surface of the cell. In some embodiments, the cell is a T cell. In some embodiments, the cell is a CD8+ T cell. Provided herein is a T cell, such as a CD8+ T cell, that expresses any of the provided peptide binding molecules or any of the provided recombinant receptors containing the peptide binding molecule. In some embodiments, the peptide binding molecule specifically binds a peptide epitope in the context of an MHC-E molecule. In some embodiments, the peptide binding molecule is a T cell receptor (TCR), an antigen-binding fragment of a TCR, an antibody or an antigen-binding fragment of an antibody. In some embodiments, the recombinant antigen receptor is a chimeric antigen receptor (CAR).

Provided herein is a composition, comprising any of the provided peptide binding molecules, any of the provided recombinant receptors or any of the provided genetically engineered cells. In some embodiments, the composition further comprises a pharmaceutically acceptable excipient.

Provided herein is a method of treating a disease or condition by administering to a subject, such as a human subject, any of the provided compositions, e.g. pharmaceutical compositions. In some embodiments, the peptide binding molecule or recombinant receptor comprising the peptide binding molecule of any of the provided compositions binds to an antigen associated with the disease or condition. In some embodiments, the disease or condition is a tumor or a cancer.

### Detailed Description

### I. Non-Classical Major Histocompatibility Complex-E (MHC-E) and Targeting MHC-E Restricted Epitopes

Provided are methods of identifying peptides of an antigen capable of binding to or that do bind to or are recognized by a major histocompatibility complex (MHC)-E molecule. In some embodiments, the peptide is an MHC-E -restricted peptide epitope of an antigen and/or can be displayed or presented in the context of or form a complex with an MHC-E molecule expressed on the surface of a cell. In some embodiments, the peptide is an epitope of a tumor antigen, an autoimmune antigen or a pathogenic antigen (e.g. a bacterial or viral antigen). In some embodiments, the peptide is a universal, supertope and/or non-canonical peptide epitope.

In some embodiments, the MHC-E restricted peptide epitope can be an antigen target of a T cell receptor (TCR) or a TCR-like antibody, including a TCR-like antibody present in a chimeric antigen receptor (CAR), e.g. a TCR-like CAR. In some aspects, also provided are methods of identifying a molecule that binds to an MHC-peptide complex containing an MHC-E-restricted peptide epitope, such as methods for identifying a TCR or antibody molecule that binds to an MHC-peptide complex containing such peptide epitope. In some embodiments, such identified molecules can be used to generate recombinant receptors, including transgenic TCRs or chimeric antibody receptors. Such recombinant receptors, in some aspects, can be used to engineer cells, such as T cells, for adoptive cell therapy.

In general, methods for identifying peptide epitopes in the art have primarily focused on identification of canonical peptide epitopes, which are peptides that exhibit a conserved sequence motif and/or length for the classical MHC molecules, i.e. MHC class I or MHC class II. In some embodiments, various algorithms have been developed to predict if a peptide of interest should bind to a given MHC molecule, but such approaches are biased to classical MHC class I and/or MHC class II molecules. For example, bioinformatics approaches have been utilized to predict classical MHC class I and MHC class II peptide epitopes based on considerations of binding affinity, length and/or the presence of one or more canonical anchor residues (see, e.g., Southwood, et al., J. Immunol. 160:3363 (1998); Honeyman, et al., Nat. Biotechnol. 16:966-969 (1998); Breisie, et al., Bioinformatics 14:121-131 (1998); Larsen et al. (2005) Eur. J. Immunol., 35:2295-2303; Nielsen et al. (2004) Bioinformatics, 20:1388-1397). Further, given interest in identification of peptide epitopes using these approaches, and other approaches for identification of MHC class I and MHC class II epitopes, most canonical peptides epitopes of relevant antigens, e.g. tumor antigens, that can be identified from such approaches have been identified and/or the information obtained from such approaches has been exhausted.

However, in some cases, not all peptide epitopes are presented in the context of classical MHC molecules. In an exemplary study, a shared prostate/colon carcinoma antigen was found to be presented on a non-classical MHC I-like molecule with limited or no polymorphism (Housseau et al. (1999) J of Immunol., 163: 6330-6337). MHC-E (also called HLA-E) is a nonclassical MHC I-like molecule that can be overexpressed on tumor cells. In some cases, MHC-E has been shown to bind to viral peptides, bacterial peptides and peptides derived from cellular-associated antigens. In some aspects, CD8+ T cell can recognize peptides in the context of an MHC-E molecule, i.e., MHC-E-peptide complexes, through their αβ TCR to induce MHC-E-restricted CD8+ T cell responses (Pietra et al. (2010) Journal of Biomedicine and Biotechnology, 1-8). In some aspects, MHC-E can bind to peptides also recognized by MHC class la, albeit with lower affinity (Pietra et al. (2010)). In some aspects, MHC-E also can present noncanonical or nonconventional peptides and/or supertopes (Pietra et al. (2010)).

Since immune reactivity, including antitumor response, is, in many aspects, not limited by processing and presentation of classical MHC class I and MHC class I epitopes, there is a need for other approaches to identify MHC-restricted peptide epitopes, including epitopes able to elicit an immune response to an antigen of interest, such as a tumor antigen. The provided methods offer an alternative approach for epitope identification, including identification of nonconventional peptide epitopes that are not necessarily restricted to classical MHC molecules. In particular, the provided methods use MHC-E to present non-canonical peptide epitopes. In some aspects, it is believed that such epitopes can include tumor antigens.

In general, an MHC, including an MHC-E, contains a polymorphic peptide binding site or binding groove that can, in some cases, complex with peptide antigens of polypeptides, including peptide antigens processed by the cell machinery. In general, an MHC molecule can include an effective portion of an MHC that contains an antigen binding site or sites for binding a peptide and the sequences necessary for recognition by the appropriate binding molecule, such as TCR or other peptide binding molecule. In some cases, MHC molecules can be displayed or expressed on the cell surface, including as a complex with peptide (MHC-peptide complex) for presentation of an antigen in a conformation recognizable by TCRs on T cells or other peptide binding molecules. Generally, MHC molecules are encoded by a group of linked loci, which are collectively termed H-2 in the mouse and human leukocyte antigen (HLA) in humans. Hence, typically human MHC can also be referred to as human leukocyte antigen (HLA).

MHC molecules can include MHC class I and class II molecules. Among MHC class I molecules are classical and non-classical MHC molecules, which differ in their polymorphism. For example, class I molecules include highly polymorphic classical MHC class Ia or HLA class Ia molecules (e.g. HLA-A: 3129 alleles, 2245 proteins; HLA-B: 39779 alleles, 2938 proteins; and HLA-C (or HLA-CW): 2740 alleles, 1941 proteins) and the less polymorphic non-classical MHC class-Ib or HLA-Ib molecules (HLA-E:17 alleles, 6 proteins; HLA-F: 22 alleles, 4 proteins; and HLA-G: 50 alleles, 16 proteins), based on information published in August 2015 in EBML-EBI Website at www.ebi.ac.uk/imgt/hla/stats.html. Thus, MHC-E (or HLA-E) is a non-classical MHC class I molecule.

Generally, MHC class I molecules, including classical (i.e. MHC class Ia) and non-classical (i.e. MHC class Ib, e.g. MHC-E), are heterodimers having a membrane spanning α chain, in some cases with three α domains, and a non-covalently associated β2 microglobulin. In some embodiments, MHC class I molecules deliver peptides originating in the cytosol to the cell surface, where a peptide:MHC complex is recognized by T cells, such as generally CD8⁺ T cells. In contrast, MHC class II molecules are generally composed of two transmembrane glycoproteins, α and β, both of which typically span the membrane, and generally deliver peptides originating in the vesicular system to the cell surface, where they are typically recognized by CD4⁺ T cells, but, in some cases, CD8+ T cells.

In some embodiments, an MHC-E molecule (or can be designated as an HLA-E molecule) is a non-classical MHC class I molecule that is encoded by the HLA-E gene in humans or an ortholog or homolog in another species. For example, the homolog in mice is called Qa-1b. The HLA-E gene is ubiquitously expressed throughout tissues, although, in some cases, at much lower levels than the other nonclassical MHC Class I genes, HLA-G and HLA-F (see Strong et al., J Biol Chem. 2003 Feb 14;278(7):5082-90). MHC-E exhibits limited polymorphism, especially compared to MHC class I molecules; there are only two known alleles of MHC-E in human, alleles E^{∗}0101 and E^{∗}0103, which are found at approximately equal frequencies throughout diverse populations. Generally, MHC-E is structurally similar to other MHC class I molecules, and exists as a heterodimer containing an α heavy chain and a light chain (also called β-2 microglobulin). MHC-E molecules are known to bind to peptides (e.g. nonameric peptides) derived from signal peptides of classical MHC class I molecules, which stabilizes MHC-E on the surface of cells, and, in some cases, can be recognized by NK cells to modulate NK cell activation. For example, in some aspects, MHC-E can bind the inhibitory NK cell receptor CD94/NKG2A to inhibit activity of NK cells. In some cases, MHC-E complexed with peptides also can interact with TCRs expressed on CD8+ cells (Pietra et al. (2010) Journal of Biomedicine and Biotechnology, Article ID 907092).

In some embodiments, the provided method involves preparing cells, such as MHC-E-expressing cells, that are capable of displaying one or more peptide epitopes on the cell surface in the context of the MHC-E molecule. In some embodiments, the method results in the presentation or display of peptide epitopes, including, in some cases, non-canonical peptide epitopes on the cell surface. In some embodiments, such cells are prepared by contacting, exposing and/or incubating the MHC-E-expressing cell with one or more peptides derived from a target antigen, such as a tumor antigen or pathogenic antigen, including a bacterial or viral antigen. In some embodiments, the method includes identifying or detecting a peptide epitope bound in the context of an MHC-E molecule. The method can include one or more of identifying and/or isolating the peptide epitope, detecting and/or identifying a peptide epitope that exhibits an MHC-E-restricted immune response (e.g. CTL immune response), and/or identifying or selecting a binding molecule, e.g. a TCR or TCR-like antibody or antigen-binding fragments thereof, that binds to such MHC-E-restricted peptide epitope. For example, in some embodiments, the methods include identifying or determining the sequence of the presented or displayed peptide, assessing the affinity of the presented or displayed peptide and/or assessing or determining immune reactivity (e.g. CTL response) of the presented or displayed peptide. In some embodiments, the peptide epitope is a universal, supertope and/or non-canonical (nonconventional) peptide epitope.

In some embodiments, the antigen is a tumor antigen or is a tumor-related viral antigen, such as an oncogenic viral antigen. In some aspects, the provided embodiments are based on observations that MHC-E represents a tumor-specific target for therapeutic interventions. In general, MHC-E is expressed in most all human tissues similar to classical MHC class la, although at much lower levels than classical MHC class Ia molecules. In some cases, however, high levels of MHC-E has been detected in neoplastic cells, such as in tumors, including fresh lymphomas, ovarian carcinomas, gliomas, colon cancer, melanomas and others, and, in some cases, has been found to correlate with a worse clinical outcome (Pietra et al. 2010; Kruijf et al. (2010) J. Immunol., 185:7452-7459. In contrast, classical MHC class Ia is downregulated in many malignant cells, including in many tumors, which may limit the ability to target MHC-class la-restricted peptide epitopes in tumor therapies. It is contemplated herein that the high level expression of MHC-E in tumors, and other inflammatory environments, renders MHC-E an attractive target for tumor-targeted therapies and other disease-related therapies in which MHC-E is highly expressed, including stress-related and pathogen-associated conditions.

In some embodiments, an advantage of targeting MHC-E-restricted peptide epitopes, as opposed to other classical MHC class I-restricted epitopes, is that MHC-E is the least polymorphic of all MHC class I molecules. For example, MHC alleles E^{∗}0101 (HLA-E^{107R}) and E^{∗}0103 (HLA-E^{107G}) are found at approximately equal frequencies throughout diverse populations, and differ by only one amino acid at position 107 (Pietra et al. (2010) Journal of Biomedicine and Biotechnology)

In some cases, a problem of many TCR-related therapies, including therapies with TCR-like antibodies, is that the therapeutic must be MHC (or HLA)- matched to the subject, since classical MHC molecules are highly polymorphic. For example, world-wide, the most frequent MHC allele, HLA-A^{∗}2402, is only present in 18.8% of the populations (Solberg et al., (2008) Hum Immunol. 2008 Jul; 69(7):443-6). Although the frequency of classical MHC molecules can be higher in specific populations, it can be difficult to generate a universal therapeutic that is MHC-class la-restricted. On the other hand, MHC-E molecules are far less diverse among populations of subjects, since only two HLA-E alleles exist that are, in most cases, functionally identical.

In some embodiments, the identified peptide epitope, can be a universal peptide epitope and/or elicit a universal T cell response. In some embodiments, a universal peptide epitope is a peptide that is recognized and displayed by MHC molecule or molecules, such as an MHC-E molecule, including alleles, in a manner that is able to elicit an immune response, such as a CD8+ immune response, in a majority of subjects of the same species. In some cases, a universal peptide epitope can elicit such immune response in greater than 50% of subjects within a population of a particular species, such as mammalian or human species, such as generally in greater than 60%, 70%, 80%, 90% or more of a population of subjects exposed to such peptide antigen. For example, in some cases, a peptide having a universal epitope sequence can induce an immune response, e.g. a cytotoxic T cell response, from samples from a majority of subjects of the same species that are genetically divergent in the MHC loci, e.g., differ in the MHC-E allele. In some cases, a universal epitope is a supertope.

In some embodiments, the identified peptide epitope can be a supertope and/or elicit a supertope response. In some embodiments, a supertope is a peptide epitope that, in some cases, can be a highly promiscuous epitope that represents a common epitope or peptide recognized or presented by MHC of a population of subjects. In some embodiments, a supertope or supertope response is associated with recognition by an MHC-E molecule, such as an HLA-E, which is capable of recognizing a broader peptide repertoire than classical MHC, such as MHC class la, molecules. For other MHC molecules, a supertope can be a peptide epitope that represents a common epitope or peptide recognized by different MHC alleles of the same MHC (or HLA) supertype, such as due to primary or tertiary structural similarity and/or an overlapping or shared peptide binding motif. In some embodiments, a supertope can elicit a CD8+ T cell response. In some embodiments, the identified peptide epitope is one that can elicit an immune response, e.g. a CD8+ immune response, in greater than 50% of subjects within a population of a particular species, such as mammalian or human species, such as generally in greater than 60%, 70%, 80%, 90% or more of the population of subjects exposed to such peptide antigen. In some cases, the population can be genetically divergent at the MHC loci.

In some embodiments, the identified peptide epitope can be a non-canonical (or nonconventional) epitope and/or elicit a non-canonical response. In some cases, a nonconventional epitope or non-canonical epitope is a peptide epitope displayed or presented on an MHC molecule that may not exhibit a conserved sequence motif for MHC binding, for example, due to the absence of one or more anchor residues, may exhibit a low or medium affinity binding interaction to MHC molecules and/or may be of a longer length as compared to conventional or canonical peptide epitopes. Hence, a non-canonical epitope may be one that does not exhibit a canonical sequence motif, length and/or binding affinity for MHC interaction. In general, a canonical peptide epitope is generally recognized by classical MHC class I or MHC class II peptide epitopes, in which recognition is based on the presence of a conserved residue(s), length and/or binding affinity. In some cases, a non-canonical epitope is an epitope that is displayed or presented on a non-classical MHC molecule, such as an MHC-E molecule.

In some embodiments, the methods permit identification of epitopes that are peptides bound or recognized in the context of an MHC-E molecule and that have a length of 8 to 13 amino acids, such as generally at least or about at least 8 amino acids, 9 amino acids, 10 amino acids, 11 amino acids or 12 amino acids. In some embodiments, the peptide bound or recognized in the context of an MHC-E molecule is a nonamer, i.e. 9 amino acids in length.

In some embodiments, the methods permit the identification of a peptide epitope with a binding affinity, such as determined by maximal inhibitory concentration (IC50), for a bound MHC that is of high affinity, intermediate affinity, or, in some cases, low affinity. In some embodiments, the relative binding capacity or affinity of a peptide can be assessed by measuring IC50 for binding by determining the concentration necessary to reduce binding of a labelled reporter peptide by 50%. In some embodiments, a non-canonical peptide can include peptides that exhibit a lower affinity for an MHC molecule than is otherwise observed for canonical peptide epitopes. In some embodiments, a peptide epitope identified by the provided methods as a binding affinity with an IC₅₀ of between or about 200 nM and 5000 nM, such as generally greater than 200 nM and less than 4000 nM, 2000 nM, 1000 nM or 500 nM.

In some cases, the provided methods can be used to identify peptide epitopes associated with a disease or condition in which such peptide epitopes are naturally processed or presented. In some aspects, such peptide epitopes can include or be a universal, supertope or non-canonical peptide epitopes. In some aspects, the presence of immunoregulatory mechanisms and/or changes in immune regulation associated with pathogenic states may support processing or presentation of universal, supertope and/or non-canonical epitopes over conventional epitopes in a particular disease or condition. In some embodiments, the methods can be used to identify peptide epitopes associated with tumors or cancers. In some embodiments, the methods can be used to identify peptide epitopes related to virally-associated cancers.

In some embodiments, the methods can be used to identify MHC-E restricted peptides associated with a pathogenic or diseased state. In some embodiments, the methods can be used to identify MHC E-restricted peptides derived from a tumor-associated antigen (TAA). In some embodiments, the methods can be used to identify MHC-E restricted peptide derived from a viral antigen, including an antigen found in a viral-associated cancer. In some embodiments, the MHC-E-peptide complex is recognized by a CD8+ T cell and/or be associated with a CTL response. In some embodiments, identified MHC E -restricted peptides or epitopes can be used as targets in the development or identification of molecules that specifically recognize the peptide target in the context of MHC-E.

In some embodiments, an identified peptide or epitope elicits a T cell response. In some embodiments, the identified peptide or epitope that elicits a T cell response is a universal, supertope, and/or non-canonical peptide or epitope. In some embodiments, the T cell response or responses is elicited in the context of a cell population containing CD8+ cells that has been exposed or contacted with the peptide. In a particular example, peripheral blood mononuclear cells (PBMCs) obtained from a subject, such as a subject with a tumor or cancer, can be stimulated with the peptide and assessed for activation. Various assays to assess activation of T cells are well known in the art. In some embodiments, the identified epitope can activate a CD8+ T cell response. In one embodiment, CD8+ T cell responses can be assessed by monitoring CTL reactivity using assays that include, but are not limited to, target cell lysis via ⁵¹Cr release or detection of interferon gamma release, such as by enzyme-linked immunosorbent spot assay (ELISA), intracellular cytokine staining or ELISPOT.

In some embodiments, methods also are provided for selecting or screening for a binding molecule that binds to a particular MHC-E-restricted peptide. In some embodiments, the MHC-E-restricted peptide is a peptide epitope identified by any of the above methods, which, in some cases, can be a universal, supertope and/or noncanonical peptide epitope. In some embodiments, the binding molecule is a TCR or antigen-binding portion thereof. In some embodiments, the binding molecule is an antibody, such as a TCR-like antibody, or antigen-binding portion thereof. In some embodiments, the methods including contacting a binding molecule (e.g. a TCR, antibody or antigen-binding portions thereof) or a library of binding molecules (e.g. a library of TCRs, antibodies or antigen-binding portions thereof) with an MHC-E-restricted epitope and identifying or selecting molecules that specifically bind such an epitope. In some embodiments, the methods can be performed *in vivo, ex vivo* or *in vitro.* In some embodiments, a library or collection containing a plurality of different binding molecules, such as a plurality of different TCRs or a plurality of different antibodies, can be screened or assessed for binding to an MHC-E-restricted epitope. In some embodiments, such as for selecting an antibody molecule that specifically binds an MHC-E-restricted peptide, hybridoma methods can be employed.

In some embodiments, the methods can be employed to identify a MHC-E-peptide binding molecule, such as a TCR or antibody that exhibits cross-reactive and/or promiscuous binding between and among both MHC-E alleles. In some embodiments, the MHC-E-peptide binding molecule, such as a TCR or antibody, specifically binds or recognizes a peptide presented in the context of MHC-E alleles, such as the HLA-E^{∗}0101 and/or HLA-E^{∗}0103 alleles.

In some embodiments, an identified antibody, such as a TCR-like antibody, can be used to produce or generate chimeric antigen receptors (CARs) containing a non-TCR antibody that specifically binds to a MHC-peptide complex.

In some embodiments, the methods of identifying an MHC-E-peptide binding molecule, such as a TCR or TCR-like antibody or TCR-like CAR, can be used to engineer cells expressing or containing an MHC-E-peptide binding molecule. In some embodiments, a cell or engineered cell is a T cell. In some embodiments, the T cell is a CD8+ T cell. In some embodiments, the MHC-E-peptide binding molecule recognizes peptides in the context of an MHC-E molecule, i.e., an MHC-E peptide complex. In some embodiments, also provided is a composition of engineered CD8+ T cells expressing or containing the MHC-E-binding molecule, such as a TCR, TCR-like antibody or TCR-like CAR, for recognition of a peptide presented in the context of MHC-E molecule. In any of such embodiments, the cells can be used in methods of adoptive cell therapy.

### II. Methods of Identifying MHC-E Restricted T cell Epitopes

Provided in some aspects are methods of identifying or detecting a peptide epitope in the context of an MHC-E molecule, including peptide epitopes derived from a target protein antigen, such as a tumor antigen. In some embodiments, the identified or detected peptide epitope can be a universal, supertope and/or non-canonical peptide epitope. In some embodiments, the target antigen is a tumor antigen. In some embodiments, the target antigen is a viral antigen. In some embodiments, the target antigen is an oncogenic viral tumor antigen.

In some embodiments, the methods involve contacting, exposing and/or incubating an MHC-E molecule with one or more peptides derived from the target antigen, such as a tumor or viral antigen. In some embodiments, the MHC-E molecule is expressed on the surface of a cell. In some embodiments, the contacting or exposing of an MHC-E molecule with one or more peptides can be extracellular or can be intracellular. In some embodiments, the peptide or peptides are derived from a pool of peptides, such as overlapping peptides present in the target antigen, which are incubated with the cell. In some embodiments, the peptide or peptides are derived from a synthetic nucleic acid molecule, e.g. cDNA molecule, which is transferred into the cell for antigen expression of a heterologous or exogenous target antigen under conditions in which the expressed target antigen is processed to produce peptide epitopes of the target antigen displayed on a major histocompatibility (MHC) molecule of the cell to generate an MHC-peptide complex.

In some embodiments, the provided methods include identifying or detecting a peptide epitope, recognized or bound in the context of a non-classical MHC class Ib, such as in the context of an MHC-E (e.g. HLA-E). In some embodiments, the peptide bound or recognized in the context of an MHC-E molecule is a universal, supertope and/or non-canonical peptide epitope. In some embodiments, the peptide epitope identified or detected by the instant method are not peptides presented, recognized or bound in the context of a classical MHC class Ia (e.g. HLA-A, B or C).

In some embodiments, after identifying or detecting a peptide epitope that is recognized or bound to an MHC-E molecule, the method further includes isolating or purifying the peptide bound or recognized in the context of an MHC-E molecule. In some embodiments, the provided methods include assessing or determining an immune response, such as a cytotoxic T lymphocyte (CTL) response elicited by a peptide bound in the context of an MHC-E molecule. In some embodiments, peptide epitopes that elicit an immune response in the context of an MHC-E molecule, such as elicit a CTL response, are identified, isolated and/or purified.

In some embodiments, provided are methods that include contacting, exposing and/or incubating an MHC-E molecule (e.g. expressed on the surface of a cell) with one or more peptides derived from the target antigen (e.g. pool of overlapping peptides or peptides derived from a exogenous or heterologous antigen expressed in the cell), and detecting or identifying peptides epitopes, such as peptide epitopes of a tumor antigen, that elicit an immune response (e.g. CTL response) in the context of an MHC-E molecule. In some embodiments, MHC-E expressing cells that have been contacted, exposed and/or incubated with one or more peptides derived from a target antigen (e.g. tumor or viral antigen) can be assessed for a CTL immune response. In some embodiments, the methods further include isolating or purifying a peptide that, in the context of an MHC-E molecule, elicits an immune response, e.g. CTL response.

In some embodiments, the method includes determining the sequence of the peptide bound or recognized by the MHC-E molecule and/or that elicits an immune response, e.g. CTL immune response, in the context of an MHC-E molecule.

In some embodiments, a binding molecule, such as a TCR, antibody or antigen-binding fragments thereof, that recognize, e.g. specifically bind, a peptide recognized or bound to an MHC-E molecule can be identified.

### A. MHC-E Expressing Cells

In some embodiments, cells expressing an MHC-E molecule are used in the provided methods. An MHC-E expressing cell may be any cell that expresses MHC-E on the cell surface. In some embodiments, the cell is a primary cell. In some embodiments, the cell is a cell line. In some embodiments, the cell is a human cell.

In some embodiments, the MHC-E expressing cells are cells or cell lines that contain endogenous expression of MHC-E on the cell surface. MHC-E expressing cells are known in the art (see e.g. Grimsley et al. (2002) Tissue Antigens, 60:206-212; Monaco et al. (2008) J. Immunol., 181:5442-5450). In some cases, it is well within the level of a skilled artisan to perform standard typing of cells to determine or confirm the MHC (e.g. HLA) genotype, such as by using sequence-based typing (SBT), PCR-SSP or other typing method (see e.g. Grimsley et al. (2002); Adams et al., (2004) J. Transl. Med., 2:30; Smith (2012) Methods Mol Biol., 882:67-86).

In general, MHC-E molecules are commonly expressed on endothelial cells, fibroblasts and immune cells (e.g. B cells, T cells, NK cells, monocytes, macrophages, dendritic cells). In some embodiments, cells can be induced to express an MHC-E molecule. In some aspects, infection of cells with CMV induces expression of MHC-E on the surface of such cells, for example, up to 6 hours, 8 hours, 12 hours or 24 hours after contacting or introducing the cells with CMV (e.g. Rolle et al. (2014) J. Clin. Invest., 124:5305-5316). In some cases, a cell can be stimulated or activated to upregulate expression of MHC-E on the cell surface, such as by using interferon gamma or other stimulating agent. Exemplary stimulating or activating agents include, but are not limited to, one or more of IFNγ, TNEα, IL1β, mitomycin C, phorbol myristate acetate (PMA) or ionomycin, such as generally IFNγ. In some embodiments, cell surface expression of an MHC-E molecule can be induced or upregulated by incubation of cells, such as a culture of cells, at a confluency of from or from about 30% to 60%, for example about 40%, in the presence of a stimulating amount of a stimulating agent, for example interferon gamma, generally 50 U/mL to 500 U/mL, such as generally at least 100 U/mL or at least 200 U/mL. In some embodiments, a cell can be incubated with the stimulating agent, such as interferon gamma, for between or between about 10 minutes and 96 hours, such as for at least 30 minutes, 1 hour, 6 hours, 12 hours, 24 hours, 48 hours or 72 hours.

In some embodiments, the cell is a cell line, such as a cell line available from private and commercial sources, such as American Type Culture Collection (ATCC); National Institute of Medical Sciences (NGIMS); ASHI Repository; the European Collection of Cell Cultures (ECACC); or the International Histocompatibility Working (IHW) Group Cell and DNA bank. In some cases, cell lines are commercially available.

In some embodiments, the cell is an EBV-immortalized B cell line. Exemplary of such cell lines include, but are not limited to CJO (IHW No. 9243), LBF, LG2, Molt4 (IHW No. 9226), BSM (IHW No. 9032), JVM (IHW No. 9039), MGAR (IHW No. 9014), WT51 (IHW No. 9029), JY, RM-13 and LKT-3. See e.g., Monaco et al. (2008) J. Immunol., 181:5442-5450.

In some embodiments, the cell is engineered or transfected to express an MHC-E molecule. In some embodiments, cell lines can be prepared by genetically modifying a parental cells line. In some embodiments, the cells are normally deficient in MHC-E and are engineered to express an MHC-E molecule. In some embodiments, the cell can be stable cell line or can be transiently transfected. In some embodiments, the cells are deficient in one or both of MHC class Ia molecules (e.g. HLA-A, B and C) or MHC class II molecules.

In some embodiments, a cell that expresses MHC-E, such as a cell that is engineered or transfected to express an MHC-E molecule, is one that lacks or is made to lack expression of another MHC class I molecule, generally an HLA-A, B or C molecule. In some embodiments, a cell that expresses MHC-E or that is engineered to express MHC-E lacks endogenous expression of another MHC class I molecule, generally an HLA-A, B or C molecule. Exemplary cell lines that lack endogenous expression of another MHC class I molecule, such as an HLA-A, B or C molecule, can include, but are not limited to, a cell line set forth in Table 1. In some embodiments, the engineered cell is or is derived from a 221 or K562 cell line. For example, the LCL 721.221 cell line is a mutant cell line derived from LCL 721 by immunoselection following gamma-ray mutagenesis to eliminate expression of the MHC class I HLA-A- B- and C- alpha chains (Shimizu et al. (1988) PNAS) 85:227-231). Exemplary HLA-E transfected cells include, but are not limited to, K562 HLA-E B5 (see e.g., Ulbrecht et al. (2000) Journal of Immunol., 164:5019-5022) and RMA-S-EM (Borrego et al. (1998) J. Exp. Med., 187:813).

| **Table 1: Cell Lines For Engineering MHC-E** | | | | |
|---|---|---|---|---|
| **Cell Line** | **ATCC No. or Source** | **Organism** | **Cell/Tissue Type** | **MHC Deficient** |
| K562 | CCL-243 | Human | Erythromyeloid | Class I & II Deficient |
| C1R | CRL-1993 | Human | B lymphoblast | Class I Deficient |
| KerTr | CRL-2309 | Human | Skin | Class I Deficient |
| HCT-15 | CCL-225 | Human | Colon cancer | Class I Deficient |
| DLD-1 | CCL-221 | Human | Colon cancer | Class I Deficient |
| Daudi | CCL-213 | Human | Lymphoma | Class I Deficient |
| LCL 721.221 | CRL-1855 | Human | B lymphoblastoid | Class I Deficient |
| BLS-1 | | Human | B lymphocyte | Class I Deficient |
| BLS-2 | | Human | B lymphocyte | Class I Deficient |
| JEG-3 | HTB-36 | Human | Choriocarcinoma | Class I Deficient |
| JAR | HTB-144 | Human | Choriocarcinoma | Class I Deficient |
| RMA-S | Ljunggren, 1985. J. Exp. Med. 162:1745 | Mouse | | Class I Deficient |

In some embodiments, a cell expressing an MHC-E, such as a cell that is engineered or transfected to express an MHC-E molecule, is one in which another MHC class I molecule, such as an HLA-A, B or C, may be normally expressed, but in which the expression, activity and/or function of a gene encoding such an MHC class I, such as an HLA A, B or C gene, is repressed or disrupted. Exemplary methods for repressing or disrupting a gene, such as an HLA A, B or C gene, are described below.

In some embodiments, a nucleic acid molecule encoding MHC-E can be introduced into a cell, such as a cell that does not normally express MHC-E, does not express MHC-E on the cell surface and/or that may express MHC-E at a low level, for example any of the cells described above. Typically, the cells are genetically engineered using recombinant DNA techniques. The sequence of an exemplary MHC-E (e.g. allele E^{∗}01:01) is set forth in SEQ ID NO: 1 (GenBank No. AAH02578.1 or UniProt No. P13747.3) and is encoded by a sequence of nucleotides set forth in SEQ ID NO:2 (GenBank No. BC002578). The sequence of an exemplary MHC-E (e.g. allele E^{∗}0103) is set forth in SEQ ID NO: 3 (GenBank No. NP_005507) and is encoded by a sequence of nucleotides set forth in SEQ ID NO:4 (GenBank No. NM_005516.5). In some embodiments, the MHC-E heavy chain gene can be isolated from the blood of a subject, such as amplified by PCR using degenerate primers. In some embodiments, the MHC-E heavy chain gene can be generated synthetically, such as based on known sequence information readily available for MHC alleles.

In some embodiments, the sequence can be cloned into an expression vector. In some embodiments, methods for generating an expression vector can be by standard recombinant DNA techniques. Construction of expression vectors and recombinant production from the appropriate DNA sequences are performed by methods known in the art. For example, standard techniques are used for DNA and RNA isolation, amplification, and cloning. Generally enzymatic reactions involving DNA ligase, DNA polymerase and restriction endonucleases are performed according to the manufacturer's specifications. These techniques and various other techniques are generally performed according to Sambrook et al., Molecular Cloning -- A Laboratory Manual, Cold Spring Harbor Laboratory, Cold Spring Harbor, N.Y., 1989. Such procedures are well known in the art.

In some embodiments, restriction sites can be included in the gene sequence to aid insertion into expression vectors and manipulation of the gene sequence. The sequence can be inserted into an expression vector. In some embodiments, the expression vector is a mammalian expression vector or a viral expression vector. In some embodiments, the expression vector can be a pCR2.1, pLNCx, pcDNA, pEAK, pBluescript or pUC18 vector. In some embodiments, the expression vector is a retroviral expression vector, such as a lentiviral expression vector.

In some embodiments, nucleic acid, such as vectors, including particular HLA alleles are available from commercial or private sources. For example, MHC-E, i.e. HLA-E, expression plasmids, including mammalian and lentiviral expression vectors, are available from commercial sources, such as from Sino Biological, Inc. (see e.g., Catalog No. HG13375), GeneCopoeia (Cat. No. LPP-Q0324) and others.

In some embodiments, the expression vector encoding the MHC-E can be introduced into a host cell for expression. Generally, transfection or transformation is done using standard techniques appropriate to such cells depending on the host cell used. Common transfection methods include, but are not limited to, lipofection, microinjection, electroporation, methods using calcium phosphate or viral-based delivery methods. For example, in some embodiments, cells can be transduced using lentiviral or retroviral-based vectors. In some embodiments, the transformed cells can be cultured under conditions favoring expression of the MHC-E molecule and expression on the cell surface.

In some embodiments, expression of MHC-E on the surface of the cell can be stabilized by the addition of a peptide derived from an MHC class Ia molecule. In some embodiments, the peptide is a peptide of an MHC class Ia molecule leader sequence. For example, in some cases, expression of MHC-E on the surface of cells is increased in the presence of a peptide derived from an MHC class Ia leader sequence for assembly of the MHC-E complex (Lee et al. (1998) Journal of Immunology, 160:4951-4960; Braud et al. (1998) Current Biology, 8:1-10). In some cases, the peptide can be added exogenously and incubated with cells, in which case a transporter associated with antigen processing (TAP) may not be necessary. In some cases, the peptide is processed by the cell where it can be delivered into the endoplasmic reticulum (ER) by TAP for binding to a nascent MHC-E molecule. Hence, in some embodiments, the cell can contain a TAP. In some embodiments, the cell can be TAP-deficient.

In some embodiments, an exogenous peptide corresponding to a leader sequence of an MHC class Ia molecule is incubated with a cell transfected with MHC-E. In some embodiments, the incubation occurs at a temperature of from or from about 22 °C to 30 °C, such as generally 26 °C ± 3 °C. In some embodiments, the peptide is a nonamer. In some embodiments, the peptide is derived from a leader sequence of an MHC class Ia molecule in which a methionine is present at position 2 and a leucine is present at the carboxyl terminal position of the nonamer. In some embodiments, the peptide is derived from a leader sequence of an MHC class Ia molecule that is an HLA-A, HLA-B, HLA-C or an HLA-G. In some embodiments, the peptide is derived from a leader sequence of an MHC class Ia molecule that is HLA-A^{∗}0101, HLA-A^{∗}0201, HLA-A^{∗}0211, HLA-A^{∗}0301, HLA-A^{∗}2403, HLA-A^{∗}2501, HLA-A^{∗}3601, HLA-A^{∗}0702, HLA-A^{∗}0801, HLA-B^{∗}6501, HLA-Cw^{∗}0401, HLA-Cw^{∗}1502, HLA-G. In some embodiments, the peptide has a sequence of amino acids corresponding to amino acids 3-11 of an MHC class Ia leader sequence. In some embodiments, the peptide has a sequence of amino acids set forth in any of SEQ ID NOS: 5-9.

In some embodiments, a cell is co-transfected with an MHC class Ia molecule and an MHC-E. In some embodiments, the MHC class Ia molecule is one in which, within residues 3-11 of its leader sequence, a methionine is present at position 2 and a leucine is present at the carboxyl terminal position. In some embodiments, the MHC class Ia molecule is an HLA-A, HLA-B, HLA-C or an HLA-G. In some embodiments, the MHC class Ia molecule is HLA-A^{∗}0101, HLA-A^{∗}0201, HLA-A^{∗}0211, HLA-A^{∗}0301, HLA-A^{∗}2403, HLA-A^{∗}2501, HLA-A^{∗}3601, HLA-A^{∗}0702, HLA-A^{∗}0801, HLA-B^{∗}6501, HLA-Cw^{∗}0401, HLA-Cw^{∗}1502, or HLA-G.

In some embodiments, a hybrid MHC-E gene containing a leader sequence of an MHC class Ia molecule fused to the HLA-E mature protein can be transfected into a cell. In some embodiments, the hybrid molecule contains a promoter and leader sequence of an MHC class Ia molecule fused to the HLA-E mature protein. In some embodiments, the MHC class Ia molecule is one in which, within residues 3-11 of its leader sequence, a methionine is present at position 2 and a leucine is present at the carboxyl terminal position. In some embodiments, the leader sequence can be derived from an MHC class Ia molecule that is an HLA-A, HLA-B, HLA-C or an HLA-G. In some embodiments, the leader sequence is from an MHC class I molecule that is HLA-A^{∗}0101, HLA-A^{∗}0201, HLA-A^{∗}0211, HLA-A^{∗}0301, HLA-A^{∗}2403, HLA-A^{∗}2501, HLA-A^{∗}3601, HLA-A^{∗}0702, HLA-A^{∗}0801, HLA-B^{∗}6501, HLA-Cw^{∗}0401, HLA-Cw^{∗}1502, or HLA-G. For example, exemplary of such a hybrid is an AEH hybrid gene containing the HLA-A2 promoter and signal sequence and the HLA-E mature protein sequence, which, in some cases, can result in a mature protein identical to that encoded by the HLA-E gene but that can be stably expressed on the cell surface (see e.g. Lee et al. (1998) Journal of Immunology, 160:4951-4960).

In some embodiments, the cell is an LCL 721.221, K562 cell or RMA-S cell that is transfected to express an MHC-E molecule stabilized in the presence of an MHC class Ia leader sequence. Cells lines that are engineered to express cell surface MHC-E stabilized in the presence of an MHC class Ia leader sequence peptide are known in the art (Lee et al. (1998) Journal of Immunology, 160:4951-4960; Zhongguo et al. (2005) 13:464-467; Garcia et al. (2002) Eur J. Immunol., 32:936-944). Exemplary of such as cell line for use in the methods provided herein are 221-AEH cells.

In some embodiments, the cell is an artificial antigen presenting cell (aAPC). Typically, aAPCs include features of natural APCs, including expression of an MHC molecule, stimulatory and costimulatory molecule(s), Fc receptor, adhesion molecule(s) and/or the ability to produce or secrete cytokines (e.g. IL-2). Normally, an aAPC is a cell line that lacks expression of one or more of the above, and is generated by introduction (e.g. by transfection or transduction) of one or more of the missing elements from among an MHC molecule, a low affinity Fc receptor (CD32), a high affinity Fc receptor (CD64), one or more of a co-stimulatory signal (e.g. CD7, B7-1 (CD80), B7-2 (CD86), PD-L1, PD-L2, 4-1BBL, OX40L, ICOS-L, ICAM, CD30L, CD40, CD70, CD83, HLA-G, MICA, MICB, HVEM, lymphotoxin beta receptor, ILT3, ILT4, 3/TR6 or a ligand of B7-H3; or an antibody that specifically binds to CD27, CD28, 4-1BB, OX40, CD30, CD40, PD-1, ICOS, LFA-1, CD2, CD7, LIGHT, NKG2C, B7-H3, Toll ligand receptor or a ligand of CD83), a cell adhesion molecule (e.g. ICAM-1 or LFA-3) and/or a cytokine (e.g. IL-2, IL-4, IL-6, IL-7, IL-10, IL-12, IL-15, IL-21, interferon-alpha (IFNα), interferon-beta (IFNβ), interferon-gamma (IFNγ), tumor necrosis factor-alpha (TNFα), tumor necrosis factor-beta (TNFβ), granulocyte macrophage colony stimulating factor (GM-CSF), and granulocyte colony stimulating factor (GCSF)). In some cases, an aAPC does not normally express an MHC molecule, but can be engineered to express an MHC molecule or, in some cases, is or can be induced to express an MHC molecule, such as by stimulation with cytokines. In some cases, aAPCs also can be loaded with a stimulatory or co-stimulatory ligand, which can include, for example, an anti-CD3 antibody, an anti-CD28 antibody or an anti-CD2 antibody. Exemplary of a cell line that can be used as a backbone for generating an aAPC is a K562 cell line or fibroblast cell line. Various aAPCs are known in the art, see e.g., U.S. Patent No. 8,722,400, published application No. US2014/0212446; Butler and Hirano (2014) Immunol Rev., 257(1):10. 1111/imr.12129; Suhoshki et al. (2007) Mol. Ther., 15:981-988).

In some embodiments, the cell line can be, optionally, genetically modified with a β2-microglobulin, such as if the parent cell does not express endogenous β2-microglobulin. In some embodiments, a single MHC-E heavy chain is introduced into the cells, such as by using an expression vector. In some embodiments, an MHC-E heavy chain and β2-microglobulin are introduced, either simultaneously or sequentially in any order, such as by using one or more expression vectors.

In some embodiments, transfected cells expressing the expressed, such as genetically engineered, MHC-E molecule can be selected via standard procedures. In some embodiments, an HLA-specific antibody can be used to select or identify the cells. Exemplary antibodies are known in the art and non-limiting examples are described elsewhere herein. In some embodiments, the particular MHC expression is confirmed by flow cytometry.

In some embodiments, the provided methods can employ a control cell line that expresses a classical MHC class I (i.e. MHC class Ia) and/or an MHC class II on the cell surface. In some embodiments, the control cells can be used in subtractive methods to eliminate peptide epitopes presented in the context of an MHC class Ia molecule or in the context of an MHC class II molecule. In some embodiments, the control cells can be used for negative selection of binding molecules that bind or recognize a peptide epitope presented in the context of an MHC class Ia molecule or an MHC class II molecule. In some embodiments, the parent or backbone cell or cell line is the same as the MHC-E expressing cells, except that such control cells additionally express MHC class Ia or express MHC class Ia instead of MHC-E. In some embodiments, an MHC-E-expressing cell (e.g. test cell) is used for positive selection and a classical MHC class I molecule (e.g. HLA-A, -B or -C) and/or MHC class II molecule (e.g. HLA-DR or HLA-DQ) is used for negative selection.

Generally, most nucleated cells express MHC class Ia molecules. In some embodiments, cells can be induced to express an MHC class Ia molecule. In some embodiments, cells can be engineered to express an MHC class Ia molecule, such as a particular MHC class Ia allele. For example, in some embodiments, the MHC class la-expressing cell is one that has been prepared by genetically modifying a parental cell line, such as a mammalian cell line (e.g. human cell line), with a class Ia HLA α chain. In some embodiments, the cell line can be, optionally, genetically modified with a β2-microglobulin, such as if the parent cell does not express endogenous β2-microglobulin. In some embodiments, a single class Ia α allele can be introduced into the cells, such as by using an expression vector. In some embodiments, a single class Ia α allele and β2-microglobulin can be introduced into cells, either simultaneously or sequentially in any order, into the cells, such as by using one or move expression vectors.

In some embodiments, the MHC class la-expressing cell expresses an MHC class Ia allele that can be any known to be present in a subject, such as a human subject. In some embodiments, the MHC class Ia allele is an HLA-A2, HLA-A1, HLA- A3, HLA-A24, HLA-A28, HLA-A31, HLA-A33, HLA-A34, HLA-B7, HLA-B45 or HLA-Cw8 allele. In some embodiments, the MHC class Ia allele can be any set forth in Table 2, which are among the most frequent MHC class Ia alleles in the U.S. population.

| **Table 2: HLA class I** | | |
|---|---|---|
| **Class I** | **allele** | **Frequency in population** |
| 1 | HLA-A*01:01 | 12.94 |
| 2 | HLA-A*02:01 | 42.88 |
| 3 | HLA-A*02:03 | 0.19 |
| 4 | HLA-A*02:06 | 1.55 |
| 5 | HLA-A*03:01 | 13.50 |
| 6 | HLA-A*11:01 | 11.60 |
| 7 | HLA-A*23:01 | 8.30 |
| 8 | HLA-A*24:02 | 22.56 |
| 9 | HLA-A^{∗}*26:01 | 5.36 |
| 10 | HLA-A*30:01 | 6.29 |
| 11 | HLA-A*30:02 | 5.21 |
| 12 | HLA-A*31:01 | 6.87 |
| 13 | HLA-A*32:01 | 3.71 |
| 14 | HLA-A*33:01 | 2.62 |
| 15 | HLA-A*68:01 | 6.36 |
| 16 | HLA-A*68:02 | 4.79 |
| 17 | HLA-B^{∗}*07:02 | 12.96 |
| 18 | HLA-B*08:01 | 9.23 |
| 19 | HLA-B*15:01 | 6.54 |
| 20 | HLA-B*35:01 | 13.03 |
| 21 | HLA-B*40:01 | 9.79 |
| 22 | HLA-B*44:02 | 7.22 |
| 23 | HLA-*44:03 | 8.96 |
| 24 | HLA-B^{∗}*51:01 | 8.51 |
| 25 | HLA-B*53:01 | 7.26 |
| 26 | HLA-B*57:01 | 3.49 |
| 27 | HLA-B*58:01 | 4.82 |

In some embodiments, the MHC class Ia allele is an HLA-A2 allele, which in some populations is expressed by approximately 50% of the population. In some embodiments, the HLA-A2 allele can be an HLA-A*0201, *0202, *0203, *0206, or *0207 gene product. In some cases, there can be differences in the frequency of subtypes between different populations. For example, in some embodiments, more than 95% of the HLA-A2 positive Caucasian population is HLA-A*0201, whereas in the Chinese population the frequency has been reported to be approximately 23% HLA-A*0201, 45% HLA-A*0207, 8% HLA-A*0206 and 23% HLA-A*0203. In some embodiments, the MHC molecule is HLA-A*0201. In some embodiments, nucleic acids, such as vectors, encoding particular HLA alleles are available from commercial or private sources, e.g. from International Histocompatibility Working Group available at www.ihwg.org/hla., Sino Biological, Inc. (Beijing, CN), GeneCopoeia (Rockville, MD), DNASU Plasmid Repository (Arizona State University, Tempe, AZ), Addgene (Cambridge, MA) and others.

### Methods for Repressing or Disrupting a non-MHC-E gene in a Cell

In some embodiments, a cell expressing an MHC-E, such as a cell that is engineered or transfected to express an MHC-E molecule, is one in which another MHC class I molecule, such as an HLA-A, B or C, may be normally expressed, but in which the expression, activity and/or function of a gene encoding such other MHC class I, such as an HLA-A, -B or -C gene, is repressed or disrupted. In particular examples, gene repression or disruption is caused by targeting a heavy chain of another MHC class I molecule, such as an HLA-A, -B or -C molecule. Methods of repressing or disrupting genes are well known in the art, and, in some aspects, can include the use of inhibitory nucleic acid molecules or gene editing methods. In some embodiments, following repression or disruption of the gene using such methods, including any described herein, the expression of an MHC class I molecule (other than MHC-E) on the cell surface is no more than 50%, 40%, 30%, 20%, 10%, 5% or less of the expression of the molecule on the same cells in which the same MHC class I gene was not repressed or disrupted. In some embodiments, the level or degree of expression can be assessed via standard procedures. In some embodiments, an HLA-specific antibody can be used to select or identify the cells. Exemplary antibodies are known in the art and non-limiting examples are described elsewhere herein. In some embodiments, the expression of a particular MHC is determined by flow cytometry. In some embodiments, allele-specific PCR can be used to determine the level of gene expression, and hence, gene modification.

In some embodiments, gene repression is achieved using an inhibitory nucleic acid molecule that is an RNA interfering agent, which can be used to selectively suppress or repress expression of the gene. For example, gene repression can be carried out by RNA interference (RNAi), short interfering RNA (siRNA), short hairpin (shRNA), antisense, and/or ribozymes. In some embodiments, RNA interfering agents also can include other RNA species that can be processed intracellularly to produce shRNAs including, but not limited to, RNA species identical to a naturally occurring miRNA precursor or a designed precursor of an miRNA-like RNA.

In some embodiments, an RNA interfering agent is at least a partly double-stranded RNA having a structure characteristic of molecules that are known in the art to mediate inhibition of gene expression through an RNAi mechanism or an RNA strand comprising at least partially complementary portions that hybridize to one another to form such a structure. When an RNA contains complementary regions that hybridize with each other, the RNA will be said to self-hybridize. In some embodiments, an inhibitory nucleic acid, such as an RNA interfering agent, includes a portion that is substantially complementary to a target gene. In some embodiments, an RNA interfering agent targeted to a transcript can also considered targeted to the gene that encodes and directs synthesis of the transcript. In some embodiments, a target region can be a region of a target transcript that hybridizes with an antisense strand of an RNA interfering agent. In some embodiments, a target transcript can be any RNA that is a target for inhibition by RNA interference.

In some embodiments, an RNA interfering agent is considered to be "targeted" to a transcript and to the gene that encodes the transcript if (1) the RNAi agent comprises a portion, e.g., a strand, that is at least approximately 80%, approximately 85%, approximately 90%, approximately 91%, approximately 92%, approximately 93%, approximately 94%, approximately 95%, approximately 96%, approximately 97%, approximately 98%, approximately 99%, or approximately 100% complementary to the transcript over a region about 15-29 nucleotides in length, e.g., a region at least approximately 15, approximately 17, approximately 18, or approximately 19 nucleotides in length; and/or (2) the Tₘ of a duplex formed by a stretch of 15 nucleotides of one strand of the RNAi agent and a 15 nucleotide portion of the transcript, under conditions (excluding temperature) typically found within the cytoplasm or nucleus of mammalian cells is no more than approximately 15° C lower or no more than approximately 10° C lower, than the Tₘ of a duplex that would be formed by the same 15 nucleotides of the RNA interfering agent and its exact complement; and/or (3) the stability of the transcript is reduced in the presence of the RNA interfering agent as compared with its absence.

In some embodiments, an RNA interfering agent optionally includes one or more nucleotide analogs or modifications. One of ordinary skill in the art will recognize that RNAi agents can include ribonucleotides, deoxyribonucleotides, nucleotide analogs, modified nucleotides or backbones, etc. In some embodiments, RNA interfering agents may be modified following transcription. In some embodiments, RNA interfering agents can contain one or more strands that hybridize or self-hybridize to form a structure that includes a duplex portion between about 15-29 nucleotides in length, optionally having one or more mismatched or unpaired nucleotides within the duplex.

In some embodiments, the term "short, interfering RNA" (siRNA) refers to a nucleic acid that includes a double-stranded portion between about 15-29 nucleotides in length and optionally further includes a single-stranded overhang {e.g., 1-6 nucleotides in length) on either or both strands. In some embodiments, the double-stranded portion can be between 17-21 nucleotides in length, e.g., 19 nucleotides in length. In some embodiments, the overhangs are present on the 3' end of each strand, can be about or approximately 2 to 4 nucleotides long, and can be composed of DNA or nucleotide analogs. An siRNA may be formed from two RNA strands that hybridize together, or may alternatively be generated from a longer double-stranded RNA or from a single RNA strand that includes a self-hybridizing portion, such as a short hairpin RNA. One of ordinary skill in the art will appreciate that one or more mismatches or unpaired nucleotides can be present in the duplex formed by the two siRNA strands. In some embodiments, one strand of an siRNA (the "antisense" or "guide" strand) includes a portion that hybridizes with a target nucleic acid, e.g., an mRNA transcript. In some embodiments, the antisense strand is perfectly complementary to the target over about 15-29 nucleotides, sometimes between 17-21 nucleotides, e.g., 19 nucleotides, meaning that the siRNA hybridizes to the target transcript without a single mismatch over this length. However, one of ordinary skill in the art will appreciate that one or more mismatches or unpaired nucleotides may be present in a duplex formed between the siRNA strand and the target transcript.

In some embodiments, a short hairpin RNA (shRNA) is a nucleic acid molecule comprising at least two complementary portions hybridized or capable of hybridizing to form a duplex structure sufficiently long to mediate RNAi (typically between 15-29 nucleotides in length), and at least one single-stranded portion, typically between approximately 1 and 10 nucleotides in length that forms a loop connecting the ends of the two sequences that form the duplex. In some embodiments, the structure may further comprise an overhang. In some embodiments, the duplex formed by hybridization of self-complementary portions of the shRNA may have similar properties to those of siRNAs and, in some cases, shRNAs can be processed into siRNAs by the conserved cellular RNAi machinery. Thus shRNAs can be precursors of siRNAs and can be similarly capable of inhibiting expression of a target transcript. In some embodiments, an shRNA includes a portion that hybridizes with a target nucleic acid, e.g., an mRNA transcript, and can be perfectly complementary to the target over about 15-29 nucleotides, sometimes between 17-21 nucleotides, e.g., 19 nucleotides. However, one of ordinary skill in the art will appreciate that one or more mismatches or unpaired nucleotides may be present in a duplex formed between the shRNA strand and the target transcript.

In some embodiments, the shRNA comprises a nucleotide (e.g. DNA) sequence of the structure A-B-C or C-B-A. In some embodiments, the cassette comprises at least two DNA segments A and C or C and A, wherein each of said at least two segments is under the control of a separate promoter as defined above (such as the Pol III promoter including inducible U6, H1 or the like). In the above segments: A can be a 15 to 35 bp or a 19 to 29 bp DNA sequence being at least 90%, or 100% complementary to the gene to be knocked down (e.g. an HLA-A, B or C gene); B can be a spacer DNA sequence having 5 to 9 bp forming the loop of the expressed RNA hairpin molecule, and C can be a 15 to 35 or a 19 to 29 bp DNA sequence being at least 85% complementary to the sequence A.

Methods using RNA interference technology, such as siRNA or shRNA, to repress cell expression of an MHC molecule, such as an HLA-A, -B or -C molecule, are well within the level of a skilled artisan. In some embodiments, an allele specific sequence can be employed to specifically repress, downregulate and/or disrupt expression of a particular HLA allele. In some embodiments, an HLA-A, -B, -C consensus sequence can be employed to repress, downregulate and/or disrupt expression of each of the HLA-A, -B and-C loci. For example, methods using RNA interference technology, including allele-specific or consensus sequences, to target such molecules have been described (see e.g., Gonzalez et al. (2004) Molecular Therapy, 11:811-818; Haga et al. (2006) Transplant Proc., 38:3184-3188; Figueiredo et al. (2006) J. Mol. Med., 84:425-37; Figueiredo et al. (2007) Transfusion, 47:18-27; Hacke et al. (2009) Immunol. Res., 44:112-126; Lemp et al. (2013) Clin. Transpl., 93-101). Non-limiting examples of siRNA sequences that are allele-specific (e.g. HLA-A) are set forth in SEQ ID NOS: 28-31 and that are pan-specific (i.e. consensus, such as against conserved regions in HLA-A, -B, -C) are set forth in SEQ ID NOS:32-35. Non-limiting examples of shRNA sequences that are allele-specific (e.g., HLA-A) or are consensus HLA-A,-B,-C sequence are set forth in SEQ ID NO:36 or 37, respectively. Commercially available reagents, such as siRNA or shRNA reagents, also are readily available, see e.g. from Santa Cruz Biotechnology (HLA-A, catalog number sc-42908 and related reagents; HLA-B, catalog number sc-42922 and related reagents; and HLA-C, catalog number sc-105525 and related reagents).

In some embodiments, the gene repression is carried out by effecting a disruption in the gene, such as a knock-out, insertion, missense or frameshift mutation, such as a biallelic frameshift mutation, deletion of all or part of the gene, e.g., one or more exon or portion thereof, and/or knock-in. In some aspects, the disruption of another MHC class I (e.g. HLA-A, B or C) is carried out by gene editing, such as using a DNA binding protein or DNA-binding nucleic acid, which specifically binds to or hybridizes to the gene at a region targeted for disruption. In some aspects, the disruption results in a deletion, mutation and or insertion within an exon of the gene, such as within the first or second exon.

In some aspects, the protein or nucleic acid is coupled to or complexed with a gene editing nuclease, such as in a chimeric or fusion protein. In some embodiments, such disruptions are effected by an inhibitory nucleic acid molecule, which can encode sequence-specific or targeted nucleases, including DNA-binding targeted nucleases and gene editing nucleases such as zinc finger nucleases (ZFN) and transcription activator-like effector nucleases (TALENs), and RNA-guided nucleases such as a CRISPR-associated nuclease (Cas), specifically designed to be targeted to the sequence of a gene or a portion thereof.

Zinc finger, TALE, and CRISPR system binding domains can be "engineered" to bind to a predetermined nucleotide sequence, for example via engineering (e.g. altering one or more amino acids) of the recognition helix region of a naturally occurring zinc finger or TALE protein. Engineered DNA binding proteins (zinc fingers or TALEs) are proteins that are non-naturally occurring. Rational criteria for design include application of substitution rules and computerized algorithms for processing information in a database storing information of existing ZFP and/or TALE designs and binding data. See, for example, U.S. Pat. Nos. 6,140,081; 6,453,242; and 6,534,261; see also WO 98/53058; WO 98/53059; WO 98/53060; WO 02/016536 and WO 03/016496 and U.S. Publication No. 20110301073.

In some embodiments, the repression is carried out using DNA-targeting molecule includes a DNA-binding protein such as one or more zinc finger protein (ZFP) or transcription activator-like protein (TAL), fused to an effector protein such as an endonuclease. Examples include ZFNs, TALEs, and TALENs. See Lloyd et al., Frontiers in Immunology, 4(221), 1-7 (2013).

A ZFP or domain thereof is a protein or domain within a larger protein that binds DNA in a sequence-specific manner through one or more zinc fingers, regions of amino acid sequence within the binding domain whose structure is stabilized through coordination of a zinc ion. The term zinc finger DNA binding protein is often abbreviated as zinc finger protein or ZFP. Among the ZFPs are artificial ZFP domains targeting specific DNA sequences, typically 9-18 nucleotides long, generated by assembly of individual fingers. ZFPs include those in which a single finger domain is approximately 30 amino acids in length and contains an alpha helix containing two invariant histidine residues coordinated through zinc with two cysteines of a single beta turn, and having two, three, four, five, or six fingers. Generally, sequence-specificity of a ZFP may be altered by making amino acid substitutions at the four helix positions (-1, 2, 3 and 6) on a zinc finger recognition helix. Thus, in some embodiments, the ZFP or ZFP-containing molecule is non-naturally occurring, e.g., is engineered to bind to a target site of choice.

In some embodiments, the DNA-targeting molecule is or comprises a zinc-finger DNA binding domain fused to a DNA cleavage domain to form a zinc-finger nuclease (ZFN). In some embodiments, fusion proteins comprise the cleavage domain (or cleavage half-domain) from at least one Type IIS restriction enzyme and one or more zinc finger binding domains, which may or may not be engineered. In some embodiments, the cleavage domain is from the Type IIS restriction endonuclease Fok I. Fok I generally catalyzes double-stranded cleavage of DNA, at 9 nucleotides from its recognition site on one strand and 13 nucleotides from its recognition site on the other. See, for example, U.S. Pat. Nos. 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31,978-31,982.].

Many gene-specific engineered zinc fingers are available commercially. For example, Sangamo Biosciences (Richmond, CA, USA) has developed a platform (CompoZr) for zinc-finger construction in partnership with Sigma-Aldrich (St. Louis, MO, USA), allowing investigators to bypass zinc-finger construction and validation altogether, and provides specifically targeted zinc fingers for thousands of proteins. Gaj et al., Trends in Biotechnology, 2013, 31(7), 397-405. In some embodiments, commercially available zinc fingers are used or are custom designed. (See, for example, Sigma-Aldrich catalog numbers CSTZFND, CSTZFN, CTI1-1KT, and PZD0020). Methods of repressing or disrupting cell expression of an MHC class I molecule using ZFNs are known in the art (see e.g. Torikai et al. (2013) Blood, 122:1341-1349, which describes methods for disrupting the HLA-A locus, such as in HLA-alleles HLA-A^{∗}03:01 or HLA-A^{∗}02:01).

In some cases, the repression is carried out using clustered regularly interspaced short palindromic repeats (CRISPR) and CRISPR-associated (Cas) proteins. *See* Sander and Joung, Nature Biotechnology, 32(4): 347-355. In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), and/or other sequences and transcripts from a CRISPR locus.

In some embodiments, the CRISPR/Cas nuclease or CRISPR/Cas nuclease system includes a non-coding RNA molecule (guide) RNA (gRNA), which sequence-specifically binds to DNA, and a Cas protein (e.g., Cas9), with nuclease functionality (e.g., two nuclease domains). In general, a guide sequence is any polynucleotide sequence having sufficient complementarity with a target polynucleotide sequence, such as a gene encoding an MHC Class I molecule (e.g. HLA-A, -B or -C) to hybridize with the target sequence and direct sequence-specific binding of the CRISPR complex to the target sequence. Typically, in the context of formation of a CRISPR complex, "target sequence" generally refers to a sequence to which a guide sequence is designed to have complementarity, where hybridization between the target sequence and a guide sequence promotes the formation of a CRISPR complex. Full complementarity is not necessarily required, provided there is sufficient complementarity to cause hybridization and promote formation of a CRISPR complex. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. In some embodiments, a guide sequence is selected to reduce the degree of secondary structure within the guide sequence. Secondary structure may be determined by any suitable polynucleotide folding algorithm.

In some embodiments, a CRISPR enzyme (e.g. Cas9 nuclease) in combination with (and optionally complexed with) a guide sequence is delivered to the cell. In some embodiments, one or more elements of a CRISPR system is derived from a type I, type II, or type III CRISPR system. In some embodiments, one or more elements of a CRISPR system are derived from a particular organism comprising an endogenous CRISPR system, such as *Streptococcus pyogenes.*

Method using CRISPR systems for knockout of a particular MHC class I, such as an HLA-A, -B- or -C, are known in the art (see e.g. Sanjana et al. (2014) Nat. Methods, 11:783-4). In an exemplary method, Cas9 nuclease (e.g., that encoded by mRNA from *Staphylococcus aureus* or from *Streptococcus pyogenes,* e.g. pCW-Cas9, Addgene #50661, Wang et al. (2014) Science, 3:343-80-4; or nuclease or nickase lentiviral vectors available from Applied Biological Materials (ABM; Canada) as Cat. No. K002, K003, K005 or K006) and a guide RNA specific to the target antigen gene are introduced into cells, for example, using lentiviral delivery vectors or any of a number of known delivery method or vehicle for transfer to cells, such as any of a number of known methods or vehicles for delivering Cas9 molecules and guide RNAs. Nonspecific or empty vector control cells also can be generated. Degree of Knockout of a gene (e.g., 24 to 72 hours after transfer) can be assessed using any of a number of well-known assays for assessing gene disruption in cells. For example, exemplary guide RNA sequences can include any set forth in SEQ ID NOS: 10-27. Commercially available kits, gRNA vectors and donor vectors, for knockout of a particular MHC class I, such as an HLA-A, -B or -C, via CRISPR also are readily available. For example, reagents for knockout of an HLA-A gene are available, for example, from GeneCopoeia (see e.g. catalog numbers HTN262410 or HTN208849, Origene Technologies (catalog No. KN200661). In another example, reagents for knockout of an HLA-B gene are available, for example, from Santa Cruz Biotechnology, Inc. ( see e.g. catalog number sc-400627). In a further example, reagents for knockout of an HLA-C gene are available, for example, from Santa Cruz Biotechnology, Inc. (see e.g. catalog number sc-401517).

In some embodiments, an agent capable of inducing a genetic disruption, such as a knockdown or a knockout of genes encoding a classical MHC class I, such as an HLA-A, -B- or -C, is introduced as a complex, such as a ribonucleoprotein (RNP) complex. RNP complexes include a sequence of ribonucleotides, such as an RNA or a gRNA molecule, and a polypeptide, such as a Cas9 protein or variant thereof. In some embodiments, the Cas9 protein is delivered as an RNP complex that comprises a Cas9 protein and a gRNA molecule, e.g., a gRNA targeted for a gene encoding a classical MHC class I, such as an HLA-A, -B- or -C. In some embodiments, the RNP that includes one or more gRNA molecules targeted for a gene encoding a classical MHC class (e.g. gene encoding HLA-A, -B- or -C), and a Cas9 enzyme or variant thereof, is directly introduced into the cell via physical delivery (e.g., electroporation, particle gun, Calcium Phosphate transfection, cell compression or squeezing), liposomes or nanoparticles.

In some embodiments, the degree of knockout of a gene (e.g., a gene encoding a classical MHC class I molecule, e.g. HLA-A, -B- or -C) at various time points, e.g., 24 to 72 hours after introduction of agent, can be assessed using any of a number of well-known assays for assessing gene disruption in cells. Degree of knockdown of a gene at various time points, e.g., 24 to 72 hours after introduction of agent, can be assessed using any of a number of well-known assays for assessing gene expression in cells, such as assays to determine the level of transcription or protein expression or cell surface expression.

### B. Peptide Sources, Displaying Peptide Epitopes and/or Generation of MHC-Peptide Complexes on Cells

In some embodiments, peptides derived from a target antigen or an immunogenic and/or antigenic fragment of a target antigen, are contacted, exposed, incubated or provided to an MHC-E molecule, such as an MHC-E molecule expressed on the surface of cells. In some embodiments, the immunogenic and/or antigenic portion or fragment of a target antigen can be any suitable immunogenic sequence that is known or can be determined using routine art-known methods. See, e.g., Ausubel, F. M., et al., 1998, Current Protocols in Molecular Biology, John Wiley & Sons, chapter 11.15. Typically, the target antigen or immunogenic or antigenic fragment thereof is at least 6 amino acids in length, such as at least about 8, at least about 10, at least about 20, at least about 50 residues, at least about 100 residues or the full-length sequence of the antigen.

In some embodiments, the peptides are derived or obtained from or represent a plurality of peptides of the target antigen, in which such plurality of peptides include or are an overlapping panel of potential peptide epitopes. In some embodiments, a plurality of such overlapping peptides can be contacted, exposed to or incubated with an MHC-E molecule, and assessed for binding or immune reactivity.

In some embodiments, the peptides are derived from an exogenous or heterologous target antigen present in MHC-E-expressing cells, whereby, upon antigen processing and display of the peptides on the cell surface, the MHC-E molecule is contacted with one or more peptides derived from the target antigen. Hence, in some cases, peptides that are naturally processed by the cell are contacted or exposed to the MHC-E molecule expressed by the cell for display on the cell surface as an MHC-E-peptide complex.

In some embodiments, the target antigen is a predetermined or known antigen that is known to contain or suspected of containing a T cell epitope or peptide epitope, and which may, in some cases, trigger an immune response and/or include an immunogenic region containing the peptide epitope for recognition by the immune system. In some embodiments, the peptide can be one that is derived from or based on a fragment of the target antigen, which is generally a longer biological molecule, such as a polypeptide or protein.

In some embodiments, the peptide is generally a portion of a polypeptide (e.g. heterologous antigen) less than the full length but that is greater than or equal to 2 amino acids in length, such as one that is greater than or equal to 2 amino acids and less than or equal to 50 or 40 amino acids in length. In some embodiments, the peptide is between 7 and 40 amino acids, 8 and 20 amino acids, 10 and 17 amino acids, 7 and 13 amino acids or 8 and 10 amino acids. In some embodiments, the peptide has a length of between 7 and 20 amino acids. In some embodiments, the peptide has a length of 7, 8, 9, 10, 11, 12, 13, 14, 15, 16, 17, 18, 19, or 20 amino acids. In some embodiments, the peptide, typically is about 8 to about 24 amino acids in length. In some embodiments, the peptide has a length of from or from about 8 to 13 amino acids, such as for recognition in the MHC class Ib (MHC-E) complex. In some embodiments, the peptide can associate with an MHC molecule, which, in some cases, results in recognition by specific T cells through their T cell receptor after presentation in the context of an MHC molecule. In some embodiments, the peptide is capable of inducing an immune response in an animal by its binding characteristics to MHC molecules. In some embodiments, upon recognition of the peptide epitope (e.g. MHC-peptide complex) by a peptide binding molecule (e.g. TCR or TCR-like antibody) the TCR (or other peptide binding molecule) can produce or trigger an activation signal to the T cell that induces a T cell response, such as T cell proliferation, cytokine production, a cytotoxic T cell response or other response.

In some embodiments, the MHC-E molecule, such as an MHC-E molecule expressed on a cell, is contacted, exposed and/or incubated with the one or a plurality of peptides for a time period sufficient to form an MHC-E-peptide complex or complexes on the cell surface if MHC-E peptide binding molecule has binding activity for one of the peptides. In some such embodiments, the peptides are overlapping peptides or peptides processed from a transferred antigen. It is within a level of a skilled artisan to empirically determine the time necessary to form the MHC-peptide complex. In some embodiments, the cells are incubated for about 15 minutes to 24 hours after contacting the MHC-E molecule with the one or plurality of peptides.

In some embodiments, the cells that have been contacted, exposed to and/or incubated with one or a plurality of peptides, can be assessed for formation of an MHC-E-peptide complex formed by recognition of a peptide by the MHC-E molecule. In some such embodiments, the peptides are overlapping peptides or peptides processed from a transferred antigen. In some embodiments, the formed MHC-E-peptide complex can be identified and detected, such as by using methods described below. In some cases, one or more binding molecules, e.g. a TCR or a TCR-like antibody or antigen-binding fragment thereof, can be assessed directly for binding to a formed MHC-E-peptide complex on the cell. In some embodiments, detecting and/or identifying an MHC-E-peptide complex and/or identifying a binding molecule that binds to the MHC-E-peptide complex can include assessment of cytotoxic T lymphocyte (CTL) activity.

Exemplary aspects of such methods are described in the following subsections.

### 1. Exemplary Target Antigens

In some embodiments, the target antigen from which the peptides are derived can be a polypeptide antigen or fragment thereof, including an antigen from a cellular gene. In some embodiments, the antigen is a tumor-associated antigen, an antigen expressed in a particular cell type associated with an autoimmune or inflammatory disease or an antigen derived from a viral pathogen or a bacterial pathogen. In some embodiments, the target antigen is an antigen associated with a disease. In some embodiments, the disease can be caused by malignancy or transformation of cells, such as a cancer. In some embodiments, the antigen can be a protein antigen from a tumor or cancer cell, such as a tumor-associated antigen. In some embodiments, the disease can be caused by infection, such as by bacterial or viral infection. In some embodiments, the antigen is a viral-associated cancer antigen. In some embodiments, the disease can be an autoimmune disease. Other targets include those listed in The HLA Factsbook (Marsh et al. (2000)) and others known in the art.

In some embodiments, the target antigen is one that is associated with a tumor or cancer. In some embodiments, a tumor or cancer antigen is one that can be found on a malignant cell, found inside a malignant cell or is a mediator of tumor cell growth. In some embodiments, a tumor or cancer antigen is one that is predominantly expressed or overexpressed by a tumor cell or cancer cell. In some embodiments, tumor antigens include, but are not limited to, mutated peptides, differentiation antigens, and overexpressed antigens, all of which could serve as targets for therapies.

In some embodiments, the tumor or cancer antigen is a lymphoma antigen, (e.g., non-Hodgkin's lymphoma or Hodgkin's lymphoma), a B-cell lymphoma cancer antigen, a leukemia antigen, a myeloma (i.e., multiple myeloma or plasma cell myeloma) antigen, an acute lymphoblastic leukemia antigen, a chronic myeloid leukemia antigen, or an acute myelogenous leukemia antigen. In some embodiments, the cancer antigen is an antigen that is overexpressed in or associated with a cancer that is an adenocarcinomas, such as pancreas, colon, breast, ovarian, lung, prostate, head and neck, including multiple myelomas and some B cell lymphomas. In some embodiments, the antigen is associated with a cancer, such as prostate cancer, lung cancer, breast cancer, ovarian cancer, pancreatic cancer, skin cancer, liver cancer (e.g., hepatocellular adenocarcinoma), intestinal cancer, or bladder cancer.

In some embodiments, the target antigen or fragment thereof is a tumor antigen that can be a glioma-associated antigen, B-cell maturation antigen (BCMA, BCM), B-cell activating factor receptor (BAFFR, BR3), and/or transmembrane activator and CAML interactor (TACI), Fc Receptor-like 5 (FCRL5, FcRH5), β-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, Melanin-A/MART-1, WT-1, S-100, MBP, CD63, MUC1 (e.g. MUC1-8), p53, Ras, cyclin B1, HER-2/neu, carcinoembryonic antigen (CEA), gp100, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A11, MAGE-B1, MAGE-B2, MAGE-B3, MAGE-B4, MAGE-C1, BAGE, GAGE-1, GAGE-2, pl5, tyrosinase (e.g. tyrosinase-related protein 1 (TRP-1) or tyrosinase-related protein 2 (TRP-2)), β-catenin, NY-ESO-1, LAGE-1a, PP1, MDM2, MDM4, EGVFvIII, Tax, SSX2, telomerase, TARP, pp65, CDK4, vimentin, S100, eIF-4A1, IFN-inducible p78, and melanotransferrin (p97), Uroplakin II, prostate specific antigen (PSA), human kallikrein (huK2), prostate specific membrane antigen (PSM), and prostatic acid phosphatase (PAP), neutrophil elastase, ephrin B2, BA-46, Bcr-abl, E2A-PRL, H4-RET, IGH-IGK, MYL-RAR, Caspase 8 or a B-Raf antigen. Other tumor antigens can include any derived from FRa, CD24, CD44, CD133, CD 166, epCAM, CA-125, HE4, Oval, estrogen receptor, progesterone receptor, uPA, PAI-1, CD19, CD20, CD22, ROR1, CD33/IL3Ra, c- Met, PSMA, Glycolipid F77, GD-2, insulin growth factor (IGF)-I, IGF-II, IGF-I receptor and mesothelin, or any immunogenic or antigenic fragment thereof.

In some embodiments, the target antigen is a viral antigen. Many viral antigen targets have been identified and are known, including peptides derived from viral genomes in HIV, HTLV and other viruses (see e.g., Addo et al. (2007) PLoS ONE, 2, e321 ; Tsomides et al. (1994) J Exp Med, 180, 1283-93; Utz et al. (1996) J Virol, 70, 843-51 ). Exemplary viral antigens include, but are not limited to, an antigen from hepatitis A, hepatitis B (e.g., HBV core and surface antigens (HBVc, HBVs)), hepatitis C (HCV), Epstein-Barr virus (e.g. EBVA), human papillomavirus (HPV; e.g. E6 and E7), human immunodeficiency type-1 virus (HIV1), Kaposi's sarcoma herpes virus (KSHV), human papilloma virus (HPV), influenza virus, Lassa virus, HTLN-1, HIN-1, HIN-II, CMN, EBN or HPN. In some embodiments, the target protein is a bacterial antigen or other pathogenic antigen, such as Mycobacterium tuberculosis (MT) antigens, trypanosome, e.g., Tiypansoma cruzi (T. cruzi), antigens such as surface antigen (TSA), or malaria antigens. Specific viral antigen or epitopes or other pathogenic antigens or peptide epitopes are known (see e.g., Addo et al. (2007) PLoS ONE, 2, e321 ; Anikeeva et al. (2009) Clin Immunol, 130, 98-109).

In some embodiments, the antigen is an antigen derived from a virus associated with cancer, such as an oncogenic virus. For example, an oncogenic virus is one in which infection from certain viruses are known to lead to the development of different types of cancers, for example, hepatitis A, hepatitis B (HBV ), hepatitis C (HCV), human papilloma virus (HPV), hepatitis viral infections, Epstein-Barr virus (EBV), human herpes virus 8 (HHV-8), human T-cell leukemia virus-1 (HTLV-1), human T-cell leukemia virus-2 (HTLV-2), or a cytomegalovirus (CMV) antigen.

In some embodiments, the viral antigen is an HPV antigen, which, in some cases, can lead to a greater risk of developing cervical and/or head and neck cancers. In some embodiments, the antigen can be a HPV-16 antigen, and HPV-18 antigen, and HPV-31 antigen, an HPV-33 antigen or an HPV-35 antigen. In some embodiments, the viral antigen is an HPV-16 antigens (e.g., seroreactive regions of the E1, E2, E6 or E7 proteins of HPV-16, see *e.g.* U.S. Pat. No. 6,531,127) or an HPV-18 antigens (e.g., seroreactive regions of the L1 and/or L2 proteins of HPV-18, such as described in U.S. Pat. No. 5,840,306).

In some embodiments, the viral antigen is a HBV or HCV antigen, which, in some cases, can lead to a greater risk of developing liver cancer than HBV or HCV negative subjects. For example, in some embodiments, the heterologous antigen is an HBV antigen, such as a hepatitis B core antigen or an hepatitis B envelope antigen (US2012/0308580).

In some embodiments, the viral antigen is an EBV antigen, which, in some cases, can lead to a greater risk for developing Burkitt's lymphoma, nasopharyngeal carcinoma and Hodgkin's disease than EBV negative subjects. For example, EBV is a human herpes virus that, in some cases, is found associated with numerous human tumors of diverse tissue origin. While primarily found as an asymptomatic infection, EBV-positive tumors can be characterized by active expression of viral gene products, such as EBNA-1, LMP-1 and LMP-2A. In some embodiments, the heterologous antigen is an EBV antigen that can include Epstein-Barr nuclear antigen (EBNA)-1, EBNA-2, EBNA-3A, EBNA-3B, EBNA-3C, EBNA-leader protein (EBNA-LP), latent membrane proteins LMP-1, LMP-2A and LMP-2B, EBV-EA, EBV-MA or EBV-VCA.

In some embodiments, the viral antigen is an HTLV-1 or HTLV-2 antigen, which, in some cases, can lead to a greater risk for developing T-cell leukemia than HTLV-1 or HTLV-2 negative subjects. For example, in some embodiments, the heterologous antigen is an HTLV-antigen, such as TAX.

In some embodiments, the viral antigen is a HHV-8 antigen, which, in some cases, can lead to a greater risk for developing Kaposi's sarcoma than HHV-8 negative subjects. In some embodiments, the heterologous antigen is a CMV antigen, such as pp65 or pp64 (see U.S. Patent No. 8361473).

In some embodiments, the heterologous antigen is an autoantigen, such as an antigen of a polypeptide associated with an autoimmune disease or disorder. In some embodiments, the autoimmune disease or disorder can be multiple sclerosis (MS), rheumatoid arthritis (RA), Sjogren syndrome, scleroderma, polymyositis, dermatomyositis, systemic lupus erythematosus, juvenile rheumatoid arthritis, ankylosing spondylitis, myasthenia gravis (MG), bullous pemphigoid (antibodies to basement membrane at dermal-epidermal junction), pemphigus (antibodies to mucopolysaccharide protein complex or intracellular cement substance), glomerulonephritis (antibodies to glomerular basement membrane), Goodpasture's syndrome, autoimmune hemolytic anemia (antibodies to erythrocytes), Hashimoto's disease (antibodies to thyroid), pernicious anemia (antibodies to intrinsic factor), idiopathic thrombocytopenic purpura (antibodies to platelets), Grave's disease, or Addison's disease (antibodies to thyroglobulin). In some embodiments, the autoantigen, such as an autoantigen associated with one of the above autoimmune disease, can be collagen, such as type II collagen, mycobacterial heat shock protein, thyroglobulin, acetyl choline receptor (AcHR), myelin basic protein (MBP) or proteolipid protein (PLP). Specific autoimmune associated epitopes or antigens are known ( see e.g., Bulek et al. (2012) Nat Immunol, 13, 283-9; Harkiolaki et al. (2009) Immunity, 30, 348-57; Skowera et al. (2008) J Clin Invest, 1 18, 3390-402).

### 2. Overlapping Peptides of a Target Antigen

In some embodiments, provided is a method for identifying a peptide epitope that is bound or recognized by an MHC-E molecule or otherwise capable of inducing an immune response (e.g. CTL response) in the context of an MHC-E in which an overlapping panel of potential peptide epitopes within a target antigen are contacted, exposed or incubated with an MHC-E molecule. For example, methods employing a plurality of peptides as a source of peptides can be employed when the sequence of a target antigen is known, including any of the known tumor or viral antigens described. In some embodiments, peptides containing a contiguous sequence of amino acids present in a target antigen can be synthesized and screened for binding to MHC-E-expressing cells and/or for the ability to induce an immune response (e.g. CTL response) in the context of MHC-E. Exemplary assays for assessing MHC-restricted responses are described below. In some embodiments, peptides of the target antigen are 8-20 amino acids, such as 8-13 amino acids, such as generally 8-10 amino acids in length

In some embodiments, the peptides typically overlap (i.e. share a region of amino acid sequence identity) with one or more other peptides among the plurality of peptides of the target antigen. In some embodiments, the peptides overlap by 2 to 15 amino acids, such as 2 to 10 amino acids or 2 to 5 amino acids. As an example, a first peptide can be residues 1-9 of a target antigen and an overlapping peptide thereto can be residues 7-15 of the predetermined antigen. The skilled artisan will appreciate, however, that the length of the peptides and the amount of residue overlap between peptides can vary, depending on the length of the target antigen, region of interest, the degree of resolution and other factors.

In some embodiments, overlapping peptides of a target antigen can include a plurality of overlapping 9mer peptides of the antigen, a plurality of overlapping 10mer peptides of the antigen, a plurality of overlapping 11mer peptides of the antigen, a plurality of overlapping 12mer peptides of the antigen, a plurality of overlapping 13mer peptides of the antigen, a plurality of overlapping 14mer peptides of the antigen, or a plurality of overlapping 15mer peptides of the antigen.

In some cases, longer peptides, such as 10 to 20 amino acids in length, can be contacted, incubated and/or exposed to the cells, in which case such peptides will be fragmented to smaller peptides by the action of extracellular processing (Kern et al. (2000) Eur. J. Immunol., 30:1676-1682). In some embodiments, the overlapping peptide library contains peptides of greater than 12 amino acids in length, such as greater than 13, 14, 15 or more amino acids in length. For example, the overlapping peptide library contains a plurality of overlapping 15mer peptides of the antigen. In an exemplary embodiment, the overlapping 15-amino acid peptides can include overlap of at least 9 amino acids with one or more other peptides.

In some embodiments, potential candidate peptides are synthesized and individually contacted, exposed and/or incubated with an MHC-E molecule, and tested for binding to the MHC-E molecule. In some embodiments, the MHC-E molecule is a soluble MHC-E, and binding can be assessed by monitoring changes in folding or stability (Miller et al. (2003) 171:1369-1375; Strong et al. (1998) J Biol. Chem, 278:5082-5090). For example, in an exemplary embodiment, soluble HLA-E can be incubated with a candidate peptide or peptides and folded HLA-E can be detected using a sandwich ELISA with an HLA-E specific monoclonal antibody. In some embodiments, the MHC-E molecule is expressed on the surface of a cell, e.g. a cell known to express or engineered to express MHC-E as described above, and the MHC-E expressing cells are contacted, exposed and/or incubated with a candidate peptide or peptides. In some embodiments, one or more candidate peptides are capable of binding to the MHC-E molecule, e.g. the peptides can be directly loaded onto the cell surface. Thus, in some embodiments, the peptides can be presented on MHC molecules without processing. In some embodiments, the candidate peptides require further processing. For example, in some embodiments, some epitopes bind MHC in a way that is dependent on MHC-loading in endosomes, and hence require processing (Viner et al (1995) Proc. Natl. Acad. Sci. 92:2214-2218).

In some embodiments, pools or mixtures of peptides, such as overlapping peptides, can be prepared, where each pool contains at least two peptides having a contiguous sequence of amino acids of the target antigen. By combining members into pools, the method can permit the screening of a large number of peptides using a smaller number of samples. In some cases, when the target antigen is a longer polypeptide, the efficiency of screening can be increased by screening a plurality of peptides as a pool of peptides, although such method also can be performed when the sequence of the target antigen is shorter. In some embodiments, each peptide pool can contain about 2 to about 20 different peptides, such as about 2 to 15 different peptides, about 2 to 10 different peptides or about 2 to 5 different peptides. In some embodiments, the peptides contain a contiguous sequence of the target antigen and share a region of amino acid sequence identity with at least one other peptide in the library. Typically, the criteria for sorting the peptides into pools can vary, as will be appreciated by a skilled artisan. In some embodiments, pools are provided of at least or about 2, at least or about 3, at least or about 4, at least or about 6, at least or about 8, at least or about 10 or more overlapping peptides (e.g. spanning a contiguous region of the target antigen). In some embodiments, one or more peptide pools are identified or detected that bind or recognize MHC-E expressing cells and/or that elicit an immune response in the context of MHC-E.

In some embodiments, one or more additional rounds (or cycles) of screening can be performed, in which individual peptides of the identified or detected peptide pool(s) are further tested for binding to an MHC-E and/or for immune reactivity in the context of an MHC-E. By analysis of the individual peptides, the peptide epitope can be localized to a peptide or peptides, or to a portion of one or more peptides.

In some embodiments, the pools of overlapping peptides can be screening or tested in an orthogonal pooling strategy. For example, in some embodiments, peptide pools are generated that each contain a plurality of different overlapping peptides, where at least one overlapping peptide is present or is the same in at least two peptide pools. Typically, each peptide present in a particular pool also is present in one other pool, such that each peptide is present in two different pools. In some embodiments, the identity of members of each peptide pool is known. In some embodiments, a peptide epitope can be identified or detected by deconvoluting the pools, whereby a positive signal for binding and/or immune reactivity that is identified or detected in multiple pools can be assigned to the particular peptide that is common or unique only to those pools.

In some embodiments, the peptide screening can be performed in an array, such as in a spatial or addressable array. As used herein, "a spatial array" refers to a collection or library of individual peptides or pools of peptides in which members are separated or occupy a distinct space in the array such that each member of the library is positionally located in an array to permit identification or detection from other members of the library. In some embodiments, the array can contain a solid support containing a plurality of different, known locations at which a component or sample can be placed. In some embodiments, a spatial array can include, for example, microtiter plates with addressable wells. For examples, members of the library can be arrayed by positioning each in a well of a multi-well plate, e.g. 48-well, 96-well, 144-well, 192-well, 240-well, 288-well, 336-well, 384-well, 432-well, 480-well, 576-well, 672-well, 768-well, 864-well, 960-well, 1056-well, 1152-well, 1248-well, 1344-well, 1440-well, or 1536-well plates. In some embodiments, assessing binding to an MHC-E molecule and/or immune reactivity in the context of an MHC-E molecule can be performed in the array.

In some embodiments, an MHC-E molecule and/or an MHC-E expressing cell, is contacted with an array of peptides, where each peptide or pool of peptides is positioned in a spatial address of the array (e.g. well of a multiwell plate). In some embodiments, the identity of members of the peptide library at each position in the array is known. In some embodiments, such as where the assay is performed in an addressable format, the spatial location of a peptide or pool of peptides that exhibits a positive interaction with an MHC-E molecule, e.g. binding to the MHC-E molecule and/or capability to elicit an immune response in the context of the MHC-E molecule, can be used to identify the source of the detected or identified peptide epitope.

In some embodiments, screening assays employing overlapping peptides can indicate the location of the epitope in the antigen or the area or region of the antigen in which the epitope is located. In some embodiments, the minimal peptide MHC-E-restricted epitope can be assessed by further measuring the response to truncated peptides of an identified or detected peptide epitope. For example, if a response is identified or detected to a peptide that is 15 amino acid residues in length in the overlapping peptide library, truncated peptides or sets of truncated peptides can be generated that are missing one or more amino acids at both ends (e.g. 1-14, 1-13, 1-12 and 2-15, 3-15, 4-15). In some embodiments, the one or more truncated peptides or sets of truncated peptides can be contacted with an MHC-E molecule (e.g. as expressed on MHC-expressing cells) and a minimal peptide epitope can be identified that is bound or recognized by an MHC-E molecule or otherwise capable of inducing an immune response (e.g. CTL response) in the context of an MHC-E molecule.

In some embodiments, prior to or simultaneously with contacting, incubating and/or exposing a cell with one or more peptides, the interactions of MHC-E with endogenous peptides is reduced, reversed and/or inhibited. In some embodiments, the cell can be stripped of endogenous peptides bound to MHC on the cell surface. In some embodiments, endogenous peptides can be stripped off the surface of cells by incubating the cells at a low pH, e.g. pH of from or from about 2-3, for a short period of time, such as incubated from or from about 1 minute to 1 hours, such as 5 minutes to 30 minutes. In some embodiments, the gene encoding the transporter associated with antigen presentation (TAP) is disrupted and/or repressed in the cell, thereby blocking the intracellular peptide supply for the molecule. For example, in some embodiments, an inhibitory nucleic acid molecule, e.g. RNAi, that exhibits complementarity for the TAP gene is introduced into the cell.

In some embodiments, the contacting, exposing and/or incubating is performed at a temperature of or about 15 °C to 42 °C, such as 20 °C to 28 °C (e.g. 24 °C ± 2 °C) or 32 °C to 40 °C (e.g. 37 ± 2 °C). In some cases, the contacting, exposing and/or incubating is performed at or about 26 °C.

In some embodiments, prior to or simultaneously with contacting, incubating and/or exposing a cell with one or more peptides, expression of the MHC-E molecule on the cell surface is stabilized. In some embodiments, exogenous beta-2-microglobulin is added to the medium, which, in some cases, can stabilize the free heavy chain on the surface of cells (see e.g.,. Marin et al. (2003) Immunogenetics, 54:767-775). It is within the level of a skilled artisan to empirically determine the concentration of beta-2-microglobulin to be added. In some aspects, about 1 µg/mL to 100 µg/mL, such as generally 1 µg/mL to 20 µg/mL is added.

### 3. Processed, Presented and/or Displayed Peptides of a Target Antigen in the Context of an MHC molecule

In some embodiments, provided is a method for identifying a peptide epitope or peptide epitopes in the context of an MHC-E in which potential candidate peptide or peptides are contacted with the MHC-E molecule upon antigen processing of peptides derived from an exogenous or heterologous target antigen present in MHC-E-expressing cells. In some embodiments, nucleic acid encoding the target antigen or fragment thereof is transferred to the cell by introduction of an exogenous or heterologous nucleic acid encoding the target antigen or an immunogenic or antigenic portion thereof into the cell. In some embodiments, the MHC-E expressing cell containing the exogenous or heterologous target antigen or fragment thereof is cultured or incubated under conditions in which the target antigen is expressed and naturally processed peptides thereof are presented or displayed on the cell surface in the context of the MHC-E molecule.

As used herein, "nucleic acid" or "nucleic acid molecule" refers to any of deoxyribonucleic acid (DNA), ribonucleic acid (RNA) and analogs thereof, including peptide nucleic acids (PNA) and mixtures thereof. Nucleic acid molecules include those generated, for example, by the polymerase chain reaction (PCR) or by *in vitro* translation, and fragments generated by any of ligation, scission, endonuclease action, or exonuclease action. In certain embodiments, the nucleic acids can be produced by PCR. Nucleic acids may be composed of monomers that are naturally occurring nucleotides (such as deoxyribonucleotides and ribonucleotides), analogs of naturally occurring nucleotides (e.g., α-enantiomeric forms of naturally-occurring nucleotides), or a combination of both. Modified nucleotides can have modifications in or replacement of sugar moieties, or pyrimidine or purine base moieties. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. Nucleic acid molecules can be either single stranded or double stranded.

The term "exogenous," e.g. with reference to a nucleic acid or protein, refers to molecules which are not present in a cell and/or are introduced from outside a cell. The exogenous molecules can be transferred or introduced to the cells from outside. The exogenous molecules can include genetic material (such as DNA or RNA), proteins, peptides, or small molecules (e.g. dyes). In some cases, an exogenous molecule can be a molecule that is endogenous to the cell, but that is nevertheless introduced from outside the cell. In some cases, an exogenous molecule can be heterologous to the cell.

The term "heterologous," e.g. with reference to a nucleic acid or protein, refers to a molecule that is not normally present or produced in a cell, i.e. a molecule that is not normally endogenous to a cell into which it is introduced. Typically, the heterologous molecule is transferred or introduced to the cell from outside. In some embodiments, a heterologous molecule can refer to a molecule from another cell, such as from another organism, including the same species or another species. The heterologous molecules can include genetic material (such as DNA or RNA), proteins, peptides, or small molecules.

In some embodiments, the nucleic acid molecule is a synthetic nucleic acid. As used herein, "synthetic nucleic acid" refers to a nucleic acid that is not naturally occurring, but is man-made using such methods as chemical synthesis or recombinant DNA technology. In some embodiments, the synthetic DNA is complementary DNA (cDNA). In some embodiments, the synthetic nucleic acid is of human origin. In some embodiments, the synthetic DNA, such as cDNA, contains an open reading frame (ORF) encoding target antigen. The term ORF generally refers to the protein coding sequence of a gene from its start to its stop codon and excluding the 5' and 3' UTRs.

In some embodiments, libraries or collections of synthetic nucleic acid molecules, such as cDNA libraries, encoding a plurality of target antigens or portions thereof, are introduced into cells as a source of antigenic peptides. In some embodiments, such libraries can be constructed from a primary tumor sample, metastatic lesion or a tumor cell line. In some embodiments, multiple tumor samples, multiple metastatic lesions and/or multiple tumor cell lines are used in generating the library. In some embodiments, the library is generated from a plurality of subjects having the same tumor. Various cDNA library from primary specimens and cell lines are known and available from public or commercial sources, or can be generated using standard techniques.

In some embodiments, a synthetic nucleic acid library, such as a cDNA library, can be prepared by purifying or isolating messenger RNA, such as by using commercially available kits or reagents. Typically, poly(A)+ RNA is isolated from a total RNA preparation, such as by using oligo(dT)-cellulose chromatography. In some embodiments, double-stranded cDNA molecules can be synthesized from poly(A)+RNA using techniques well-known in the art, including using commercially available kits. In some embodiments, individual cDNA can be cloned into an expression vector. In some embodiments, amplification of the libraries is accomplished using techniques known to a skilled artisan, e.g. by plating, growth, elution, purification and concentration.

In some embodiments, individual nucleic acids can be inserted into any appropriate cloning vector, such as an expression vector, for transfer or introduction into a cell. A large number of vector-host systems known in the art can be used. Possible vectors include, but are not limited to, mammalian plasmids or viral vectors. Generally, the vector system is compatible with the host cell used. In some embodiments, the vector contains the necessary elements for the transcription and translation of the inserted protein coding sequence, such as for recombinant expression of one or more of the target antigens or fragments thereof in cells. In some embodiments, the cDNA can be inserted into the vector, such as by using standard recombinant DNA techniques. In some embodiments, the pools of vectors or plasmids can be transformed into bacteria to amplify the synthetic nucleic acid, e.g. cDNA. Pools of bacterial clones can be used to isolate the plasmid DNA, which can then be used for transfection or transduction of the cells that can process and express the protein derived from the inserted nucleic acid, e.g. cDNA. In some embodiments, nucleic acid encoding an MHC-E molecule and the nucleic acid encoding the target antigen or portion thereof are co-transfected together into a cell.

In some embodiments, corresponding libraries can be prepared from pooled normal counterparts, i.e. primary normal tissue or normal cell lines. In some embodiments, subtraction screening can be performed by comparing the profile from identified or detected peptide epitopes obtained from tumor-specific or control libraries, and peptide epitopes unique to a tumor identified.

In some embodiments, nucleic acid libraries, such as cDNA libraries, can be provided as orthogonal pools. In some cases, an orthogonal pooling strategy includes expressing a pool or mixture of nucleic acid molecules into the same MHC-E expressing cells. In some aspects, the identity of members of a pool is known. In some embodiments, by combining members into pools, the method can permit the expression and assessment of a large number of potentially antigenic molecules using a smaller number of samples. For example, in some cases, an orthogonal pool of nucleic acid molecules, such as a cDNA library prepared as orthogonal pool, can include a mixture desired pool size, typically 5 to 30, and generally up to 10 or up to 20 unique members. Typically, each member present in a particular pool also is present in one other pool, such that each nucleic acid molecule is present in two different pools. Generally, the identity of any hit is determined by deconvoluting the pools, which can occur by including each molecule in only two different pools so that a positive signal in both pools indicates that the particular molecule unique to only those pools is responsible for the positive read-out.

In some embodiments, the library of nucleic acid molecules is clonal, and each contains a single open reading frame (ORF) or coding sequence that is present as a single clonal isolate. In some embodiments, the nucleic acid is sequenced and the sequence of the clonal isolate is known. In some aspects, the nucleic acid library, such as cDNA library, is provided in an addressably arrayed format, such as a spatially addressable format, where each address or position (e.g. spatial address) of the array contains a unique or distinct exogenous or heterologous nucleic acid molecule, or in some cases a pool of such nucleic acid molecules, compared to one or more other addresses in the array. In some embodiments, a nucleic acid molecule containing a coding sequence or ORF or a pool of such nucleic acid molecules is/are distinct from nucleic acid molecules or pools of nucleic acid molecules present in other addresses in the array. In some embodiments, an MHC-E expressing cell is contacted with an array of synthetic nucleic acid molecules (e.g. cDNA library) where each nucleic acid molecule or pool of nucleic acid molecules is positioned in a spatial address of the array (e.g. well of a multiwell plate). In some cases, the identity of the synthetic nucleic acid sequence (e.g. cDNA molecule) in each address is known, whereby identification of a positive read-out of binding activity or immune reactivity at a particular address can be correlated to the particular heterologous or exogenous nucleic acid molecule (e.g. cDNA member) that is unique to the particular spatial address.

In some aspects, among the plurality of cell types that can be tested can include cells that contain a standard proteasome and/or an immunoproteasome. For example, in some cases, processing of peptides can differ in cells having a conventional proteasome versus an immunoproteasome or inflammasome. In particular, upon stimulation of cells, such as with interferon-gamma (IFNγ), the proteasome machinery can become structurally and/or functionally changed to produce an immunoproteasome, which can result in altered peptide processing. In some aspects, cells that contain an immunoproteasome are cells that are stimulated or activated, such as stimulated or activated with IFNγ.In some embodiments, the cells that have been contacted, exposed and/or incubated with one or a plurality of peptides, e.g. overlapping peptides or peptides processed from transferred antigen, can be further stimulated or activated, such as with a cytokine or other stimulating agent, such as with IFNγ.

### C. Identification and Assessment of Peptide Epitopes

In some embodiments, the methods provided herein include detecting and/or identifying a peptide epitope presented on the surface of cells in the context of an MHC-E molecule. In some embodiments, detecting and/or identifying a peptide epitope in the context of an MHC-E molecule includes extracting one or more peptides from a lysate of an MHC-E - expressing cell or eluting one or more peptides from the surface of a cell expressing an MHC-E molecule. In some embodiments, detecting and/or identifying a peptide epitope in the context of an MHC-E molecule involves isolating the MHC-E molecule or molecules, such as from the cell, and eluting the one or more peptides therefrom. In some embodiments, the method can further include determining if the identified peptide epitope in the context of the MHC-E molecule elicits an antigen-specific immune response. In some cases, assessing an antigen-specific immune response includes determining if an MHC-peptide complex on the surface of the cells is capable of inducing a T cell response, such as a cytotoxic (e.g. CD8+ ) T cell response. In some embodiments, a peptide epitope that is bound to or recognized by an MHC-E molecule and/or that is capable of inducing an immune response in the context of an MHC-E molecule can be isolated or purified. In some embodiments, the sequence of the peptide epitope is determined.

In some embodiments, recognition or binding of a peptide epitope or epitopes by an MHC-E molecule can be determined by elution of peptides from MHC followed by mass spectrometry sequencing. In some embodiments, the best binders can be selected for further characterization with regard to their reactivity with T cells. In some embodiments, reactivity of peptides to T cells in the context of MHC-E can be assessed directly. In some embodiments, potential candidate peptides are synthesized and individually contacted or exposed to an MHC-E molecule, such as an MHC-E-expressing cell, and screened using a biological assay as a read-out for immune activation. In some embodiments, assays for assessing an immune response include, but are not limited to, ELISPOT, ELISA, cellular proliferation, cytotoxic lymphocyte (CTL) assay or intracellular cytokine staining. In some embodiments, binding and/or immune reactivity to a given peptide within the library or mixture in any of the assays is an indication that an antigenic epitope is present. Exemplary assays for testing binding or induction of an immune response (e.g. CTL response) are described below. Such assays are known and have been used for identifying T cell epitopes, including from overlapping peptide libraries (see e.g. Martin (2003) Methods., 29:236-47; Giginat et al. (2001) J. Immunol., 166:1877-84; Mutch (1994) Scquir. Immune Defic. Syndr., 7:879-90; Karlsson (2003) J. Immunol. Methods, 283:141-53; Hunt (1992) Science, 255:1261-3).

In some embodiments, methods of detecting and/or identifying an MHC-E-peptide complex can include assessing for stabilization of MHC-E on the surface of cells (Terrazzano et al. (2007) Journal of Immunology, 179:372-381). In some embodiments, after contacting, incubating and/or exposing cells with the one or plurality of peptides, the cells can be recovered and analyzed for cell surface expression of MHC-E, such as by flow cytometry. A skilled artisan is familiar with stabilization assays and can empirically determine conditions for performing such assays. In an exemplary embodiment, cells are cultured with peptides, such as at a concentration of from or from about 10 µM to 1000 µM, such as 50 µM to 500 µM, e.g. about 100 µM. The cells can be cultured, such as contacted, exposed and/or incubated, with the peptides for about 1 hour to 24 hours, such as from or from about 12 hours to 18 hours. In some embodiments, surface expression of an MHC-E molecule can be determined with an anti-MHC-E-specific antibody, such as any known in the art, including the exemplary antibodies described herein. Control cells can also be assessed that were not contacted, incubated and/or exposed to the one or a plurality of peptides.

In some embodiments, methods of detecting and/or identifying an MHC-E- peptide complex can include isolation or separation of MHC molecules from the surface of a particular cell or cell line, such as a cell that has been contacted, exposed and/or incubated with potential candidate peptide epitopes according to the provided methods, and determining or assessing binding of a peptide thereto. For example, methods of detecting, isolating and/or identifying peptides can involve lysis of the cells, affinity purification of the MHC molecule from cell lysates and subsequent elution and analysis of peptides from the MHC (Falk et al. (1991) Nature, 351:290; Kowalewski and Stevanovic (2013). Biochemical Large-Scale Identification of Class I Ligands. In Antigen Processing: Methods and Protocols, Methods in Molecular Biology, vol. 960, Ch. 12 (pp. 145-157; and U.S. Patent No. 5,989,565). In some cases, affinity purification can involve immunoprecipitation or affinity chromatography. In some cases, one or more of ion exchange chromatography, lectin chromatography, size exclusion high performance liquid chromatography and a combination of any of the above can be used.

In some embodiments, immunoprecipitation is employed to isolate an MHC molecule, such as a particular MHC class or a particular MHC allele. Typically, immunoprecipitation methods utilize antibodies, such as monoclonal antibodies, that are specific for a particular MHC class or MHC allele. For example, in some aspects, allele specific antibodies can be used. In some cases, broadly reactive or monomorphic antibodies can be used that generally recognize more than one MHC allele, such as a particular MHC class or classes. It is within the level of a skilled artisan to choose the particular antibody depending on the particular MHC expressed by the cell and/or the specificity of MHC detection that is desired. Various anti-MHC antibodies, including anti-HLA antibodies, are well known in the art and available from commercial and private sources. Exemplary antibodies that can be used for immunoprecipitation of an MHC-E molecule are described in Table 3.

| **TABLE 3: ANTI-MHC ANTIBODY** | | |
|---|---|---|
| **Anti-HLA** | **Name** | **Source or Reference** |
| pan HLA-class I (e.g. HLA-A, -B, -C and HLA-E) | W6/32 | ATCC, HB95 |
| | B9.12.1 | Beckman Coulter, Cat. No. IM0107 |
| HLA-E | MEM-E/07 | LifeSpan Biosciences, Inc. Cat. No. LS-B3730 |
| HLA-E | MEM-E/08 | Abcam No. ab11821 |
| HLA-E | 3D12 | eBioscience (San Diego, CA), Cat. No. 12-9953 |
| HLA-E, HLA-C | DT9 | Merck Millipore (Billerica, MA) Cat. No. MABF233 |
| HLA-E | various | US2014/0010825; WO2014/008206; WO2012094252 |

In some embodiments, peptide fractions can be further separated from the MHC-peptide complex. In some embodiments, peptides can be dissociated from the MHC molecule by methods well known to a skilled artisan, for example, by exposure of the complex to any of a variety of denaturing method, such as heat, pH, detergents, salts, chaotropic agent or combination thereof. For example, in some embodiments, following isolation, the peptides bound to the peptide binding groove of the isolated MHC molecules can be eluted, such as using acid treatment.

In some embodiments, peptide fractions can be further separated from the MHC molecules by reverse-phase high performance liquid chromatography (HPLC) and sequenced. In some embodiments, peptides can be separated by other methods well known to a skilled artisan, such as filtration, ultrafiltration, electrophoresis, and size chromatography, precipitation with specific antibodies, ion exchange chromatography or isoelctrofocusing. In some embodiments, eluted peptides can be analyzed by mass spectrometry (MS), liquid chromatography MS (LC-MS), tandem MS (LC-MS/MS), or MALDI-MS.

In some embodiments, peptides bound to MHC molecules on the surface of cells can be separated or isolated by acid extraction and HPLC separation. For example, cells can be acidified to a pH of about 2 ± 0.5 to 3 ± 0.5, such as with trifluoroacetic acid, citrate phosphate buffer, or other suitable acidic buffer. In some cases, acid-treated cells can be homogenized and peptides eluted into the supernatant following centrifugation. In some cases, low molecular weight compounds can be extracted or obtained from the supernatant by methods that can include size exclusion chromatography, solid-phase extraction, vacuum centrifugation and a combination thereof. In some cases, separation of peptides can be performed by HPLC and peptides can be eluted as different fractions by adjusting the flow rate, type of gradient and other parameters known to a skilled artisan.

In some embodiments, subtractive methods can be performed by performing immunoaffinity purification and peptide elution on control cells that were not contacted, exposed to and/or incubated with the candidate peptide or peptide epitopes (e.g. from an overlapping peptide library or synthetic of cDNA) and/or control cells that may express an MHC molecule other than or in addition to an MHC-E molecule. In some embodiments, the control cells are cells introduced with an empty vector that do not contain the nucleic acid molecule encoding the target antigen. In some embodiments, the control cells are cells that are incubated in the absence of contacting, exposure and/or incubation with candidate peptides or peptide epitopes. In some embodiments, the control cells are cells that express a classical MHC class I molecule (e.g. HLA-A, -B or -C) instead of or in addition to expressing an MHC-E molecule. In some embodiments, the peptides from the test and control cell samples can be compared, such as by mass spectrometry. In some embodiments, only the peptide(s) contained in the profile of test MHC-E-expressing cells contacted, exposed to and/or incubated with the candidate peptide or peptide epitopes are used to identify peptide epitopes, such as by subsequent sequencing.

In some embodiments, a peptide epitope that is bound to or recognized by an MHC-E molecule can be isolated or purified. In some embodiments, the sequence of the peptide epitope is determined.

In some embodiments, the isolated peptides can be sequenced. In some embodiments, sequencing of the isolated peptides can be performed according to standard techniques such as Edman degradation. In some embodiments, mass spectrometry sequencing of individual peptides can be performed. In some embodiments, the sequenced peptides can be compared to the sequence of the target antigen, and particular peptides identified that are present in the target antigen.

In some embodiments, the peptide can be prepared, such as for further analysis or testing. In some embodiments, the peptide can be synthesized in solution or on a solid support using techniques known to in the art. In some embodiments, peptides can be synthesized using an automated peptide synthesizer. Various automatic synthesizers are commercially available and can be used in accordance with known protocols In some embodiments, the peptides can be manually synthesized. Methods for peptide synthesis are known or described in the art, see, e.g., See, e.g., Stewart and Young, Solid Phase Peptide Synthesis, 2d. ed., Pierce Chemical Co., Rockford, IL (1984); Hunkapiller et al., (1984) Nature, 310:105-11; Bodanszky, Principles of Peptide Synthesis, Springer Verlag (1984).

In some embodiments, peptides can be isolated and purified prior to contacting with an MHC-E molecule. In some embodiments, suitable methods for purification or isolation include, for example, chromatography (e.g., ion exchange chromatography, affinity chromatography, sizing column chromatography, high pressure liquid chromatography), centrifugation, differential solubility or other suitable technique for the purification of peptides or proteins. In some embodiments, the peptides can be labeled (e.g. with a radioactive label, a luminescent label, a chemi-luminescent label or an affinity tag), such as to facilitate purification, isolation and/or assessment of an activity (e.g. binding).

In some embodiments, the binding affinity of the peptide epitope is determined. Methods of determining affinity of a peptide for an MHC molecule are well known in the art (see e.g., in PCT publications WO 94/20127 and WO 94/03205). In some embodiments, binding assays can involve evaluation of peptide binding to purified MHC molecules in relation to the binding of a radioiodinated reference peptide. Alternatively, cells expressing empty MHC molecules (i.e. cell surface HLA molecules that lack any bound peptide) may be evaluated for peptide binding by immunofluorescent staining and flow microfluorimetry. Other assays that may be used to evaluate peptide binding include peptide-dependent class I assembly assays and/or the inhibition of CTL recognition by peptide competition. In some cases, binding may also be determined using other assay systems including those using: live cells (e.g., Ceppellini et al., Nature 339:392 (1989); Christnick et al., Nature 352:67 (1991); Busch et al., Int. Immunol. 2:443 (1990); Hill et al.., J Immunol. 147:189 (1991); del Guercio et al., J Immunol. 154:685 (1995)), cell free systems using detergent lysates (e.g., Cerundolo et al., J Immunol. 21 :2069 (1991)), immobilized purified MHC (e.g., Hill et al., J Immunol. 152,2890 (1994); Marshall et al., J Immunol. 152:4946 (1994)), ELISA systems (e.g., Reay et al., EMBO J 11 :2829 (1992)), surface plasmon resonance (e.g., Khilko et al., J Biol. Chem. 268:15425 (1993)); high flux soluble phase assays (Hammer et al., J. Exp. Med. 180:2353 (1994) and ELISA-based refolding assays using denatured MHC molecules (Sylvester-Hyid et al. (2002) Tissue Antigens, 59:251-8)).

In some embodiments, affinity is determined by stabilization assays based on the ability of a peptide to stabilize an MHC class I molecule, such as an MHC-E molecule, expressed on the cell surface. In some cases, a TAP-deficient cell line, such as a T2, K562 or RMA-S, can be transfected with an MHC allele of interest. A stabilized MHC class I complex can be detecting using an anti-MHC antibody, such as a pan-MHC class I antibody, in any of a variety of assays, such as by flow cytometry. In some cases, binding can be assessed or compared in relation to a non-binding negative control.

In some embodiments, affinity is determined using a peptide-dependent refolding assay (Strong et al. (2003) J Biol. Chem., 278:5082-5090). For example, in an exemplary embodiment, refolding of an MHC class I molecule, such as MHC-E, can be assessed in the presence of β2m, heavy chain and peptide at various concentrations, which can be incubated for an appropriate time for refolding to occur (e.g. 30 minutes to 3 hours (e.g. about 1 to 2 hours) at or about between 4 °C and 8, and in some cases at room temperature, e.g. between or about between 21 °C and 25 °C). Refolded MHC, e.g. MHC-E, can be detected using an MHC, e.g., MHC-E, specific antibody, such as in a sandwich ELISA or other similar method. In some embodiments, the relative amount of MHC refolded in the presence or absence of various concentrations of peptide can be compared to a standard. For example, for MHC-E refolding can be compared to assembly achieved using a nonamer peptide known to bind MHC-E (e.g. HLA-B7 nonamer; VMAPRTLVL, SEQ ID NO: 6). In some embodiments, relative binding affinity can be determined based on peptide concentrations yielding half maximal assembly.

In some embodiments, binding affinity is determined using a competition assay, such as a competition radioimmunoassay, with a known or reference peptide. For example, in some aspects, relative affinities can be determined by comparison of escalating concentrations of the test peptide versus a known or reference binding peptide. In some embodiments, the IC50 for binding can be determined, which is the concentration of peptide in a binding assay at which 50% inhibition of binding of a known or reference peptide is observed. In some cases, such as depending on the conditions in which the assays are run (i.e. limiting MHC proteins and labeled peptide concentrations), these values can approximate K_{D} values. In some embodiments, binding can be expressed relative to a reference or known peptide.

In some embodiments, a peptide can be assessed for binding to MHC on the surface of cells of a specific MHC type, such as an engineered cell line, PBMCs, leukemia cells lines or EBV-transformed T cell lines. In some embodiment, binding assays, such as competition assays, can be performed with excess unlabeled peptide known to bind to the same or disparate MHC molecules. In some embodiments, binding can be assessed to cells that express the same or disparate MHC types. In some embodiments, a peptide can be tested for binding to other MHC molecules of the same supertype. In some embodiments, the specificity and/or selectivity for binding to a particular MHC or MHC allele can be determined.

In some embodiments, the peptide has an affinity for binding an MHC that is high, intermediate or low affinity. Typically, "high affinity" with respect to an MHC class I molecule is defined as binding with an IC₅₀, or K_{D} value, of 50 nM or less, "intermediate affinity" with respect to MHC class I molecules is defined as binding with an IC₅₀ or K_{D} value of between about 50 and about 500 nM and "low affinity" with respect to MHC class I molecules is defined as binding with an IC₅₀ or K_{D} value of greater than 500 nM, such as generally between about 500 nM and about 5000 nM. For MHC class II molecules, typically, "high affinity" with respect to binding to MHC class II molecules is defined as binding with an IC₅₀ or K_{D} value of 100 nM or less; "intermediate " affinity with respect to binding to MHC class II molecules is defined as binding with an IC₅₀ or K_{D} value of between about 100 and about 1000 nM, and "low affinity" with respect to binding to MHC class II molecules is defined as binding with an IC₅₀ or K_{D} value of greater than 1000 nM, such as generally between about 1000 nM and about 5000 nM.

In some embodiments, a peptide epitope can include peptides having an affinity for an MHC molecule, such as an MHC-E molecule, with an IC₅₀ or K_{D} value of less than or about less than 5000 nM, 4000 nM, 3000 nM, 2000 nM, 1000 nM, 900 nM, 800 nM, 700 nM, 600 nM, 500 nM, 400 nM, 300 nM, 200 nM, 100 nM or less. In some embodiments, the binding affinity is intermediate or low affinity. For example, in some embodiments, the peptide has a binding affinity for an MHC molecule, such as an MHC-E molecule, with an IC₅₀ or K_{D} value of greater than 50 nM or 100 nM or greater, such as greater than 200 nM, 500 nM or 1000 nM, but generally less than 5000 nM.

In some embodiments, methods of detecting and/or identifying a peptide epitope includes assessing if a peptide displayed in the context of an MHC-E molecule on the surface of a cell, is a T cell epitope capable of inducing an immune response.

In some embodiments, after detecting and/or identifying a peptide epitope that is bound to an MHC-E molecule, such as using any of the procedures described above, the provided methods further include testing a T cell response to the peptide, such as a purified or isolated peptide. In some embodiments, a peptide epitope that elicits a T cell response can be identified.

In some embodiments, the peptide can be verified to be an immune epitope by assessing functional activity of the peptide to induce a helper or cell-mediated immune response. In some embodiments, the peptide can be assessed for its ability to serve as a target for a cytotoxic T lymphocytes (CTLs) derived from healthy subjects primed *in vitro* with the peptide or derived from infected or diseased (e.g. tumor-bearing) subjects. In some embodiments, CTL activity can be assessed using tumor-specific CTL clones. Such assays can be performed in vitro or in vivo. In some embodiments, methods of detecting T cell responses include proliferation assays, lymphokine secretion assays, direct cytotoxicity assays, and limiting dilution assays.

In some embodiments, antigen-presenting cells matching the HLA restriction of the peptide (e.g. MHC-E expressing cells) can be incubated with a peptide and assayed for the ability to induce CTL responses in responder cell populations. In some embodiments, such responses are induced by incubating in vitro, such as in tissue culture, CTL precursor lymphocytes together with a source of antigen presenting cells and the peptide. In some cases, antigen-presenting cells can be peripheral blood mononuclear cells, macrophages, dendritic cells or activated B cells. In some embodiments, cells engineered or transfected with an MHC molecule can be used. In some cases, peripheral blood mononuclear cells (PBMCs) or CD8+ cells can be used as the source of CTL precursors or responder cells. In some embodiments, PBMCs are fractionated to obtain a source of antigen-presenting cells and autologous T cells, such as CD8+ T cells. Alternatively, the antigen presentation system may comprise a particular T cell line/clone and/or a particular antigen presenting cell type. Many *in vitro* CTL stimulation protocols have been described and the choice of which one to use is well within the knowledge of the skilled artisan.

In some embodiments, antigen-presenting cells can be incubated with peptide, after which the peptide-loaded antigen-presenting cells are then incubated with the responder cell population under optimized culture conditions. In some embodiments, the peptide is provided at concentrations between 10 and 40 µg/ml. In some embodiments, the peptide is pre-incubated with the antigen presenting cells for periods ranging from 1 to 18 hrs. In some embodiments, β2-microglobulin (e.g. 4 µg/ml) can be added during this time period to enhance binding. In some embodiments, the antigen presenting cells can be held at room temperature during the incubation period (Ljunggren, H.-G. et al, Nature, 346:476-480, (1990)) or pretreated with acid (Zeh, H. J., Ill et al., Hum. Immunol., 39:79-86, (1994)) to promote the generation of denatured MHC molecules that can then bind the peptide. Following peptide loading of antigen-presenting cells, the precursor CTLs (responders) can be added to the antigen-presenting cells to which the peptide has bound (stimulators), for example at a responder to stimulator ratio of between 5:1 and 50:1, such as between 10:1 and 20:1. In some aspects, the co-cultivation of cells is carried out under conditions in which CTL responder cells can be generated, i.e. under conditions to prime CD8+ cells. For example, in some embodiments, the co-cultivation is carried out in the presence of IL-2 or other stimulatory cytokines, such as IL-1, IL-7 and IL-12. In an exemplary embodiment, the co-cultivation of the cells is carried out at 37° C in RPMI 1640, 10% fetal bovine serum, 2 mM L-glutamine, and IL-2 (5-20 Units/ml), and optionally, with the addition of one or more of IL-1, IL7 or IL-12. In some embodiments, fresh IL-2-containing media is added to the cultures every 2-4 days, for example by removing one-half the old media and replenishing it with an equal volume of fresh media. In some embodiments, after 7-10 days, and generally every 7-10 days thereafter, the CTL are re-stimulated with antigen-presenting cells to which peptide has been bound as described above. In some embodiments, fresh IL-2- containing media is added to the cells throughout their culture as described above. In some embodiments, three to four rounds of stimulation, and sometimes as many five to eight rounds of stimulation, can be required to generate a CTL response that can then be measured in vitro. In some embodiments, the peptide-specific CTL can be further expanded to large numbers by treatment with anti-CD3 antibody. For example, see (Riddell, S. R. and Greenberg, P. D., J. Immunol. Methods, 128: 189-201, (1990); Walter, E. A. et al., N. Engl. J. Med., 333: 1038-1044, (1995)).

In some embodiments, CTL activity can be assessed directly from PBMCs isolated from infected or diseased subjects, such as tumor or cancer-bearing subjects, without *in vitro* priming (see e.g. Bredenbeck et al. (2005) J. Immunol., 174:6716-6724). For example, in some embodiments, peptide can be added directly to PBMC cells isolated from peripheral blood of the subject. In some cases, as a control, PBMCs without peptide can be assessed for CTL activity from the same subject. In some embodiments, the cancer is known or likely to express the tumor antigen from which the peptide identified by the provided method has been derived. In some embodiments, the subject has a sarcoma, melanoma, breast carcinoma, renal carcinoma, lung carcinoma, ovarian carcinoma, prostate carcinoma, colorectal carcinoma, pancreatic carcinoma, squamous tumor of the head and neck, or squamous carcinoma of the lung.

In some embodiments, CTL clones, such as tumor-specific CTL clones, can be utilized to assess CTL activity. Methods for generating CTL clones are known to a skilled artisan. In an exemplary embodiment, CTL clones can be obtained by stimulation of CD8+ T cells with antigen in the presence of antigen-presenting cells followed by continued antigen-specific expansion of antigen-specific CD8+ T cells to generate CTL lines of clones.

In some embodiments, the CTL activation can be determined. A variety of techniques exist for assaying the activity of CTL. In some embodiments, CTL activity can be assessed by assaying the culture for the presence of CTLs that lyse radio-labeled target cells, such as specific peptide-pulsed targets. These techniques include the labeling of target cells with radionuclides such as Na₂, ⁵¹CrO₄ or ³H-thymidine, and measuring the release or retention of the radionuclides from the target cells as an index of cell death. In some embodiments, CTL are known to release a variety of cytokines when they are stimulated by an appropriate target cell, such as a tumor cell expressing the relevant MHC molecule and the corresponding peptide, and the presence of such epitope-specific CTLs can be determined by measuring cytokine release. Non-limiting examples of such cytokines include IFN-γ, TNF-α, and GM-CSF. Assays for these cytokines are well known in the art, and their selection is left to the skilled artisan. Methodology for measuring both target cell death and cytokine release as a measure of CTL reactivity are given in Coligan, J. E. et al. (Current Protocols in Immunology, 1999, John Wiley & Sons, Inc., New York).

In some embodiments, alternatively, a peptide epitope can be identified based on its ability to stimulate an immune response or induce T cell reactivity, such as to induce a helper or cell-mediated immune response. Thus, in some embodiments, the methods can be performed without the need to first determine if a peptide is bound by and/or eluted from a particular MHC molecule. For example, in some embodiments, methods of detecting and/or identifying a peptide epitope includes assessing or determining if a peptide displayed in the context of an MHC molecule on the surface of a cell, such as a cell that has been contacted, exposed and/or incubated with potential candidate peptide epitopes according to the provided methods, is a T cell epitope capable of inducing an immune response. Such resulting peptide-expressing cells can be directly used as the peptide source to assess stimulation of responder or effector cells, such as whole peripheral blood mononuclear cells (PBMCs) or CD8+ T cells, either obtained from healthy or infected or diseased (e.g. tumor-bearing subjects) or T cell clones. A cytotoxic T cell response can be assessed using methods known in the art, including any described above. In some embodiments, if a T cell response is assessed, the peptide can be identified, isolated or purified, such as by immunoaffinity and elution methods described above. In some embodiments, cells that were not contacted, exposed to and/or incubated with the candidate peptide epitopes can be used as a control.

### III. Methods of Identifying Molecules that Bind to a Peptide in the Context of an MHC-Molecule

Provided are methods of identifying and/or generating antigen-binding molecules that bind to a peptide epitope displayed in the context of an MHC molecule, i.e. an MHC-peptide complex. In some embodiments, the peptide epitope is any identified using the provided methods. In some embodiments, the peptide epitope is a universal, supertope and/or non-canonical peptide epitope. In some embodiments, the peptide binding molecule, i.e. MHC-peptide binding molecule, is a molecule or portion thereof that possesses the ability to bind, e.g. specifically bind, to a peptide epitope that is presented or displayed in the context of an MHC molecule (MHC-peptide complex), such as on the surface of a cell. In some embodiments, a binding molecule may include any naturally occurring, synthetic, semi-synthetic, or recombinantly produced molecule that can bind, e.g. specifically bind, to an MHC-peptide complex. Exemplary peptide binding molecules include T cell receptors or antibodies, or antigen-binding portions thereof, including single chain immunoglobulin variable regions (e.g., scTCR, scFv) thereof, that exhibit specific ability to bind to an MHC-peptide complex. In some embodiments, the peptide binding molecule is a TCR or antigen binding fragment thereof. In some embodiments, the peptide binding molecule is a TCR-like CAR that contains an antibody or antigen binding fragment thereof, such as a TCR-like antibody, such as one that has been engineered to bind to MHC-peptide complexes. In some embodiments, the peptide binding molecule can be derived from natural sources, or it may be partly or wholly synthetically or recombinantly produced.

In some embodiments, the peptide binding molecule binds, such as specifically binds, to a peptide epitope, e.g., in complex with an MHC-E molecule, with an affinity or K_{A} (*i.e.*, an equilibrium association constant of a particular binding interaction with units of 1/M) equal to or greater than 10⁵ M⁻¹ (which equals the ratio of the on-rate [kₒₙ] to the off-rate [k_{off}] for this association reaction). In some embodiments, the TCR (or other peptide binding molecule) exhibits a binding affinity for a T cell epitope of the target polypeptide with an association constant K_{A} or half-life ranging from or from about 10⁶ M⁻¹ to 10¹⁰ M⁻¹, such as from or from about 10⁶ M⁻¹ to 10⁸ M⁻¹. In some embodiments, binding affinity may be classified as high affinity or as low affinity. For example, in some cases, a binding molecule (e.g. TCR) that exhibits high affinity binding to a particular epitope interacts with such epitope with a K_{A} of at least 10⁷ M⁻¹, at least 10⁸ M⁻¹, at least 10⁹ M⁻¹, at least 10¹⁰ M⁻¹, at least 10¹¹ M⁻¹, at least 10¹² M⁻¹, or at least 10¹³ M⁻¹. In some cases, a binding molecule (e.g. TCR) that exhibits low affinity binding exhibits a K_{A} of up to 10⁷ M⁻¹, up to 10⁶ M⁻¹, up to 10⁵ M⁻¹. Alternatively, affinity can be defined as an equilibrium dissociation constant (K_{D}) of a particular binding interaction with units of M (*e.g.,* 10⁻⁵ M to 10⁻¹³ M). In some embodiments, the identified peptide binding molecule exhibits a binding affinity for the peptide in the context of an MHC-E molecule with a K_{D} that is 10⁻⁵ M to 10⁻¹³ M, 10⁻⁵ M to 10⁻⁹ M, or 10⁻⁷ M to 10⁻¹² M, such as less than or less than about 10⁻⁵ M, 10⁻⁶ M, 10⁻⁷ M, 10⁻⁸ M, 10⁻⁹M, 10⁻¹⁰ M, 10⁻¹¹ M, 10⁻¹² M, 10⁻¹³ M or less.

Typically, specific binding of a peptide binding molecule to a peptide epitope, e.g. in complex with an MHC-E, is governed by the presence of an antigen-binding site containing one or more complementarity determining regions (CDRs). In general, it is understood that specifically binds does not mean that the particular peptide epitope, e.g. in complex with an MHC-E, is the only thing to which the MHC-peptide molecule may bind, since non-specific binding interactions with other molecules may also occur. In some embodiments, binding of a peptide binding molecule to an MHC-peptide complex is with a higher affinity than binding to such other molecules, e.g. molecules other than an MHC-peptide complex or an irrelevant (control) MHC-peptide complex, such as at least about 2-fold, at least about 10-fold, at least about 20-fold, at least about 50-fold, or at least about 100-fold higher than binding affinity to such other molecules.

In some embodiments, a binding molecule that binds to a peptide epitope can be identified by contacting one or more candidate binding molecules, such as one or more candidate TCR molecules, antibodies or antigen-binding fragments thereof, with an MHC-peptide complex, and assessing whether each of the one or more candidate binding molecules binds, such as specifically binds, to the MHC-peptide complex. The methods can be performed in vitro, ex vivo or in vivo.

In some embodiments, screening methods can be employed in which a plurality of candidate binding molecules, such as a library or collection of candidate binding molecules, are individually contacted with a peptide binding molecule, either simultaneously or sequentially. Library members that specifically bind to a particular MHC-peptide complex can be identified or selected. In some embodiments, the library or collection of candidate binding molecules can contain at least 2, 5, 10, 100, 10³, 10⁴, 10⁵, 10⁶, 10⁷, 10⁸, 10⁹, or more different peptide binding molecules.

In some embodiments, an antibody or antigen-binding portion thereof that binds, such as specifically binds, to an MHC-peptide complex can be produced by immunizing a host with an effective amount of an immunogen containing a particular MHC-peptide complex. In some embodiments, the antibody or portion thereof can be isolated from the host and binding to the MHC-peptide complex assessed to confirm specific binding thereto.

A variety of assays are known for assessing binding affinity and/or determining whether a binding molecule specifically binds to a particular ligand (e.g. MHC-peptide complex). It is within the level of a skilled artisan to determine the binding affinity of a TCR for a T cell epitope of a target polypeptide, such as by using any of a number of binding assays that are well known in the art. For example, in some embodiments, a BIAcore machine can be used to determine the binding constant of a complex between two proteins. The dissociation constant for the complex can be determined by monitoring changes in the refractive index with respect to time as buffer is passed over the chip. Other suitable assays for measuring the binding of one protein to another include, for example, immunoassays such as enzyme linked immunosorbent assays (ELISA) and radioimmunoassays (RIA), or determination of binding by monitoring the change in the spectroscopic or optical properties of the proteins through fluorescence, UV absorption, circular dichroism, or nuclear magnetic resonance (NMR). Other exemplary assays include, but are not limited to, Western blot, ELISA, analytical ultracentrifugation, spectroscopy and surface plasmon resonance (Biacore^{®}) analysis (*see, e.g.,* Scatchard et al., Ann. N.Y. Acad. Sci. 51:660, 1949; Wilson, Science 295:2103, 2002; Wolff et al., Cancer Res. 53:2560, 1993; and U.S. Patent Nos. 5,283,173, 5,468,614, or the equivalent), flow cytometry, sequencing and other methods for detection of expressed nucleic acids. In one example, apparent affinity for a TCR is measured by assessing binding to various concentrations of tetramers, for example, by flow cytometry using labeled tetramers. In one example, apparent K_{D} of a TCR is measured using 2-fold dilutions of labeled tetramers at a range of concentrations, followed by determination of binding curves by non-linear regression, apparent K_{D} being determined as the concentration of ligand that yielded half-maximal binding.

In some embodiments, the methods can be used to identify binding molecules that bind only if the particular peptide is present in the complex, and not if the particular peptide is absent or if another, non-overlapping or unrelated peptide is present. In some embodiments, the binding molecule does not substantially bind the MHC in the absence of the bound peptide, and/or does not substantially bind the peptide in the absence of the MHC. In some embodiments, the binding molecules are at least partially specific. In some embodiments, an exemplary identified binding molecule may bind to an MHC-peptide complex if the particular peptide is present, and also bind if a related peptide that has one or two substitutions relative to the particular peptide is present.

### A. Binding Molecules and Libraries

In some embodiments, the peptide binding molecule is a TCR or antigen binding fragment thereof. In some embodiments, the peptide binding molecule is antibody or antigen binding fragment thereof, such as a TCR-like antibody. In some embodiments, the peptide binding molecule can be derived from natural sources, or may be partly or wholly synthetically or recombinantly produced.

In some embodiments, one or more binding molecules are assessed for binding to a particular peptide epitope, such as a peptide epitope identified using any of the above described methods.

In some embodiments, a library containing a plurality of variants of a binding molecule, such as a TCR or antibody or antigen-binding fragment thereof, can be generated.

In some embodiments, a library or collection containing binding molecules each having a sequence present in the genome of a subject can be generated and assessed for binding. In some embodiments, a library or collection of binding molecules in which one or more members have been evolved, randomized and/or mutagenized, such as by directed evolution methods, can be generated and assessed for binding.

### 1. T cell Receptor (TCR)

In aspects of the provided method, the peptide binding molecule is a T cell receptor (TCR) or an antigen-binding fragment thereof. In some embodiments, a "T cell receptor" or "TCR" is a molecule that contains a variable α and β chains (also known as TCRα and TCRβ, respectively) or a variable γ and δ chains (also known as TCRγ and TCRδ, respectively), or antigen-binding portions thereof, and which is capable of specifically binding to a peptide bound to an MHC molecule. In some embodiments, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some embodiments, the TCR is an intact or full-length TCR, including TCRs in the αβ form or γδ form. In some embodiments, the TCR is an antigen-binding portion that is less than a full-length TCR but that binds to a specific peptide bound in an MHC molecule, such as binds to an MHC-peptide complex. In some cases, an antigen-binding portion or fragment of a TCR can contain only a portion of the structural domains of a full-length or intact TCR, but yet is able to bind the peptide epitope, such as MHC-peptide complex, to which the full TCR binds. In some cases, an antigen-binding portion contains the variable domains of a TCR, such as variable α chain and variable β chain of a TCR, sufficient to form a binding site for binding to a specific MHC-peptide complex. Generally, the variable chains of a TCR contain complementarity determining regions (CDRs) involved in recognition of the peptide, MHC and/or MHC-peptide complex.

In some embodiments, the variable domains of the TCR contain hypervariable loops, or CDRs, which generally are the primary contributors to antigen recognition and binding capabilities and specificity. In some embodiments, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (*see, e.g.,* Jores et al., Proc. Nat'l Acad. Sci. U.S.A. 87:9138, 1990; Chothia et al., EMBO J. 7:3745, 1988; *see also* Lefranc et al., Dev. Comp. Immunol. 27:55, 2003). In some embodiments, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some embodiments, the variable region of the β-chain can contain a further hypervariable region (CDR4 or HVR4), which generally is involved in superantigen binding and not antigen recognition (Kotb (1995) Clinical Microbiology Reviews, 8:411-426).

In some embodiments, a TCR contains a variable alpha domain (V_{α}) and/or a variable beta domain (V_{β}) or antigen-binding fragments thereof. In some embodiments, the α-chain and/or β-chain of a TCR also can contain a constant domain, a transmembrane domain and/or a short cytoplasmic tail (*see, e.g.,* Janeway et al., Immunobiology: The Immune System in Health and Disease, 3rd Ed., Current Biology Publications, p. 4:33, 1997). In some embodiments, the α chain constant domain is encoded by the TRAC gene (IMGT nomenclature) or is a variant thereof. In some embodiments, the β chain constant region is encoded by TRBC1 or TRBC2 genes (IMGT nomenclature) or is a variant thereof. In some embodiments, the constant domain is adjacent to the cell membrane. For example, in some cases, the extracellular portion of the TCR formed by the two chains contains two membrane-proximal constant domains, and two membrane-distal variable domains, which variable domains each contain CDRs.

It is within the level of a skilled artisan to determine or identify the various domains or regions of a TCR. In some aspects, residues of a TCR are known or can be identified according to the International Immunogenetics Information System (IMGT) numbering system (see e.g. www.imgt.org; see also, Lefranc et al. (2003) Developmental and Comparative Immunology, 2&;55-77; and The T Cell Factsbook 2nd Edition, Lefranc and LeFranc Academic Press 2001). Using this system, the CDR1 sequences within a TCR Vα chains and/or Vβ chain correspond to the amino acids present between residue numbers 27-38, inclusive, the CDR2 sequences within a TCR Vα chain and/or Vβ chain correspond to the amino acids present between residue numbers 56-65, inclusive, and the CDR3 sequences within a TCR Vα chain and/or Vβ chain correspond to the amino acids present between residue numbers 105-117, inclusive.

In some embodiments, the TCR may be a heterodimer of two chains α and β (or optionally γ and δ) that are linked, such as by a disulfide bond or disulfide bonds. In some embodiments, the constant domain of the TCR may contain short connecting sequences in which a cysteine residue forms a disulfide bond, thereby linking the two chains of the TCR. In some embodiments, a TCR may have an additional cysteine residue in each of the α and β chains, such that the TCR contains two disulfide bonds in the constant domains. In some embodiments, each of the constant and variable domains contains disulfide bonds formed by cysteine residues.

In some embodiments as described, the TCR can contain an introduced disulfide bond or bonds. In some embodiments, the native disulfide bonds are not present. In some embodiments, the one or more of the native cysteines (e.g. in the constant domain of the α chain and β chain) that form a native interchain disulfide bond are substituted to another residue, such as to a serine or alanine. In some embodiments, an introduced disulfide bond can be formed by mutating non-cysteine residues on the alpha and beta chains, such as in the constant domain of the α chain and β chain, to cysteine. Exemplary non-native disulfide bonds of a TCR are described in published International PCT No. WO2006/000830 and WO2006037960. In some embodiments, cysteines can be introduced at residue Thr48 of the α chain and Ser57 of the β chain, at residue Thr45 of the α chain and Ser77 of the β chain, at residue Tyr10 of the α chain and Ser17 of the β chain, at residue Thr45 of the α chain and Asp59 of the β chain and/or at residue Ser15 of the α chain and Glu15 of the β chain. In some embodiments, the presence of non-native cysteine residues (e.g. resulting in one or more non-native disulfide bonds) in a recombinant TCR can favor production of the desired recombinant TCR in a cell in which it is introduced over expression of a mismatched TCR pair containing a native TCR chain.

In some embodiments, the TCR chains contain a transmembrane domain. In some embodiments, the transmembrane domain is positively charged. In some cases, the TCR chain contains a cytoplasmic tail. In some aspects, each chain (e.g. alpha or beta) of the TCR can possess one N-terminal immunoglobulin variable domain, one immunoglobulin constant domain, a transmembrane region, and a short cytoplasmic tail at the C-terminal end. In some embodiments, a TCR, for example via the cytoplasmic tail, is associated with invariant proteins of the CD3 complex involved in mediating signal transduction. In some cases, the structure allows the TCR to associate with other molecules like CD3 and subunits thereof. For example, a TCR containing constant domains with a transmembrane region may anchor the protein in the cell membrane and associate with invariant subunits of the CD3 signaling apparatus or complex. The intracellular tails of CD3 signaling subunits (e.g. CD3y, CD3δ, CD3ε and CD3ζ chains) contain one or more immunoreceptor tyrosine-based activation motif or ITAM that are involved in the signaling capacity of the TCR complex.

In some embodiments, the TCR or antigen binding portion thereof may be a recombinantly produced natural protein or mutated form thereof in which one or more property, such as binding characteristic, has been altered. In some embodiments, a TCR may be derived from one of various animal species, such as human, mouse, rat, or other mammal.

In some embodiments, the TCR is a full-length TCR. In some embodiments, the TCR is an antigen-binding portion. In some embodiments, the TCR is a dimeric TCR (dTCR). In some embodiments, the TCR is a single-chain TCR (sc-TCR). A TCR may be cell-bound or in soluble form. In some embodiments, for purposes of the provided methods, the TCR is in cell-bound form expressed on the surface of a cell.

In some embodiments a dTCR contains a first polypeptide wherein a sequence corresponding to a TCR α chain variable region sequence is fused to the N terminus of a sequence corresponding to a TCR α chain constant region extracellular sequence, and a second polypeptide wherein a sequence corresponding to a TCR β chain variable region sequence is fused to the N terminus a sequence corresponding to a TCR β chain constant region extracellular sequence, the first and second polypeptides being linked by a disulfide bond. In some embodiments, the bond can correspond to the native interchain disulfide bond present in native dimeric αβ TCRs. In some embodiments, the interchain disulfide bonds are not present in a native TCR. For example, in some embodiments, one or more cysteines can be incorporated into the constant region extracellular sequences of dTCR polypeptide pair. In some cases, both a native and a non-native disulfide bond may be desirable. In some embodiments, the TCR contains a transmembrane sequence to anchor to the membrane.

In some embodiments, a dTCR contains a TCR α chain containing a variable α domain, a constant α domain and a first dimerization motif attached to the C-terminus of the constant α domain, and a TCR β chain comprising a variable β domain, a constant β domain and a first dimerization motif attached to the C-terminus of the constant β domain, wherein the first and second dimerization motifs easily interact to form a covalent bond between an amino acid in the first dimerization motif and an amino acid in the second dimerization motif linking the TCR α chain and TCR β chain together.

In some embodiments, the TCR is a scTCR, which is a single amino acid strand containing an α chain and a β chain that is able to bind to MHC-peptide complexes. Typically, a scTCR can be generated using methods known to those of skill in the art, See e.g., International published PCT Nos. WO 96/13593, WO 96/18105, WO99/18129, WO 04/033685, WO2006/037960, WO2011/044186; U.S. Patent No. 7,569,664; and Schlueter, C. J. et al. J. Mol. Biol. 256, 859 (1996).

In some embodiments, a scTCR contains a first segment constituted by an amino acid sequence corresponding to a TCR α chain variable region, a second segment constituted by an amino acid sequence corresponding to a TCR β chain variable region sequence fused to the N terminus of an amino acid sequence corresponding to a TCR β chain constant domain extracellular sequence, and a linker sequence linking the C terminus of the first segment to the N terminus of the second segment.

In some embodiments, a scTCR contains a first segment constituted by an amino acid sequence corresponding to a TCR β chain variable region, a second segment constituted by an amino acid sequence corresponding to a TCR α chain variable region sequence fused to the N terminus of an amino acid sequence corresponding to a TCR α chain constant domain extracellular sequence, and a linker sequence linking the C terminus of the first segment to the N terminus of the second segment.

In some embodiments, a scTCR contains a first segment constituted by an α chain variable region sequence fused to the N terminus of an α chain extracellular constant domain sequence, and a second segment constituted by a β chain variable region sequence fused to the N terminus of a sequence β chain extracellular constant and transmembrane sequence, and, optionally, a linker sequence linking the C terminus of the first segment to the N terminus of the second segment.

In some embodiments, a scTCR contains a first segment constituted by a TCR β chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some embodiments, for the scTCR to bind an MHC-peptide complex, the α and β chains must be paired so that the variable region sequences thereof are orientated for such binding. Various methods of promoting pairing of an α and β in a scTCR are well known in the art. In some embodiments, a linker sequence is included that links the α and β chains to form the single polypeptide strand. In some embodiments, the linker should have sufficient length to span the distance between the C terminus of the α chain and the N terminus of the β chain, or vice versa, while also ensuring that the linker length is not so long so that it blocks or reduces bonding of the scTCR to the target peptide-MHC complex.

In some embodiments, the linker of a scTCR that links the first and second TCR segments can be any linker capable of forming a single polypeptide strand, while retaining TCR binding specificity. In some embodiments, the linker sequence may, for example, have the formula -P-AA-P-, wherein P is proline and AA represents an amino acid sequence wherein the amino acids are glycine and serine. In some embodiments, the first and second segments are paired so that the variable region sequences thereof are orientated for such binding. Hence, in some cases, the linker has a sufficient length to span the distance between the C terminus of the first segment and the N terminus of the second segment, or vice versa, but is not too long to block or reduces bonding of the scTCR to the target ligand. In some embodiments, the linker can contain from or from about 10 to 45 amino acids, such as 10 to 30 amino acids or 26 to 41 amino acids residues, for example 29, 30, 31 or 32 amino acids. In some embodiments, the linker has the formula -PGGG-(SGGGG)₅-P- or -PGGG-(SGGGG)₆-P-, wherein P is proline, G is glycine and S is serine (SEQ ID NO:54 or 55). In some embodiments, the linker has the sequence GSADDAKKDAAKKDGKS (SEQ ID NO:53).

In some embodiments, a scTCR contains a disulfide bond between residues of the single amino acid strand, which, in some cases, can promote stability of the pairing between the α and β regions of the single chain molecule (see e.g. U.S. Patent No. 7,569,664). In some embodiments, the scTCR contains a covalent disulfide bond linking a residue of the immunoglobulin region of the constant domain of the α chain to a residue of the immunoglobulin region of the constant domain of the β chain of the single chain molecule. In some embodiments, the disulfide bond corresponds to the native disulfide bond present in a native dTCR. In some embodiments, the disulfide bond in a native TCR is not present. In some embodiments, the disulfide bond is an introduced non-native disulfide bond, for example, by incorporating one or more cysteines into the constant region extracellular sequences of the first and second chain regions of the scTCR polypeptide. Exemplary cysteine mutations include any as described above. In some cases, both a native and a non-native disulfide bond may be present.

In some embodiments, a scTCR is a non-disulfide linked truncated TCR in which heterologous leucine zippers fused to the C-termini thereof facilitate chain association (see e.g. International published PCT No. WO99/60120). In some embodiments, a scTCR contain a TCRα variable domain covalently linked to a TCRβ variable domain via a peptide linker (see e.g., International published PCT No. WO99/18129).

In some embodiments, the TCR is a soluble TCR. In some embodiments, the soluble TCR has a structure as described in WO99/60120 or WO 03/020763. In some embodiments, the TCR does not contain a sequence corresponding to the transmembrane sequence, for example, to permit membrane anchoring into the cell in which it is expressed. In some embodiments, the TCR does not contain a sequence corresponding to cytoplasmic sequences.

In some embodiments, any of the TCRs, including a dTCR or scTCR, can be linked to signaling domains that yield an active TCR on the surface of a T cell. In some embodiments, the TCR is expressed on the surface of cells. In some embodiments, the TCR does contain a sequence corresponding to a transmembrane sequence. In some embodiments, the transmembrane domain can be a Cα or Cβ transmembrane domain. In some embodiments, the transmembrane domain can be from a non-TCR origin, for example, a transmembrane region from CD3z, CD28 or B7.1. In some embodiments, the TCR does contain a sequence corresponding to cytoplasmic sequences. In some embodiments, the TCR contains a CD3z signaling domain. In some embodiments, the TCR is capable of forming a TCR complex with CD3.

In some embodiments, the TCR or antigen-binding fragment thereof exhibits an affinity with an equilibrium binding constant for an MHC-peptide complex or ligand of between or between about 10⁻⁵ and 10⁻¹² M and all individual values and ranges therein.

In some embodiments, the TCR can be obtained from known TCR sequences, such as sequences of Va,β chains, for which a substantially full-length coding sequence is readily available. Methods for obtaining full-length TCR sequences, including V chain sequences, from cell sources are well known. In some embodiments, nucleic acid encoding the TCR can be obtained from a variety of sources, such as by polymerase chain reaction (PCR) amplification of publicly available TCR DNA sequences. In some embodiments, the TCR is obtained from a biological source, such as from cells such as from a T cell (e.g. cytotoxic T cell), T cell hybridomas or other publicly available source. In some embodiments, the T cells can be obtained from *in vivo* isolated cells, such as from normal (or healthy) subjects or diseased subjects, including T cells present in peripheral blood mononuclear cells (PBMCs) or tumor-infiltrating lymphocytes (TILs). In some embodiments, the T cells can be a cultured T cell hybridoma or clone. In some embodiments, the TCR or antigen-binding portion thereof can be synthetically generated from knowledge of the sequence of the TCR.

In some embodiments, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some embodiments, the vector can be a vector of the pUC series (Fermentas Life Sciences), the pBluescript series (Stratagene, La Jolla, Calif.), the pET series (Novagen, Madison, Wis.), the pGEX series (Pharmacia Biotech, Uppsala, Sweden), or the pEX series (Clontech, Palo Alto, Calif.). In some cases, bacteriophage vectors, such as λG10, λGT11, λZapII (Stratagene), λEMBL4, and λNM1149, also can be used. In some embodiments, plant expression vectors can be used and include pBI01, pBI101.2, pBI101.3, pBI121 and pBIN19 (Clontech). In some embodiments, animal expression vectors include pEUK-Cl, pMAM and pMAMneo (Clontech). In some embodiments, a viral vector is used, such as a retroviral vector.

In some embodiments, the recombinant expression vectors can be prepared using standard recombinant DNA techniques. In some embodiments, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some embodiments, the vector can contain a nonnative promoter operably linked to the nucleotide sequence encoding the TCR or antigen-binding portion (or other peptide binding molecule). In some embodiments, the promoter can be a non-viral promoter or a viral promoter, such as a cytomegalovirus (CMV) promoter, an SV40 promoter, an RSV promoter, and a promoter found in the long-terminal repeat of the murine stem cell virus. Other promoters known to a skilled artisan also are contemplated.

In some embodiments, to generate a vector encoding a TCR, the α and β chains can be PCR amplified from total cDNA isolated from a T cell clone expressing the TCR of interest and cloned into an expression vector. In some embodiments, the α and β chains can be synthetically generated. In some embodiments, the α and β chains are cloned into the same vector. In some embodiments, transcription units can be engineered as a bicistronic unit containing an IRES (internal ribosome entry site), which allows coexpression of gene products (e.g. encoding an α and β chains) by a message from a single promoter. Alternatively, in some cases, a single promoter may direct expression of an RNA that contains, in a single open reading frame (ORF), multiple genes (e.g. encoding an α and β chains) separated from one another by sequences encoding a self-cleavage peptide (*e.g.,* a 2A sequence) or a protease recognition site (e.g., furin). The ORF thus encodes a single polypeptide, which, either during (in the case of 2A) or after translation, is processed into the individual proteins. In some cases, the peptide, such as T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and deFelipe et al. Traffic 5:616-626 (2004)). Many 2A elements are known in the art. Examples of 2A sequences that can be used in the methods and nucleic acids disclosed herein, without limitation, 2A sequences from the foot-and-mouth disease virus (F2A, *e.g.,* SEQ ID NO: 60), equine rhinitis A virus (E2A, *e.g.,* SEQ ID NO: 59), Thosea asigna virus (T2A, *e.g.,* SEQ ID NO: 44 or 56), and porcine teschovirus-1 (P2A, *e.g.,* SEQ ID NO: 57 or 58) as described in U.S. Patent Publication No. 20070116690.. In some embodiments, the α and β chains are cloned into different vectors. In some embodiments, the generated α and β chains are incorporated into a retroviral, e.g. lentiviral, vector.

In some embodiments, a plurality, e.g., library, of TCRs or antigen-binding fragments can be generated or obtained.

In some embodiments, TCR libraries can be generated by amplification of the repertoire of Vα and Vβ from T cells isolated from a subject, including cells present in PBMCs, spleen or other lymphoid organ. In some cases, T cells can be amplified from tumor-infiltrating lymphocytes (TILs). In some embodiments, TCR libraries can be generated from CD4⁺ or CD8⁺ cells. In some embodiments, the TCRs can be amplified from a T cell source of a normal of healthy subject, i.e. normal TCR libraries. In some embodiments, the TCRs can be amplified from a T cell source of a diseased subject, i.e. diseased TCR libraries. In some embodiments, degenerate primers are used to amplify the gene repertoire of Vα and Vβ, such as by RT-PCR in samples, such as T cells, obtained from humans. In some embodiments, scTv libraries can be assembled from naive Vα and Vβ libraries in which the amplified products are cloned or assembled to be separated by a linker. Depending on the source of the subject and cells, the libraries can be HLA allele-specific.

Alternatively, in some embodiments, TCR libraries can be generated by mutagenesis or diversification of a parent or scaffold TCR molecule. For example, in some aspects, a subject, e.g., human or other mammal such as a rodent, can be vaccinated with a peptide, such as a peptide identified by the present methods. In some embodiments, a sample can be obtained from the subject, such as a sample containing blood lymphocytes. In some instances, binding molecules, e.g., TCRs, can be amplified out of the sample, e.g., T cells contained in the sample. In some embodiments, antigen-specific T cells may be selected, such as by screening to assess CTL activity against the peptide. In some aspects, TCRs, e.g. present on the antigen-specific T cells, may be selected, such as by binding activity, e.g., particular affinity or avidity for the antigen. In some aspects, the TCRs are subjected to directed evolution, such as by mutagenesis, e.g., of the α or β chain. In some aspects, particular residues within CDRs of the TCR are altered. In some embodiments, selected TCRs can be modified by affinity maturation. In some aspects, a selected TCR can be used as a parent scaffold TCR against the antigen.

In some embodiments, the subject is a human, such as a human with cancer, e.g., melanoma. In some embodiments, the subject is a rodent, such as a mouse. In some such embodiments, the mouse is a transgenic mouse, such as a mouse expressing human MHC (i.e. HLA) molecules, such as HLA-A2. See Nicholson et. al, Adv Hematol. 2012; 2012: 404081.

In some embodiments, the subject is a transgenic mouse expressing human TCRs or is an antigen-negative mouse. See Li et. al, Nat Med. 2010 Sep;16(9):1029-34; Obenaus et. al, Nat Biotechnol. 2015 Apr;33(4):402-7. In some aspects the subject is a transgenic mouse expressing human HLA molecules and human TCRs.

In some embodiments, such as where the subject is a transgenic HLA mouse, the identified TCRs are modified, e.g. to be chimeric or humanized. In some aspects, the TCR scaffold is modified, such as analogous to known antibody humanizing methods.

In some embodiments, such a scaffold molecule is used to generate a library of TCRs.

For example, in some embodiments, the library includes TCRs or antigen-binding portions thereof that have been modified or engineered compared to the parent or scaffold TCR molecule. In some embodiments, directed evolution methods can be used to generate TCRs with altered properties, such as with higher affinity for a specific MHC-peptide complex. In some embodiments, display approaches involve engineering, or modifying, a known, parent or reference TCR. For example, in some cases, a wild-type TCR can be used as a template for producing mutagenized TCRs in which in one or more residues of the CDRs are mutated, and mutants with an desired altered property, such as higher affinity for a desired target antigen, are selected. In some embodiments, directed evolution is achieved by display methods including, but not limited to, yeast display (Holler et al. (2003) Nat Immunol, 4, 55-62; Holler et al. (2000) Proc Natl Acad Sci USA, 97, 5387-92), phage display (Li et al. (2005) Nat Biotechnol, 23, 349-54), or T cell display (Chervin et al. (2008) J Immunol Methods, 339, 175-84).

In some embodiments, the libraries can be soluble. In some embodiments, the libraries are display libraries in which the TCR is displayed on the surface of a phage or cell, or attached to a particle or molecule, such as a cell, ribosome or nucleic acid, e.g., RNA or DNA. Typically, the TCR libraries, including normal and disease TCR libraries or diversified libraries, can be generated in any form, including as a heterodimer or as a single chain form. In some embodiments, one or more members of the TCR can be a two-chain heterodimer. In some embodiments, pairing of the Vα and Vβ chains can be promoted by introduction of a disulfide bond. In some embodiments, members of the TCR library can be a TCR single chain (scTv or ScTCR), which, in some cases, can include a Vα and Vβ chain separated by a linker. Further, in some cases, upon screening and selection of a TCR from the library, the selected member can be generated in any form, such as a full-length TCR heterodimer or single-chain form or as antigen-binding fragments thereof.

### 2. Antibodies or antigen-binding fragments

In aspects of the provided methods, the peptide binding molecule is an antibody or antigen binding fragment that can exhibit binding specificity for a T cell epitope or peptide epitope when displayed or presented in the context of an MHC molecule, i.e. the antibody or antigen-binding portion thereof can be a TCR-like antibody. In some embodiments, the antibody or antibody-binding portion thereof is reactive against a specific MHC-peptide complex, wherein the antibody or antibody fragment can differentiate the specific MHC-peptide complex from the MHC molecule alone, the specific peptide alone, and, in some cases, a complex of MHC and an irrelevant peptide. In some embodiments, an antibody or antigen-binding portion thereof can exhibit a higher binding affinity than a T cell receptor, including a TCR that may exhibit binding specificity for the same MHC-peptide complex.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, variable heavy chain (V_{H}) regions capable of specifically binding the antigen, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

In some embodiments, the heavy and light chains of an antibody can be full-length or can be an antigen-binding portion (a Fab, F(ab')2, Fv or a single chain Fv fragment (scFv)). In other embodiments, the antibody heavy chain constant region is chosen from, e.g., IgG1, IgG2, IgG3, IgG4, IgM, IgA1, IgA2, IgD, and IgE, particularly chosen from, e.g., IgG1, IgG2, IgG3, and IgG4, more particularly, IgG1 (e.g., human IgG1). In another embodiment, the antibody light chain constant region is chosen from, e.g., kappa or lambda, particularly kappa.

Among the provided antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; variable heavy chain (V_{H}) regions, single-chain antibody molecules such as scFvs and single-domain V_{H} single antibodies; and multispecific antibodies formed from antibody fragments. In particular embodiments, the antibodies are single-chain antibody fragments comprising a variable heavy chain region and/or a variable light chain region, such as scFvs.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, e.g., Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, e.g., Portolano et al., J. Immunol. 150:880-887 (1993); Clarkson et al., Nature 352:624-628 (1991).

Single-domain antibodies are antibody fragments comprising all or a portion of the heavy chain variable domain or all or a portion of the light chain variable domain of an antibody. In certain embodiments, a single-domain antibody is a human single-domain antibody.

Antibody fragments can be made by various techniques, including but not limited to proteolytic digestion of an intact antibody as well as production by recombinant host cells. In some embodiments, the antibodies are recombinantly-produced fragments, such as fragments comprising arrangements that do not occur naturally, such as those with two or more antibody regions or chains joined by synthetic linkers, e.g., peptide linkers, and/or that are may not be produced by enzyme digestion of a naturally-occurring intact antibody. In some aspects, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some embodiments, some FR residues in a humanized antibody are substituted with corresponding residues from a non-human antibody (e.g., the antibody from which the CDR residues are derived), e.g., to restore or improve antibody specificity or affinity.

In some embodiments, an antibody or antigen binding fragment library is generated. In some aspects, the library contains a diverse pool of polypeptides, each of which includes an immunoglobulin domain, e.g., an immunoglobulin variable domain.

In some embodiments, the antibody library contains polypeptides that include a VH domain and a VL domain. The library can include the antibody as a Fab fragment (e.g., using two polypeptide chains) or a single chain Fv (e.g., using a single polypeptide chain). Other formats can also be used.

As in the case of the Fab and other formats, the antibody can include a constant region as part of a light or heavy chain. In one embodiment, each chain includes one constant region, e.g., as in the case of a Fab. In other embodiments, additional constant regions are included.

In some embodiments, a plurality, e.g., library, of antibodies or antigen-binding fragments can be generated or obtained. In some embodiments, such methods have been used to produce a TCR-like antibody or antigen-binding portion (see e.g. US Published Application Nos. US 2002/0150914; US 2003/0223994; US 2004/0191260; US 2006/0034850; US 2007/00992530; US20090226474; US20090304679; and International PCT Publication No. WO 03/068201).

In some aspects, antibody libraries are constructed from nucleic acid from immunoglobulin genes, including immunoglobulin genes from a normal (or healthy) subject or a diseased subject. In some cases, the nucleic acid molecules can represent naive germline immunoglobulin genes. The nucleic acids generally include nucleic acids encoding the VH and/or VL domain. Sources of immunoglobulin-encoding nucleic acids are described below. In some embodiments, the nucleic acid molecules can be obtained by amplification, which can include a PCR amplification methods, e.g., with primers that anneal to the conserved constant region of a particular IgG isotype, e.g. IgM, or another amplification method (see e.g. Zhu and Dimitrov (2009) Methods Mol Biol., 525:129; Hust et al. (2012) Methods in Molecular Biology, 907:85-107). In some embodiments, the nucleic acid molecules can be obtained by *in silico* rearrangement of known germline segments encoding VH and/or VL chains (see e.g., published PCT App. No. WO2010/054007). Generally, whether by amplification or in silico approaches, a plurality of VH domains can be recombined with a plurality of VL domains. In some cases, a large number of VH genes and VL genes can be obtained such that the number of possible combinations is such that the likelihood that some of the newly formed combinations will exhibit antigen-specific binding activity is reasonably high, such as provided that the final library size is sufficiently large.

Nucleic acid encoding immunoglobulin domains can be obtained from the immune cells of, e.g., a human, a primate, mouse, rabbit, camel, or rodent. Any cells may be used as a source for a library. In some cases, immunoglobulin genes can be obtained from blood lymphocytes, bone marrow, spleen or other immunoglobulin-containing source. In some embodiments the source of cells for the library may be PBMCs, splenocytes, or bone marrow cells. In some cases, immunoglobulin genes are obtained from B cells. In one example, the cells are selected for a particular property. B cells at various stages of maturity can be selected. In another example, the B cells are naive. In some embodiments, T cells from a human donor may be used.

In some embodiments, the antibody libraries can include IgM-derived antibody genes, which generally represent non-immune or naive antibody genes, i.e. sometimes called a naive antibody library. For example, in some embodiments, naive libraries of antibody fragments have been constructed, for example, by cloning of the rearranged V-genes from the IgM RNA of B cells of un-immunized donors isolated from peripheral blood lymphocytes, bone marrow or spleen cells (see, for example, Griffiths et al, EMBO Journal, 12(2), 725-734, 1993, Marks et al, J. Mol. Biol., 222, 581-597, 1991). In some embodiments, the antibody libraries can include IgG-derived antibody genes, although IgG-based libraries are typically biased to particular antigen(s).

In one embodiment, fluorescent-activated cell sorting (FACS) is used to sort B cells that express surface-bound IgM, IgD, or IgG molecules. Further, B cells expressing different isotypes of IgG can be isolated. In another embodiment, the B or T cell is cultured in vitro. The cells can be stimulated in vitro, e.g., by culturing with feeder cells or by adding mitogens or other modulatory reagents, such as antibodies to CD40, CD40 ligand or CD20, phorbol myristate acetate, bacterial lipopolysaccharide, concanavalin A, phytohemagglutinin or pokeweed mitogen.

In some embodiments, the cells are isolated from a subject that has a disease or disorder, e.g., cancer or an immunological disorder. The subject can be a human, or a non-human animal, e.g., an animal model for the human disease, or an animal having an analogous disorder. In some embodiments, the antibody library is an immune library, such as constructed from antibodies obtained from infected or diseased subjects. In some embodiments, an immune library may contain antibody members that have higher affinity binding than can be obtained using naive antibody libraries or antibody libraries derived from normal or healthy subjects.

In some embodiments, the cells have activated a program of somatic hypermutation. Cells can be stimulated to undergo somatic mutagenesis of immunoglobulin genes, for example, by treatment with anti-immunoglobulin, anti-CD40, and anti-CD38 antibodies (see, e.g., Bergthorsdottir et al. (2001) J. Immunol. 166:2228). In another embodiment, the cells are naive.

The nucleic acid encoding an immunoglobulin variable domain can be isolated from a natural repertoire by the following exemplary method. First, RNA is isolated from the immune cell. Full length (i.e., capped) mRNAs are separated (e.g. by degrading uncapped RNAs with calf intestinal phosphatase). The cap is then removed with tobacco acid pyrophosphatase and reverse transcription is used to produce the cDNAs.

The reverse transcription of the first (antisense) strand can be done in any manner with any suitable primer. See, e.g., de Haard et al. (1999) J. Biol. Chem. 274:18218-30. The primer binding region can be constant among different immunoglobulins, e.g., in order to reverse transcribe different isotypes of immunoglobulin. The primer binding region can also be specific to a particular isotype of immunoglobulin. Typically, the primer is specific for a region that is 3' to a sequence encoding at least one CDR. In another embodiment, poly-dT primers may be used (e.g., for the heavy-chain genes).

A synthetic sequence can be ligated to the 3' end of the reverse transcribed strand. The synthetic sequence can be used as a primer binding site for binding of the forward primer during PCR amplification after reverse transcription. The use of the synthetic sequence can obviate the need to use a pool of different forward primers to fully capture the available diversity.

The variable domain-encoding gene is then amplified, e.g., using one or more rounds. If multiple rounds are used, nested primers can be used for increased fidelity. The amplified nucleic acid is then cloned into a library vector.

Any method for amplifying nucleic acid sequences may be used for amplification. Methods that maximize, and do not bias, diversity may be used. A variety of techniques can be used for nucleic acid amplification. The polymerase chain reaction (PCR; U.S. Pat. Nos. 4,683,195 and 4,683,202, Saiki, et al. (1985) Science 230, 1350-1354) utilizes cycles of varying temperature to drive rounds of nucleic acid synthesis. Transcription-based methods utilize RNA synthesis by RNA polymerases to amplify nucleic acid (U.S. Pat. No. 6,066,457; U.S. Pat. No. 6,132,997; U.S. Pat. No. 5,716,785; Sarkar et al., Science (1989) 244: 331-34; Stofler et al., Science (1988) 239: 491). NASBA (U.S. Pat. Nos. 5,130,238; 5,409,818; and 5,554,517) utilizes cycles of transcription, reverse-transcription, and RNaseH-based degradation to amplify a DNA sample. Still other amplification methods include rolling circle amplification (RCA; U.S. Pat. Nos. 5,854,033 and 6,143,495) and strand displacement amplification (SDA; U.S. Pat. Nos. 5,455,166 and 5,624,825).

Antibody libraries can be constructed by a number of processes (see, e.g., WO 00/70023). Further, elements of each process can be combined with those of other processes. The processes can be used such that variation is introduced into a single immunoglobulin domain (e.g., VH or VL) or into multiple immunoglobulin domains (e.g., VH and VL). The variation can be introduced into an immunoglobulin variable domain, e.g., in the region of one or more of CDR1, CDR2, CDR3, FR1, FR2, FR3, and FR4, referring to such regions of either and both of heavy and light chain variable domains. In one embodiment, variation is introduced into all three CDRs of a given variable domain. In another embodiment, the variation is introduced into CDR1 and CDR2, e.g., of a heavy chain variable domain. Any combination is feasible. In one process, antibody libraries are constructed by inserting diverse oligonucleotides that encode CDRs into the corresponding regions of the nucleic acid. The oligonucleotides can be synthesized using monomeric nucleotides or trinucleotides. For example, Knappik et al. (2000) J. Mol. Biol. 296:57-86 describes a method for constructing CDR encoding oligonucleotides using trinucleotide synthesis and a template with engineered restriction sites for accepting the oligonucleotides.

In some embodiments, the library contains nucleic acids that encode antibodies or antibody fragments. The nucleic acid molecules can be generated separately, such that upon expression an antibody is formed. For example, nucleic molecules can be generated encoding a VH chain of an antibody and/or nucleic acid molecules can be generated encoding a VL chain of an antibody. In some aspects, upon co-expression of the nucleic acid molecules in a cell, an antibody is generated. Alternatively, an scFv library can be generated in which a single nucleic acid molecule can be generated that encodes both the variant VH and VL chains of an antibody, generally separated by a linker.

In any of the libraries herein, the nucleic acid molecules also can further contain nucleotides for the hinge region and/or constant regions (e.g. CL or CH1, CH2 and/or CH3) of the antibody. Further, the nucleic acid molecules optionally can include nucleotides encoding peptide linkers. Methods to generate and express antibodies are described herein, and can be adapted for use in generating any antibody library. Hence, the antibody libraries can include members that are full-length antibodies, or that are antibody fragments thereof. In some embodiments, antibody libraries are scFv libraries. In some embodiments, antibody libraries are Fab libraries. Further, it is understood that upon screening and selection of an antibody from the library, the selected member can be generated in any form, such as a full-length antibody or as an antibody fragment.

### B. Screening Methods

In some embodiments, the methods include providing a library of candidate binding molecules, such as an antibody library or TCR library, including any as described above, and screening the library to identify a member that binds to an MHC-E restricted peptide epitope (e.g. an MHC-E-peptide complex), such as identified using the provided methods. In some embodiments, the format of the library can be an expression library, such as a display library.

In some embodiments, the screening methods result in the identification or selection of a protein, such as a binding molecule, e.g., a TCR, antibody or antigen-binding fragment thereof, from a plurality of candidate binding molecules, e.g. a plurality of TCRs, antibodies or portions thereof, respectively, such as a collection or library of a such molecules, based on determination of an activity or property indicative of binding. In some cases, an activity or property indicative of binding can include quantitative and/or qualitative determinations of binding in the sense of obtaining an absolute value for the binding, and/or modulation of an activity related to the binding, and also of obtaining an index, ratio, percentage, visual or other value or measure of the level of the binding or activity. Assessment can be direct or indirect. Screening can be performed in any of a variety of ways.

In some embodiments, methods of screening involves contacting members of the plurality or library of binding molecules with a target antigen or ligand, e.g. MHC-E-peptide complex or complexes, and assessing a property or activity, for example, by assays assessing direct binding (e.g. binding affinity) to a target ligand or antigen. In some embodiments, screening methods include contacting one or more members of the library with an MHC-peptide complex, washing or removing unbound binding molecules, and detecting or identifying molecules, such as peptides (e.g., peptides of a tumor antigen), that bind to the MHC-peptide complex. In some cases, members of the library can be detectably labeled or can be detected, thereby facilitating detection of binders. In other cases, binding molecules can be identified by subsequent enrichment and sequencing of positive binders.

In some embodiments, the screening assay can be high-throughput. For example, in some cases, screening can be performed by assessing binding or activity of a large number of molecules, such as generally tens to hundreds to thousands to hundreds of thousands of molecules. High-throughput methods can be performed manually or can be automated, such as using robotics or software.

In some embodiments, each binding molecule of the library can be screened individually and separately for binding to an MHC-peptide complex. In some embodiments, screening can be performed in an addressable library. Any addressable array technology known in the art can be employed for screening of library members, including antibodies or TCRs. For example, candidate binding molecules can be physically separated from each other, such as by formatting in a spatial array, such as a multi-well plate or plates, such that each individual locus of the plate corresponds to one individual antibody or TCR. Multi-well plates can include, but are not limited to, 12-well, 24-well, 48-well, 96-well plates, 384-well plates, and 1536-well plates. In some instances, the identity of each member in a position of the array, e.g. each well of the array, is known. In some embodiments, an MHC-peptide complex, either soluble or cell-expressed, can be present, e.g. added, to each position of the array, to permit contacting of members of the library with the target antigen or ligand.

In some embodiments, the candidate binding molecules can be pooled and screened, such as in a non-addressable format. Examples of such other non-addressable formats include by display, in particular, any display format that facilitates screening of the members of the libraries for an activity or activities, e.g., binding to a peptide epitope, such as an MHC-E-peptide complex that is either soluble or cell-expressed. In some embodiments, libraries are screened using a display technique in which there is a physical link between individual molecules of the library (phenotype) and the genetic information encoding them (genotype). In some embodiments, candidate binding molecules can be provided as a display library in which each protein member of the library is physically linked to its nucleic acid (e.g. cDNA) in a defined particle, such as filamentous phage, a ribosome or a cell. Display library methods are known in the art and include, but are not limited to, cell display, phage display, mRNA display, ribosome display and DNA display.

In some embodiments, the one or more binding molecules, such as a library of candidate binding molecules, are assessed for binding to an MHC-E-peptide complex containing the T cell epitope. In some cases, MHC-expressing cells in which an antigen has been transferred, e.g. by introduction of a synthetic nucleic acid molecule encoding the antigen, can be used for directly panning against such libraries to identify a binding molecule or molecules that bind to an MHC-E-restricted epitope of the antigen displayed on the surface of the cells on an MHC-E. Alternatively, in embodiments in which the identity or sequence of the peptide epitope is known, such as by identification of the peptide using the provided methods, the peptide can be complexed with an MHC-E molecule that matches the peptide restriction to generate a stable MHC-peptide complex using any of a cell-free or cell-based methods. In some embodiments, the stable MHC-peptide complex, which can be soluble or expressed on a cell, can be screened against such libraries to identify a binding molecule or molecules that bind to the MHC-peptide complex.

In some embodiments, screening assays are performed to assess binding to a particular peptide epitope, such as a peptide epitope identified using any of the above described methods. In some aspects, the peptide is stably displayed in the context of an MHC-E molecule. In some embodiments, any of a number of MHC peptide binding assays, such as any described above, can be performed in order to confirm or determine the binding affinity of the peptide for the MHC-E molecule.

Methods of preparing or generating stable MHC-peptide complexes are well known in the art. For assessing binding, the MHC-peptide complex can, in some cases, be attached to a solid support, expressed from a cell, expressed in soluble form or otherwise provided in a manner in which binding thereto can be assessed. In some embodiments, the MHC component of the complex can be tagged and recombinantly expressed. In some embodiments, the recombinant MHC is reconstituted with the peptide, e.g., that is produced synthetically. In some embodiments, the MHC-peptide complex is attached to a support, e.g., to paramagnetic beads or other magnetically responsive particle.

In some embodiments, an MHC molecule can be prepared and purified, and then these proteins can be denatured and refolded in vitro in the presence of the particular peptide epitope of the MHC-peptide complex. In some embodiments, bacterial purification and refolding improve the homogeneity of the MHC-peptide complex. In some embodiments, the particular peptide of interest that is incorporated in vitro into the complex does not have to compete with a large number of cellular peptides for binding to the MHC complex and, e.g., results in a homogenous target for binding the display library against. In some embodiments, this purified complex can be panned against the display library to identify members of the library the bind the MHC-peptide complex. In some cases, expression can be in a bacterial system, whereby the MHC molecules can be purified from inclusion bodies. For MHC class I molecules, including non-classical MHC-E molecules, β2-microglobulin also can be prepared and purified for refolding of the complex. In some embodiments, the α chain and the β2 microglobulin can be covalently linked, such as by an approximately 15 amino acid linker, e.g., as described in Denkberg and Reiter (2000) Eur. J Immunol. 30:3522-32. In some embodiments, one of the chains, such as the α chain, can include a purification handle such as the BirA sequence that is biotinylated or the hexa-histidine tag. In some embodiments, this purified complex can be panned against the display library to identify members of the library the bind the MHC-peptide complex.

In some embodiments, the MHC complex can be expressed on the surface of a cell. In some embodiments, cells are transfected with a nucleic acid that expresses an MHC protein having an allele that matches the restriction of the peptide of interest and the transfected cells are loaded with the peptide. In some embodiments, cells of interest that express the MHC-peptide complex are attached to a support. In some embodiments, the cell-expressed MHC-peptide complex can be panned against the display library to identify members of the library the bind the MHC-peptide complex.

In some embodiments, the screening methods provided herein, such as display library screening methods, can include a selection or screening process that discards library members that bind to a non-target molecule. Examples of non-target molecules can include a peptide epitope that is not bound to an MHC, an MHC that is not bound by a peptide, an MHC that is bound by a peptide that differs from the peptide of interest and/or an MHC that is bound by the peptide of interest, but has a different allele from the MHC of interest. Negative selection screening can be used. For example, in some embodiments, a negative screening step can be used to discriminate between the target MHC-peptide complex and a related non-target molecule or non-target molecules. In some embodiments, the library, e.g. TCR or antibody library, such as a display library, can be contacted to the non-target molecule. Members of the sample that do not bind the non-target can be collected and used in subsequent selections or screens for binding to the target MHC-peptide complex and/or even for subsequent negative selections. The negative selection step can be prior to or after selection library members that bind to the target MHC-peptide complex.

In some embodiments, a collection or library, such as a display library, can be contacted with the target MHC-peptide complex, either in soluble or cell-bound form. In some embodiments, members of the library that bind to the target MHC-peptide complex, such as bind to the cells, are isolated and characterized.

### 1. Display Libraries

In some embodiments, the method includes contacting members of a diverse library in which a plurality of diverse TCRs or antibody-binding molecules are displayed on the surface with a non-canonical peptide MHC-complex, detecting binding between the library members and said given peptide-MHC complex, isolating a library member detected as binding to the given peptide-MHC complex, and optionally multiplying the isolated library member in an amplification process. In some embodiments, the method is performed by contacting a plurality of TCR or antibody-like TCR binding molecules with a peptide-MHC complex of interest.

In some embodiments, a display library is used to identify binding molecules that bind to the MHC-peptide complex and recognize the peptide moiety of the complex. In some embodiments, a display library is a collection of binding molecules, such as a library of TCRs or antigen-binding portions or a library of antibodies or antigen-binding portions. In some embodiments, in a selection, a binding molecule is probed with the MHC-peptide complex and if the binding molecule binds to the MHC-peptide complex, the display library member is identified, typically by retention on a support.

In some embodiments, retained display library members are recovered from the support and analyzed. In some embodiments, the analysis can include amplification and a subsequent selection under similar or dissimilar conditions. For example, in some embodiments, positive and negative selections can be alternated. In some embodiments, the analysis can also include determining the amino acid sequence of the binding molecule and purification of the binding molecule, such as for detailed characterization.

A variety of formats can be used for display libraries. Examples include the following.

### a. Phage Display

In some embodiments, a phage display library, such as a library utilizing viruses, such as bacteriophages, is used. In some embodiments, a binding molecule, e.g., an antibody or antigen-binding portion thereof or a TCR or antigen-binding portion thereof, that potentially binds to an MHC-peptide complex can be produced by employing phage antibody libraries. Phage display is a widely used method for screening libraries of potential binding molecules for their ability to bind to a particular antigen. Generally, phage display is a cell based method in which proteins or peptides are expressed individually on the surface of phage as fusions to a coat protein, while the same phage particle carries the DNA encoding the protein or peptide (Smith, G. P. (1985) Science 228:1315-1317). In some cases, selection of the phage is achieved through a specific binding reaction involving recognition of the protein or peptide, enabling the particular phage to be isolated and cloned and the DNA for the protein or peptide to be recovered and propagated or expressed. In some embodiments, phage libraries are panned against a target antigen of interest, such as a soluble antigen, immobilized antigen, or cell-expressed antigen.

In some embodiments employing phage display, the protein of interest is fused to the N-terminus of a viral coat protein (Scott and Smith (1990) Science, 249, 386-90). In some such embodiments, the binding molecule is covalently linked to a bacteriophage coat protein. In some embodiments, the linkage results from translation of a nucleic acid encoding the binding molecule fused to the coat protein. In some embodiments, the linkage can include a flexible peptide linker, a protease site, or an amino acid incorporated as a result of suppression of a stop codon. Phage display is described, for example, in Ladner et al, U.S. Patent No. 5,223,409; Smith (1985) Science 228:1315-1317; WO 92/18619; WO 91/17271; WO 92/20791; WO 92/15679; WO 93/01288; WO 92/01047; WO 92/09690; WO 90/02809; de Haard et al (1999) J. Biol. Chem 274:18218-30; Hoogenboom et al. (1998) Immunotechnology 4:1-20; Hoogenboom et al. (2000) Immunol Today 2:371-8; Fuchs et al. (1991) Bio/Technology 9:1370-1372; Hay et al. (1992) Hum Antibod Hybridomas 3:81-85; Huse et al. (1989) Science 246:1275-1281; Griffiths et al. (1993) EMBO J 12:725-734; Hawkins et al. (1992) JMol Biol 226:889-896; Clackson et al. (1991) Nature 352:624-628; Gram et al. (1992) PNAS 89:3576-3580; Garrard βt al. (1991) Bio/Technology 9:1373-1377; Rebar et al (1996) Methods Enzymol 267:129-49; Hoogenboom et al. (1991) Nuc Acid Res 19:4133-4137; and Barbas et al. (1991) PNAS 88:7978-7982.

Phage display systems have been developed for filamentous phage (phage fl, fd, and M13) as well as other bacteriophage (e.g. T7 bacteriophage and lambdoid phages; see, e.g., Santini (1998) J. Mol. Biol 282:125-135; Rosenberg et al. (1996) Innovations 6:1-6; Houshmet al. (1999) Anal Biochem 268:363-370). In some embodiments, the filamentous phage display systems uses fusions to a minor coat protein, such as gene III protein, and gene VIII protein, a major coat protein, but fusions to other coat proteins such as gene VI protein, gene VII protein, gene IX protein, or domains thereof can also be used (see, e.g., WO 00/71694). In some embodiments, the fusion is to a domain of the gene III protein, e.g., the anchor domain or "stump," (see, e.g., U.S. Patent No. 5,658,727 for a description of the gene III protein anchor domain).

In some embodiments, the bacteriophage displaying the binding molecule can be grown and harvested using standard phage preparatory methods, e.g. PEG precipitation from growth media.

In some embodiments, after selection of individual display phages, the nucleic acid encoding the selected binding molecule is identified, such as by infecting cells using the selected phages. In some embodiments, individual colonies or plaques can be picked, and the nucleic acid may be isolated and sequenced.

In some embodiments, antibody libraries expressed primarily on filamentous phages may be used. In some aspects, immunoglobulin genes can be obtained as described above for generation of libraries. In some aspects, the raw material for these libraries is mRNA of B cells derived from immune humans or laboratory animals. In some instances, the pools of VH and VL genes are amplified by RT-PCR separately, each with a specific set of primers. The resulting genes encoding the VH and VL chains can in some aspects then be shuffled randomly and cloned in vectors, which can drive their expression on phages either as scFv or as Fab fragments. In this way, large repertoires of antibodies can be formed and displayed on the surface of the filamentous phage.

Exemplary antibodies libraries include those as described in US Patent No. 5,969,108, which describes libraries of DNA encoding respective chains of multimeric specific binding pair members such as the VH and VL chains of an antibody, in which said binding pair members are displayed in functional form at the surface of a secreted recombinant genetic display package containing DNA encoding said binding pair member or a polypeptide component thereof, by virtue of the specific binding pair member or a polypeptide thereof being expressed as a fusion with a capsid component of the recombinant genetic display package. The antibody members are thus obtained with the different chains thereof expressed, one fused to the capsid component and the other in free form for association with the fusion partner polypeptide. Packaging in a phagemid as an expression vector produces antibody libraries said to have a much greater diversity in the antibody VL and VH chains than by conventional methods. Exemplary antibody libraries also include those as described in US Patents Nos. 5,498,531 and 5,780,272, which describe in vitro intron-mediated combinatorial methods for generating a variegated population of ribonucleic acids encoding chimeric gene products comprising admixing a variegated set of splicing constructs under trans-splicing conditions. In some cases, such methods can be used for generating diverse antibody libraries.

In some embodiments, phage display libraries of mutant Fab, scFV or other antibody forms can be generated, for example, in which members of the library are mutated at one or more residues of a CDR or CDRs. Exemplary of such methods are known in the art (see e.g. US published application No. US20020150914, US2014/0294841; and Cohen CJ. et al. (2003) J Mol. Recogn. 16:324-332).

Phage display libraries also can be employed for display and screening of TCRs or antigen-binding portions thereof. In some cases, various TCRs have been engineered for higher affinity using such methods (Li et al. (2005) Nat Biotechnol, 23, 349-54; Sami et al. (2007) Protein Eng Des Sel, 20, 397-403; Varela-Rohena et al. (2008) Nat Med, 14, 1390-5). In some embodiments, phage display of TCRs can involve introduction of a non-native disulfide bond between the two C domains in order to promote pairing of the α and β chains. In some cases, systems for phage display of TCRs use full-length (VαCα/VβCβ) heterodimeric proteins.

### b. Cell Display

In some embodiments, the library is a cell-display library. Thus, in some embodiments, binding molecules are displayed on the surface of a cell. In some embodiments, a binding molecule, e.g., an antibody or antigen-binding portion thereof or a TCR or antigen-binding portion thereof, that potentially binds to an MHC-peptide complex can be produced by employing cell display libraries. Cell display is a widely used method for screening libraries of potential binding molecules for their ability to bind to a particular antigen. Generally, cell display is a cell based method in which proteins or peptides are expressed individually on the surface of a cell. Selection of the cells is achieved through a specific binding reaction involving recognition of the protein or peptide, enabling the particular cells to be isolated and cloned and the protein or peptide to be recovered and propagated or expressed. In some embodiments, cell display libraries are panned against a target antigen of interest, such as a soluble antigen, immobilized antigen, or cell-expressed antigen.

In some embodiments, the cell is, for example, a eukaryotic or prokaryotic cell. Exemplary prokaryotic cells include E. coli cells, B. subtilis cells, or spores. Exemplary eukaryotic cells include yeast (e.g., Saccharomyces cerevisiae, Schizosaccharomyces pombe, Hanseula, or Pichia pastoris). Yeast surface display is described, e.g., in Boder and Wittrup (1997) Nat. Biotechnol. 15:553-557. U.S. Provisional Patent Application Serial No. 60/326,320, filed October 1, 2001, describes a yeast display system that can be used to display immunoglobulin proteins such as Fab fragments.

In some embodiments, nucleic acids encoding immunoglobulin variable domains are cloned into a vector for yeast display. In some embodiments, the cloning joins the nucleic acid encoding at least one of the variable domains with nucleic acid encoding a fragment of a yeast cell surface protein, e.g., Flol, a-agglutinin, α-agglutinin, or fragments derived thereof, such as Aga2p, Agalp. In some embodiments, a domain of these proteins can anchor the polypeptide encoded by the diversified nucleic acid sequence by a GPI-anchor (e.g. a-agglutinin, α-agglutinin, or fragments derived thereof, such as Aga2p, Agalp), or by a transmembrane domain (e.g., Flol). In some embodiments, the vector can be configured to express two polypeptide chains on the cell surface such that one of the chains is linked to the yeast cell surface protein. For example, the two chains can be immunoglobulin chains.

In some embodiments, peptide binding molecules, e.g., TCRs, are generated and displayed in a yeast display system, e.g., as described in US20150191524. For instance, yeast display can allow for the protein of interest to be expressed on the surface as an Aga2-fusion (Boder and Wittrup (1997) Nat. Biotech., 15, 553-557; Boder and Wittrup (2000) Methods Enzymol, 328, 430-44). In the yeast display system, the TCR can be displayed as a stabilized single-chain protein, in Vβ-linker-Vα or Vα-linker-Vβ forms (Aggen et al. (2011) Protein Engineering, Design, & Selection, 24, 361-72; Holler et al. (2000) Proc Natl Acad Sci USA, 97, 5387-92; Kieke et al. (1999) Proc Natl Acad Sci USA, 96, 5651-6; Richman et al. (2009) Mol Immunol, 46, 902-16; Weber et al. (2005) Proc Natl Acad Sci USA, 102, 19033-8), or as a two-chain heterodimer (Aggen et al. (2011) Protein Engineering, Design, & Selection, 24, 361-72; Richman et al. (2009) Mol Immunol, 46, 902-16). In some embodiments, human TCR single-chain VαVβ fragments (called scTv or scTCR) can be developed by taking advantage of the stability of the human Vα region called Vα2 (Aggen et al. (2011) Protein Engineering, Design, & Selection, 24, 361-72). In some embodiments, in vitro engineered, high-affinity T cell receptors in a single-chain format can be used to isolate human stabilized scTv fragments (Vβ-linker-Va), which can be expressed as stable proteins, both on the surface of yeast and in soluble form from E. coli.

In some embodiments, antibody or TCR libraries for screening can be expressed on the surfaces of cells, including bacteria E. coli, yeast S. cerevisiae, and mammalian cells, by fusing them with a protein that is expressed on the surface of the cell. In some embodiments, cell display may be used to screen antibody or TCR libraries wherein immobilization of the target antigen is unnecessary. In other embodiments, technologies such as fluorescence-activated cell sorting (FACS) can be used to identify desired antibodies. Generally, FACS permits the separation of subpopulations of cells on the basis of their light scatter properties as they pass through a laser beam. See e.g. published Patent Application Nos. US 2003/0100023 and US 2003/0036092. Single chain antibodies or TCRs can be expressed on the external surface of E. coli by fusing them to a protein previously shown to direct heterologous proteins to the bacterial surface (Francisco et al, (1993) Proc. Natl. Acad. Sci, USA, 90: 10444-10448). Single chain and Fab antibodies and single chain TCRs can be displayed on the surface of a yeast cell, and homologous recombination in yeast can be exploited to generate libraries of transformants (see e.g. Kieke et al, (1997) Prot. Eng., 10:1303-1310; Weaver-Feldhaus et al, (2004) FEBS Lett., 564:24-34; and Swers et al, (2004) Nucleic Acids Res., 32:e36). In some embodiments, mammalian cell display can be utilized to screen scFv libraries as well as IgGs (Ho et al, (2005) J. Biol. Chem., 280:07-617).

In some embodiments, mammalian cell display is used for the engineering of TCRs (Chervin et al. (2008) J Immunol Methods, 339, 175-84; Kessels et al. (2000) Proc Natl Acad Sci USA, 97, 14578-83). In some cases, this system uses a retroviral vector to introduce the TCR α and β-chains into a TCR-negative T cell hybridoma. In the mammalian cell display system, introduced TCRs can be expressed on the surface in its native conformation, complexed with CD3 subunits, in some aspects allowing for a fully functional T cell (signaling competent). In some embodiments, full-length, heterodimeric TCRs in their native host can be engineered using this method.

### c. Cell-free Display

In some embodiments, the library is a cell-free display library. Thus, in some embodiments, binding molecules are displayed attached to a ribosome or nucleic acid, e.g., DNA or RNA. In some embodiments, a binding molecule, e.g., an antibody or antigen-binding portion thereof, that potentially binds to an MHC-peptide complex, can be produced by employing cell-free display libraries. Cell-free display is a widely used method for screening libraries of potential binding molecules for their ability to bind to a particular antigen. Generally, cell-free display is a cell-free method in which proteins or peptides are expressed individually attached to a ribosome or nucleic acid, e.g., DNA or RNA. Selection of the binding molecules is achieved through a specific binding reaction involving recognition of the protein or peptide, enabling the particular binding molecules to be isolated and the protein or peptide to be recovered and propagated or expressed. In some embodiments, cell-free display libraries are panned against a target antigen of interest, such as a soluble antigen, immobilized antigen, or cell-expressed antigen.

Methods of library generation known in the art may be employed to create libraries suitable for use with the methods described herein. Some methods for library generation are described in U.S. Pat. Nos. 6,258,558 and 6,261,804; Szostak et al., WO989/31700; Roberts & Szostak (1997) 94:12297-12302; U.S. Pat. No. 6,385,581, WO 00/32823, U.S. Pat. Nos. 6,361,943; 7,416,847; 6,258,558; 6,214,553; 6,281,344; 6,518,018; 6,416,950; 7,195,880; 6,429,300, 9,134,304, and in U.S. Patent Publication No. US 20140113831, which are incorporated herein by reference.

In some embodiments, the display library is a ribosome display library. In some embodiments, the use of ribosome display allows for in vitro construction of binding molecule, e.g., antibody libraries. Alternatively, in some aspects, ribosome display may involve displaying proteins or peptides in nascent form on the surface of ribosomes, such that a stable complex with the encoding mRNA is formed; the complexes can be selected with a ligand for the protein or peptide and the genetic information is obtained by reverse transcription of the isolated mRNA (see e.g. United States Patent Nos. US 5,643,768 and US 5,658,754). In some aspects, selection techniques are similar to that of phage display wherein ribosome display libraries are panned against an immobilized antigen.

In some embodiments, a biotin-streptavidin interaction can be utilized. In some aspects, such as covalent DNA display, a bacteriophage P2 protein genetically fused to an antibody fragment can bind to its own DNA sequence (Reiersen et al. (2005) Nucl. Acids Res. 33:e10). Alternatively, the DNA and the peptide can be compartmentalized, such as in an oil-in-water emulsion. In some embodiments, selection techniques are similar to that of phage display wherein DNA display libraries are panned against an immobilized antigen. See e.g. International Patent Publication No. WO 98/037186.

In some embodiments, a peptide-nucleic acid fusion library is used. A peptide-nucleic acid library can include DNA display and mRNA display libraries.

In some aspects, where DNA display is employed, the DNA encoding the peptide is linked to the peptide. In some embodiments, non-covalent DNA display may be employed, in which the DNA-protein linkage is promoted by the recognition of the bacterial RepA protein as well as its own origin of replication sequence integrated into the template DNA (Odegrip et al. (2004) Proc. Natl. Acad. Sci., U.S.A. 101:2806-2810).

In some embodiments, the library is generated and/or screened as described in U.S. Patent No. 9,134,304 or US20140113831. In some embodiments, polypeptide-nucleic acid fusions can be generated by the in vitro translation of mRNA that includes a covalently attached puromycin group, e.g., as described in Roberts and Szostak (1997) Proc Natl. Acad. Sci. USA 94:12297-12302, and U.S. Patent No. 6,207,446. In some embodiments, the mRNA can then be reverse transcribed into DNA and crosslinked to the polypeptide.

In some embodiments, the nucleic acid constructs of the library contain the T7 promoter. In some aspects, the nucleic acids in the library may be manipulated by any means known in the art to add appropriate promoters, enhancers, spacers, or tags which are useful for the production, selection, or purification of the nucleic acid or its translation product. For example, in some embodiments, the sequences in the library may include a TMV enhancer, sequences encoding a FLAG tag, a streptavidin splay sequence, or a polyadenylation sequence or signal. In some embodiments, the nucleic acid library sequences may further include a unique source tag to identify the source of the RNA or DNA sequence. In some embodiments, the nucleic acid library sequences may include a pool tag. A pool tag may be used to identify those sequence selected during a particular round of selection. In some aspects, this may allow, e.g., sequences from multiple selection rounds to be pooled and sequenced in a single run without losing track of which selection round they originated from.

In some embodiments, the use of nucleic acid display libraries, such as mRNA display libraries, allows for in vitro construction of libraries of candidate binding molecules, including antibody libraries. In some aspects, mRNA display allows the display of proteins or peptides in which the nascent protein is caused to bind covalently to its mRNA through a puromycin link (Roberts et al. (1997) Proc. Natl. Acad. Sci., U.S.A. 64:12297-12302). Puromycin typically acts as a mimic of aminoacyl tRNA, enters the ribosome A site, and the nascent protein is bound covalently to it by the peptidyl-transferase activity of the ribosome. In some embodiments, selection is carried out on these protein-mRNA fusions after dissociation of the ribosome. In some aspects, selection techniques are similar to that of phage display wherein mRNA display libraries are panned against an immobilized antigen.

In some embodiments, the double stranded DNA library is transcribed in-vitro and associated to a peptide acceptor, such as puromycin. In one embodiment, a linker (e.g. a biotin-binding linker) attached to a high affinity ligand (e.g., biotin) is then annealed. In some embodiments, the linker is photo-crosslinked to the mRNA. In particular embodiments, a ligand acceptor, e.g., streptavidin, is then loaded. In further embodiments, a second high affinity ligand which is attached to a peptide acceptor is bound to the streptavidin. In some embodiments, the second high affinity ligand/peptide acceptor is a biotin-puromycin linker, e.g., BPP.

In some embodiments, in vitro translation may be carried out wherein the peptide acceptor reacts with the nascent translation product.

In some embodiments, the result, after purification, is a library of peptide-nucleic acid complexes. Such complexes may then undergo reverse transcription after, in some embodiments, being purified. The complexes may be purified by any method known in the art, e.g., by affinity chromatography, column chromatography, density gradient centrifugation, affinity tag capture, etc. In one embodiment, an oligo-dT cellulose purification is employed wherein the complex has been designed to include an mRNA with a poly-A tail. In such embodiments, oligo-dT is covalently bound to the cellulose in the column or purification device. In some embodiments, the oligo-dT participates in complementary base pairing with the poly-A tail of the mRNA in the complex, thereby impeding its progress in through the purification device. In some aspects, the complex may be eluted with water or buffer.

In some embodiments, reverse transcription generates a cDNA/RNA hybrid, which, in some aspects, is non-covalently linked to the transcribed peptide through association with the linker, the high affinity ligand, the ligand acceptor, the peptide acceptor (possibly linked to a second high affinity ligand), or some operable combination thereof.

In some embodiments, the resulting, purified complex may then be treated with RNAse to degrade the remaining mRNA, followed by second strand DNA synthesis to generate a complete cDNA. In some embodiments, the nucleic acids in the NA linker may serve as a primer for reverse transcription. Accordingly, in some aspects, the cDNA remains attached to the high affinity ligand and part of the complex.

In some embodiments, the complex may be further purified, e.g., if the complex is engineered to contain a tag. Any tag known in the art may be used to purify the complex. For example, it is possible to use a FLAG tag, myc tag, Histidine tag (His tag), or HA tag, among others. In some embodiments, a sequence encoding a FLAG tag is engineered into the original DNA sequence such that the final transcribed protein contains the FLAG tag.

In some embodiments, the resulting complex is then selected for by using any selection method known in the art. In some embodiments, affinity selection is used. For example, the desired binding target or antigen (e.g. MHC-peptide complex) may be immobilized on a solid support for use in an affinity column. Examples of methods useful in affinity chromatography are described in U.S. Pat. Nos. 4,431,546, 4,431,544, 4,385,991, 4,213,860, 4,175,182, 3,983,001, 5,043,062, which are all incorporated herein by reference in their entirety. Binding activity can be evaluated by standard immunoassay and/or affinity chromatography. Screening of complexes for catalytic function, e.g., proteolytic function can be accomplished using a standard hemoglobin plaque assay as described, for example, in U.S. Pat. No. 5,798,208. Assessing binding of candidate binding molecules (e.g., antibodies or TCRs or antigen-binding portions thereof) can be assayed in vitro using, e.g., a Biacore instrument, which measures binding rates of an antibody to a given target or antigen. In some embodiments, the target or antigen (e.g. MHC-peptide complex) is expressed on a cell surface, and the display complex of candidate binding molecules is assessed for binding to the cell surface. In some embodiments, the display complex is first screened or selected against cells not expressing the target or antigen (e.g. MHC-peptide complex), such as to remove display molecules that bind the cells but do not bind the target or interest, then the remaining display complex of candidate binding molecules are selected against cell that do express the target of interest (e.g. MHC-peptide complex), for example, to identify specific binders.

In some embodiments, the selected complexes may be identified by sequencing of the DNA component. Any sequencing technology known in the art may be employed, e.g., 454 Sequencing, Sanger sequencing, sequencing by synthesis, or the methods described in U.S. Pat. Nos. 5,547,835, 5,171,534, 5,622,824, 5,674,743, 4,811,218, 5,846,727, 5,075,216, 5,405,746, 5,858,671, 5,374,527, 5,409,811, 5,707,804, 5,821,058, 6,087,095, 5,876,934, 6,258,533, 5,149,625 which are all incorporated herein by reference in their entirety.

In some embodiments, the selection may be performed multiple times to identify higher affinity binders, and may further be implemented with competitive binders or more stringent washing conditions. One of skill in the art will appreciate that variants of the procedure described herein may be employed.

### 2. Iterative Methods

In some embodiments, libraries of candidate peptide binding molecules, including libraries of antibodies or antigen-binding portions or libraries of TCRs or antigen-binding portions, may be screened to identify binding molecules that bind to a particular peptide epitope, such as an MHC-peptide complex, in accord with the provided methods. In related embodiments, a particular binding molecule (e.g. antibody or TCR or antigen-binding portion thereof) identified or selected by such methods may be further altered by affinity maturation or mutagenesis, thereby producing a library of related binding molecules. In some embodiments, the further or related library of binding molecules can be screened for binding to the same or similar peptide epitope, such as MHC-peptide complex, in order to identify potential binding molecules that bind to the target (e.g. MHC-peptide complex) with higher binding affinity. Any methods for library generation and target selection known in the art or described herein may be used.

In some aspects, the methods may be employed in an iterative fashion. For example, the nucleic acid or protein selected by one of the methods described herein may serve as the basis for the generation of a new library from which the process may begin again. An example of such a scheme may include wherein the products of one round of selection are used to regenerate a new library.

In some embodiments, display library technology is used in an iterative mode. A first display library is used to identify one or more ligands for a target. These identified ligands are then varied using a mutagenesis method to form a second display library. Higher affinity ligands are then selected from the second library, e.g., by using higher stringency or more competitive binding and washing conditions.

In some embodiments, the mutagenesis is targeted to regions known or likely to be at the binding interface. In some embodiments, mutagenesis can be directed to the CDR regions of the heavy or light chains of the antibody or of the alpha or beta chains of the TCR. Further, mutagenesis can be directed to framework regions near or adjacent to the CDRs. In some cases, mutagenesis can be directed to one or a few of the CDRs, e.g., to make precise step-wise improvements.

Some exemplary mutagenesis techniques include: error-prone PCR (Leung et al. (1989) Technique 1:11-15), recombination, DNA shuffling using random cleavage (Stemmer (1994) Nature 389-391; termed "nucleic acid shuffling"), RACHITT^{™} (Coco et al. (2001) Nature Biotech. 19:354), site-directed mutagenesis (Zooler et al. (1987) Nucl Acids Res 10:6487-6504), cassette mutagenesis (Reidhaar-Olson (1991) Methods Enzymol. 208:564-586) and incorporation of degenerate oligonucleotides (Griffiths et al. (1994) EMBO J. 13:3245).

In one example of iterative selection, the methods described herein are used to first identify a binding molecule from a display library that binds an MHC-peptide complex with at least a requisite activity or binding specificity for the ligand, which then can be improved with subsequent iterations. In some embodiments, the nucleic acid sequence encoding the initial identified binding molecule can then be used as a template nucleic acid for the introduction of variations, e.g., to identify a second protein ligand that has enhanced properties (e.g., binding affinity, kinetics, or stability) relative to the initial binding molecule.

### C. Hybridoma Selection

In some embodiments, hybridoma technology can be used for the generation of antibodies that bind, such as specifically bind, to an MHC-peptide complex. In some embodiments, transgenic mice can be employed, which contain the human immunoglobulin repertoire, allow for in vivo affinity maturation, and, in some cases, permit the generation of human antibodies by the hybridoma technology.

In some embodiments, an antibody or antigen-binding portion thereof that potentially binds to an MHC-peptide complex can be produced by immunizing a host, e.g., a mouse, with an effective amount of an immunogen containing a specific MHC-peptide complex. In some cases, the peptide of the MHC-peptide complex is capable of being presented by an MHC molecule. In some embodiments, an effective amount of the immunogen is then administered to a host for eliciting an immune response, wherein the immunogen retains a three-dimensional form thereof for a period of time sufficient to elicit an immune response against the three-dimensional presentation of the peptide in the binding groove of the MHC molecule. In some cases, serum can be collected from the host and then can be assayed to determine if desired antibodies that recognize a three-dimensional presentation of the peptide in the binding groove of the MHC molecule is being produced. In some embodiments, the produced antibodies can be assessed to confirm that the antibody can differentiate the MHC-peptide complex from the MHC molecule alone, the peptide alone, and a complex of MHC and an irrelevant peptide. The desired antibodies can then be isolated.

In some embodiments, an animal, e.g., a rodent, is immunized with the MHC-peptide complex that includes a specific peptide, such as a peptide identified using the provided methods. In some embodiments, an animal, e.g. rodent, is immunized with a cell that presents a specific peptide on its surface bound to the MHC, such as a cell into which has been introduced a CMV vector encoding a heterologous antigen in accord with the provided methods. The cell can have a particular allele of the MHC protein. The animal is optionally boosted with the antigen (e.g. MHC-peptide complex) to further stimulate the response. In some aspects, spleen cells are isolated from the animal, and nucleic acids encoding VH and/or VL domains are amplified and cloned, such as for expression in a library.

In some embodiments, antibodies or antigen binding fragments are generated by immunization of laboratory mice or any other rodent with the relevant antigen and isolation of splenocytes, including antibody-producing B cells, which are then immortalized by fusion with myeloma cells to produce B cell hybridomas (Harlow and Lane, 1988). Generally, hybridomas preserve the ability of B cells to synthesize antigen-specific antibodies, and large amounts of products can be obtained. In some embodiments, this yields high affinity antibodies, which are generated and selected in vivo by the affinity maturation process in the course of the immune response, prior to the isolation of the B cells. In some embodiments, lines of transgenic mice harboring sizable portions of the human immunoglobulin heavy and light chain gene loci can be generated, thus permitting the production of murine B-cell hybridomas secreting fully human mAbs (reviewed by Bruggemann and Neuberger, 1996).

### IV. Recombinant Receptors, Chimeric Antigen Receptors and Genetically Engineered Cells

Provided are recombinant receptors that include or contain binding molecules (e.g. peptide binding molecules) identified by the provided methods. Such binding molecules can include TCRs, TCR-like antibodies or antigen-binding fragments thereof, as well as other recombinant receptors that contain a provided binding molecule or antigen-binding fragment thereof. For example, among such recombinant receptors are chimeric receptors, including functional non-TCR antigen receptors, such as chimeric antigen receptors (CARs), containing a provided TCR-like antibody or antigen-binding fragment thereof. In some embodiments, the methods include genetic engineering of cells, such as to introduce into the cells recombinant genes (or transgenes) for expression of recombinant receptors or transgenic antigen receptors, including transgenic TCRs and chimeric antigen receptors.

Also provided are cells, e.g. CD4+ and/or CD8+ T cells, expressing the recombinant antigen receptors and uses thereof in adoptive cell therapy, such as treatment of diseases and disorders associated with the antigen.

### A. Recombinant Receptors and Chimeric Antigen Receptors

The engineering generally includes introduction of gene or genes for expression of a genetically engineered antigen receptor. Among such recombinant receptors are genetically engineered TCRs and components thereof, and antigen receptors in which the antibody or antigen-binding portion thereof is expressed on cells as part of a recombinant receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Exemplary of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, e.g., and in which the antigen-binding portion, e.g., scFv, is replaced by an antibody, e.g., as provided herein.

In some embodiments, the CARs generally include an extracellular antigen (or ligand) binding domain of a TCR-like antibody, including as an antibody or antigen-binding fragment thereof specific for an MHC-peptide complex, linked to one or more intracellular signaling components, in some aspects via linkers and/or transmembrane domain(s). In some embodiments, such molecules can typically mimic or approximate a signal through a natural antigen receptor, such as a TCR, and, optionally, a signal through such a receptor in combination with a costimulatory receptor.

In some embodiments, the CAR typically includes in its extracellular portion one or more antigen binding molecules, such as one or more antigen-binding fragment, domain, or portion, or one or more antibody variable domains and/or antibody molecules of a TCR-like antibody identified by the provided methods. In some embodiments, the CAR includes an antigen-binding portion or portions of an antibody molecule, such as a single-chain antibody fragment (scFv) derived from the variable heavy (VH) and variable light (VL) chains of a monoclonal antibody (mAb).

In some embodiments, the antigen binding molecule of the CAR can further include a spacer, which may be or include at least a portion of an immunoglobulin constant region or variant or modified version thereof, such as a hinge region, e.g., an IgG4 hinge region, and/or a CH1/CL and/or Fc region. In some embodiments, the constant region or portion is of a human IgG, such as IgG4 or IgG1. In some aspects, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. In some examples, the spacer is at or about 12 amino acids in length or is no more than 12 amino acids in length. Exemplary spacers include those having at least about 10 to 229 amino acids, about 10 to 200 amino acids, about 10 to 175 amino acids, about 10 to 150 amino acids, about 10 to 125 amino acids, about 10 to 100 amino acids, about 10 to 75 amino acids, about 10 to 50 amino acids, about 10 to 40 amino acids, about 10 to 30 amino acids, about 10 to 20 amino acids, or about 10 to 15 amino acids, and including any integer between the endpoints of any of the listed ranges. In some embodiments, a spacer region has about 12 amino acids or less, about 119 amino acids or less, or about 229 amino acids or less. Exemplary spacers include IgG4 hinge alone, IgG4 hinge linked to CH2 and CH3 domains, or IgG4 hinge linked to the CH3 domain. Exemplary spacers include, but are not limited to, those described in Hudecek et al. (2013) Clin. Cancer Res., 19:3153 or international patent application publication number WO2014031687, U.S. Patent No. 8,822,647 or published app. no. US2014/0271635.

In some embodiments, the constant region or portion is of a human IgG, such as IgG4 or IgG1. In some embodiments, the spacer has the sequence ESKYGPPCPPCP (set forth in SEQ ID NO: 38), and is encoded by the sequence set forth in SEQ ID NO: 39. In some embodiments, the spacer has the sequence set forth in SEQ ID NO: 40. In some embodiments, the spacer has the sequence set forth in SEQ ID NO: 41. In some embodiments, the constant region or portion is of IgD. In some embodiments, the spacer has the sequence set forth in SEQ ID NO:42. In some embodiments, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 38, 40, 41, or 42.

The antigen recognition domain generally is linked to one or more intracellular signaling components, such as signaling components that mimic activation through an antigen receptor complex, such as a TCR complex, in the case of a CAR, and/or signal via another cell surface receptor. Thus, in some embodiments, the antigen binding molecule (e.g., TCR-like antibody or antigen-binding fragment) is linked to one or more transmembrane and intracellular signaling domains. In some embodiments, the transmembrane domain is fused to the extracellular domain. In one embodiment, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD 16, CD22, CD33, CD37, CD64, CD80, CD86, CD 134, CD137, CD 154. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some embodiments, the linkage is by linkers, spacers, and/or transmembrane domain(s).

In some embodiments, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

The CAR generally includes at least one intracellular signaling component or components. Among the intracellular signaling domains are those that mimic or approximate a signal through a natural antigen receptor, a signal through such a receptor in combination with a costimulatory receptor, and/or a signal through a costimulatory receptor alone. In some embodiments, the CAR includes an intracellular component of the TCR complex, such as a TCR CD3 chain that mediates T-cell activation and cytotoxicity, e.g., CD3 zeta chain. Thus, in some aspects, the antigen binding molecule is linked to one or more cell signaling modules. In some embodiments, cell signaling modules include CD3 transmembrane domain, CD3 intracellular signaling domains, and/or other CD transmembrane domains. In some embodiments, the CAR further includes a portion of one or more additional molecules such as Fc receptor γ, CD8, CD4, CD25, or CD16. For example, in some aspects, the CAR includes a chimeric molecule between CD3-zeta (CD3-ζ) or Fc receptor γ and CD8, CD4, CD25 or CD16.

In some embodiments, upon ligation of the CAR, the cytoplasmic domain or intracellular signaling domain of the CAR activates at least one of the normal effector functions or responses of the immune cell, e.g., T cell engineered to express the cell. For example, in some contexts, the CAR induces a function of a T cell such as cytolytic activity or T-helper activity, such as secretion of cytokines or other factors. In some embodiments, a truncated portion of an intracellular signaling domain of an antigen receptor component or costimulatory molecule is used in place of an intact immunostimulatory chain, for example, if it transduces the effector function signal. In some embodiments, the intracellular signaling domain or domains include the cytoplasmic sequences of the T cell receptor (TCR), and in some aspects also those of co-receptors that in the natural context act in concert with such receptor to initiate signal transduction following antigen receptor engagement, and/or any derivative or variant of such molecules, and/or any synthetic sequence that has the same functional capability.

In the context of a natural TCR, full activation generally requires not only signaling through the TCR, but also a costimulatory signal. Thus, in some embodiments, to promote full activation, a component for generating secondary or co-stimulatory signal is also included in the CAR. In other embodiments, the CAR does not include a component for generating a costimulatory signal. In some aspects, an additional CAR is expressed in the same cell and provides the component for generating the secondary or costimulatory signal. In some aspects, the cell comprises a first CAR which contains signaling domains to induce the primary signal and a second CAR which binds to a second antigen and contains the component for generating a costimulatory signal. For example, a first CAR can be an activating CAR and the second CAR can be a costimulatory CAR. In some aspects, both CARs must be ligated in order to induce a particular effector function in the cell, which can provide specificity and selectivity for the cell type being targeted.

T cell activation is in some aspects described as being mediated by two classes of cytoplasmic signaling sequences: those that initiate antigen- dependent primary activation through the TCR (primary cytoplasmic signaling sequences), and those that act in an antigen-independent manner to provide a secondary or co-stimulatory signal (secondary cytoplasmic signaling sequences). In some aspects, the CAR includes one or both of such signaling components.

In some aspects, the CAR includes a primary cytoplasmic signaling sequence that regulates primary activation of the TCR complex. Examples of primary cytoplasmic signaling sequences include those derived from CD3 zeta, FcR gamma, CD3 gamma, CD3 delta and CD3 epsilon. In some embodiments, cytoplasmic signaling molecule(s) in the CAR contain(s) a cytoplasmic signaling domain, portion thereof, or sequence derived from CD3 zeta.

In some embodiments, the CAR includes a signaling domain and/or transmembrane portion of a costimulatory receptor, such as CD28, 4-1BB, OX40, DAP10, and ICOS. In some aspects, the same CAR includes both the activating and costimulatory components; in other aspects, the activating domain is provided by one CAR whereas the costimulatory component is provided by another CAR recognizing another antigen.

In some embodiments, the activating domain is included within one CAR, whereas the costimulatory component is provided by another CAR recognizing another antigen. In some embodiments, the CARs include activating or stimulatory CARs, and costimulatory CARs, both expressed on the same cell (*see* WO2014/055668). In some aspects, the TCR-like CAR is the stimulatory or activating CAR; in other aspects, it is the costimulatory CAR. In some embodiments, the cells further include inhibitory CARs (iCARs, *see* Fedorov et al., Sci. Transl. Medicine, 5(215) (December, 2013), such as a CAR recognizing an antigen other than the particular MHC-peptide complex recognized by the TCR-like antibody, whereby an activating signal delivered through the TCR-like CAR is diminished or inhibited by binding of the inhibitory CAR to its ligand, e.g., to reduce off-target effects.

In certain embodiments, the intracellular signaling domain comprises a CD28 transmembrane and signaling domain linked to a CD3 (e.g., CD3-zeta) intracellular domain. In some embodiments, the intracellular signaling domain comprises a chimeric CD28 and CD137 (4-1BB, TNFRSF9) co-stimulatory domains, linked to a CD3 zeta intracellular domain.

In some embodiments, the TCR-like CAR encompasses two or more costimulatory domain combined with an activation domain, e.g., primary activation domain, in the cytoplasmic portion. One example is a receptor including intracellular components of CD3-zeta, CD28, and 4-1BB.

In some embodiments, the CAR or other antigen receptor further includes a marker, which may be used to confirm transduction or engineering of the cell to express the receptor. In some embodiments, the marker is a molecule, e.g., cell surface protein, not naturally found on T cells or not naturally found on the surface of T cells, or a portion thereof. In some embodiments, the molecule is a non-self molecule, e.g., non-self protein, i.e., one that is not recognized as "self' by the immune system of the host into which the cells will be adoptively transferred. In some embodiments, the marker serves no therapeutic function and/or produces no effect other than to be used as a marker for genetic engineering, e.g., for selecting cells successfully engineered. In other embodiments, the marker may be a therapeutic molecule or molecule otherwise exerting some desired effect, such as a ligand for a cell to be encountered in vivo, such as a costimulatory or immune checkpoint molecule to enhance and/or dampen responses of the cells upon adoptive transfer and encounter with ligand.

In some aspects, the marker includes all or part (e.g., truncated form) of CD34, a NGFR, or epidermal growth factor receptor (e.g., tEGFR). In some embodiments, the marker is a truncated version of a cell surface receptor, such as truncated EGFR, i.e. tEGFR. An exemplary polypeptide for a truncated EGFR (e.g. tEGFR) comprises the sequence of amino acids set forth in SEQ ID NO: 43 or 61, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 43 or 61. In some embodiments, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (tEGFR) that is, optionally, linked to a linker sequence, such as a T2A cleavable linker sequence. An exemplary T2A linker sequence comprises the sequence of amino acids set forth in SEQ ID NO: 44 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 44.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR in some aspects is one that include multiple costimulatory domains of different costimulatory receptors.

In some embodiments, the chimeric antigen receptor includes an extracellular portion containing a TCR-like antibody or fragment identified in the provided methods and an intracellular signaling domain. In some embodiments, the antibody or fragment includes an scFv and the intracellular domain contains an ITAM. In some aspects, the intracellular signaling domain includes a signaling domain of a zeta chain of a CD3-zeta (CD3ζ) chain. In some embodiments, the chimeric antigen receptor includes a transmembrane domain linking the extracellular domain and the intracellular signaling domain. In some aspects, the transmembrane domain contains a transmembrane portion of CD28. The extracellular domain and transmembrane can be linked directly or indirectly. In some embodiments, the extracellular domain and transmembrane are linked by a spacer, such as any described herein. In some embodiments, the chimeric antigen receptor contains an intracellular domain of a T cell costimulatory molecule, such as between the transmembrane domain and intracellular signaling domain. In some aspects, the T cell costimulatory molecule is CD28 or 41BB.

For example, in some embodiments, the CAR contains a TCR-like antibody, e.g., an antibody fragment, as provided herein, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some embodiments, the CAR contains a TCR-like antibody, e.g. Antibody fragment, as provided herein, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such embodiments, the receptor further includes a spacer containing a portion of an Ig molecule, such as a human Ig molecule, such as an Ig hinge, e.g. an IgG4 hinge, such as a hinge-only spacer.

In some embodiments, the transmembrane domain of the receptor, e.g., the TCR-like CAR, is a transmembrane domain of human CD28 (e.g. Accession No. P01747.1) or variant thereof, such as a transmembrane domain that comprises the sequence of amino acids set forth in SEQ ID NO: 45 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 45. In some embodiments, the transmembrane-domain containing portion of the recombinant receptor comprises the sequence of amino acids set forth in SEQ ID NO: 46 or a sequence of amino acids having at least at or about 85%, 86%, 87%, 88%, 89%, 90%, 91%, 92%, 93%, 94%, 95%, 96%, 97%, 98%, 99% or more sequence identity to SEQ ID NO: 46.

In some embodiments, the intracellular signaling component(s) of the recombinant receptor, *e.g.* the TCR-like CAR, contains an intracellular costimulatory signaling domain of human CD28 or a functional variant or portion thereof, such as a domain with an LL to GG substitution at positions 186-187 of a native CD28 protein. For example, the intracellular signaling domain can comprise the sequence of amino acids set forth in SEQ ID NO: 47 or 48 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 47 or 48. In some embodiments, the intracellular domain comprises an intracellular costimulatory signaling domain of 4-1BB (e.g. (Accession No. Q07011.1) or functional variant or portion thereof, such as the sequence of amino acids set forth in SEQ ID NO: 49 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 49.

In some embodiments, the intracellular signaling domain of the recombinant receptor, *e.g.* the CAR, comprises a human CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. For example, in some embodiments, the intracellular signaling domain comprises the sequence of amino acids set forth in SEQ ID NO: 50, 51 or 52 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 50, 51, or 52.

In some aspects, the spacer contains only a hinge region of an IgG, such as only a hinge of IgG4 or IgG1, such as the hinge only spacer set forth in SEQ ID NO: 38. In other embodiments, the spacer is or contains an Ig hinge, e.g., an IgG4-derived hinge, optionally linked to a CH2 and/or CH3 domains. In some embodiments, the spacer is an Ig hinge, e.g., an IgG4 hinge, linked to CH2 and CH3 domains, such as set forth in SEQ ID NO: 40. In some embodiments, the spacer is an Ig hinge, e.g., an IgG4 hinge, linked to a CH3 domain only, such as set forth in SEQ ID NO: 41. In some embodiments, the spacer is or comprises a glycine-serine rich sequence or other flexible linker such as known flexible linkers.

For example, in some embodiments, the TCR-like CAR includes a TCR-like antibody or fragment, such as any identified in the methods provided herein, including scFvs, a spacer such as any of the Ig-hinge containing spacers, a CD28 transmembrane domain, a CD28 intracellular signaling domain, and a CD3 zeta signaling domain. In some embodiments, the TCR-like CAR includes the a TCR-like antibody or fragment, such as any identified in the methods provided herein, including scFvs, a spacer such as any of the Ig-hinge containing spacers, a CD28 transmembrane domain, a CD28 intracellular signaling domain, and a CD3 zeta signaling domain. In some embodiments, such TCR-like CAR constructs further includes a T2A ribosomal skip element and/or a tEGFR sequence, e.g., downstream of the CAR.

In some embodiments, such CAR constructs further includes a T2A ribosomal skip element and/or a tEGFR sequence, e.g., downstream of the CAR, such as set forth in SEQ ID NO: 44 and/or 43, respectively, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 44 or 43.

### B. Engineered Cells

Also provided are cells, e.g. engineered cells, engineered to contain a transgenic antigen receptor containing a binding molecule for specific recognition of a peptide epitope in the context of an MHC molecule, i.e. an MHC-peptide complex. Among such cells are cells engineered with a transgenic TCR or TCR-like CAR. In some embodiments, the peptide epitope is an MHC-restricted epitope of an antigen, including an intracellular antigen, such as any MHC-restricted antigen associated with malignancy or transformation of cells (e.g. cancer), an autoimmune or inflammatory disease, or an antigen derived from an infectious disease, e.g. viral pathogen or a bacterial pathogen. Also provided are populations of such cells and composition containing the cell or population of cells. Among the compositions are pharmaceutical compositions and formulations for administration, such as for adoptive cell therapy. Also provided are therapeutic methods for administering the cells and compositions to subjects, e.g., patients.

The cells generally are eukaryotic cells, such as mammalian cells, and typically are human cells. In some embodiments, the cells are derived from the blood, bone marrow, lymph, or lymphoid organs, are cells of the immune system, such as cells of the innate or adaptive immunity, *e.g*., myeloid or lymphoid cells, including lymphocytes, typically T cells and/or NK cells. Other exemplary cells include stem cells, such as multipotent and pluripotent stem cells, including induced pluripotent stem cells (iPSCs). In some embodiments, the cells are monocytes or granulocytes, e.g., myeloid cells, macrophages, neutrophils, dendritic cells, mast cells, eosinophils, and/or basophils. The cells typically are primary cells, such as those isolated directly from a subject and/or isolated from a subject and frozen. With reference to the subject to be treated, the cells may be allogeneic and/or autologous. Among the methods include off-the-shelf methods. In some aspects, such as for off-the-shelf technologies, the cells are pluripotent and/or multipotent, such as stem cells, such as induced pluripotent stem cells (iPSCs). In some embodiments, the methods include isolating cells from the subject, preparing, processing, culturing, and/or engineering them, as described herein, and re-introducing them into the same patient, before or after cryopreservation.

In some embodiments, the cells include one or more subsets of T cells or other cell types, such as whole T cell populations, CD4+ cells, CD8+ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. Among the sub-types and subpopulations of T cells and/or of CD4+ and/or of CD8+ T cells are naive T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some embodiments, the cells are CD8+ T cells and such cells are engineered with an antigen receptor, e.g. TCR or TCR-like CAR, that specifically binds to a peptide epitope in the context of an MHC class I molecule. In some cases, the MHC class I molecule is a classical MHC class I molecule or a non-classical MHC class I molecule. In some embodiments, the MHC class I molecule is an MHC-E. In some embodiments, methods are provided for engineering CD8+ cells to express an engineered antigen receptor by introducing into such cells one or more nucleic acid molecules encoding a TCR or TCR-like CAR specific for a peptide epitope in the context of an MHC class I molecule, e.g. an MHC class Ia molecule and/or an MHC-E molecule.

In some embodiments, the cells are CD8+ T cells and such cells are engineered with an antigen receptor, e.g. TCR or TCR-like CAR, that specifically binds to a peptide epitope in the context of an MHC class II molecule. In some embodiments, methods are provided for engineering CD8+ cells to express an engineered antigen receptor by introducing into such cells one or more nucleic acid molecules encoding a TCR or TCR-like CAR specific for a peptide epitope in the context of an MHC class II molecule.

In some embodiments, the cells are CD4+ T cells and such cells are engineered with an antigen receptor, e.g. TCR or TCR-like CAR, that specifically binds a peptide epitope in the context of an MHC class II molecule. In some embodiments, methods are provided for engineering CD4+ cells to express an engineered antigen receptor by introducing into such cells one or more nucleic acid molecules encoding a TCR or TCR-like CAR specific for a peptide epitope in the context of an MHC class II molecule.

In some embodiments, provided are CD4+ cells and CD8+ cells engineered with an antigen receptor, e.g. TCR or TCR-like CAR, that specifically binds to a peptide epitope in the context of an MHC class II molecule. In some embodiments, the antigen receptor expressed on the CD4+ cells and CD8+ cells is the same. In some embodiments, the antigen receptor expressed on CD4+ cells is different from the antigen receptor expressed on CD8+ cells, but both expressed antigen receptors specifically bind to a peptide epitope in the context of an MHC class II molecule. In some embodiments, methods are provided for engineering a population of CD4+ and/or CD8+ cells to express an engineered antigen receptor by introducing into such cells one or more nucleic acid molecules encoding a TCR or TCR-like CAR specific for a peptide epitope in the context of an MHC class II molecule.

In some embodiments, the binding molecule of the engineered antigen receptor, e.g. TCR or TCR-like antibody or antigen-binding fragments thereof, is one identified by the provided methods.

### Cells for engineering

In some embodiments, preparation of the engineered cells includes one or more culture and/or preparation steps. The cells for introduction of the antigen receptor, e.g., TCR or TCR-like CAR, may be isolated from a sample, such as a biological sample, e.g., one obtained from or derived from a subject. In some embodiments, the subject from which the cell is isolated is one having the disease or condition or in need of a cell therapy or to which cell therapy will be administered. The subject in some embodiments is a human in need of a particular therapeutic intervention, such as the adoptive cell therapy for which cells are being isolated, processed, and/or engineered.

Accordingly, the cells in some embodiments are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some embodiments, the cells are derived from cell lines, e.g., T cell lines. The cells in some embodiments are obtained from a xenogeneic source, for example, from mouse, rat, non-human primate, and pig.

In some embodiments, isolation of the cells includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contain cells other than red blood cells and platelets.

In some embodiments, the blood cells collected from the subject are washed, e.g., to remove the plasma fraction and to place the cells in an appropriate buffer or media for subsequent processing steps. In some embodiments, the cells are washed with phosphate buffered saline (PBS). In some embodiments, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some embodiments, the cells are resuspended in a variety of biocompatible buffers after washing, such as, for example, Ca⁺⁺/Mg⁺⁺ free PBS. In certain embodiments, components of a blood cell sample are removed and the cells directly resuspended in culture media.

In some embodiments, the methods include density-based cell separation methods, such as the preparation of white blood cells from peripheral blood by lysing the red blood cells and centrifugation through a Percoll or Ficoll gradient.

In some embodiments, the isolation methods include the separation of different cell types based on the expression or presence in the cell of one or more specific molecules, such as surface markers, e.g., surface proteins, intracellular markers, or nucleic acid. In some embodiments, any known method for separation based on such markers may be used. In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, e.g., CD28⁺, CD62L⁺, CCR7⁺, CD27⁺, CD127⁺, CD4⁺, CD8⁺, CD45RA⁺, and/or CD45RO⁺ T cells, are isolated by positive or negative selection techniques.

For example, CD3⁺, CD28⁺ T cells can be positively selected using anti-CD3/anti-CD28-conjugated magnetic beads.

In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In some embodiments, T cells are separated from a PBMC sample by negative selection of markers expressed on non-T cells, such as B cells, monocytes, or other white blood cells, such as CD14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some embodiments, CD8⁺ cells are further enriched for or depleted of naive, central memory, effector memory, and/or central memory stem cells, such as by positive or negative selection based on surface antigens associated with the respective subpopulation. In some embodiments, enrichment for central memory T (T_{CM}) cells is carried out to increase efficacy, such as to improve long-term survival, expansion, and/or engraftment following administration, which in some aspects is particularly robust in such sub-populations. *See* Terakura et al. (2012) Blood. 1:72-82; Wang et al. (2012) J Immunother. 35(9):689-701. In some embodiments, combining T_{CM}-enriched CD8⁺ T cells and CD4⁺ T cells further enhances efficacy.

In embodiments, memory T cells are present in both CD62L⁺ and CD62L⁻ subsets of CD8⁺ peripheral blood lymphocytes. PBMC can be enriched for or depleted of CD62L⁻CD8⁺ and/or CD62L⁺CD8⁺ fractions, such as using anti-CD8 and anti-CD62L antibodies.

In some embodiments, the enrichment for central memory T (T_{CM}) cells is based on positive or high surface expression of CD45RO, CD62L, CCR7, CD28, CD3, and/or CD 127; in some aspects, it is based on negative selection for cells expressing or highly expressing CD45RA and/or granzyme B. In some aspects, isolation of a CD8⁺ population enriched for T_{CM} cells is carried out by depletion of cells expressing CD4, CD14, CD45RA, and positive selection or enrichment for cells expressing CD62L. In one aspect, enrichment for central memory T (T_{CM}) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8⁺ cell population or subpopulation, also is used to generate the CD4⁺ cell population or subpopulation, such that both the positive and negative fractions from the CD4-based separation are retained and used in subsequent steps of the methods, optionally following one or more further positive or negative selection steps.

In a particular example, a sample of PBMCs or other white blood cell sample is subjected to selection of CD4⁺ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4⁺ T helper cells are sorted into naive, central memory, and effector cells by identifying cell populations that have cell surface antigens. CD4⁺ lymphocytes can be obtained by standard methods. In some embodiments, naive CD4⁺ T lymphocytes are CD45RO⁻, CD45RA⁺, CD62L⁺, CD4⁺ T cells. In some embodiments, central memory CD4⁺ cells are CD62L⁺ and CD45RO⁺. In some embodiments, effector CD4⁺ cells are CD62L⁻ and CD45RO⁻.

In one example, to enrich for CD4⁺ cells by negative selection, a monoclonal antibody cocktail typically includes antibodies to CD14, CD20, CD11b, CD16, HLA-DR, and CD8. In some embodiments, the antibody or binding partner is bound to a solid support or matrix, such as a magnetic bead or paramagnetic bead, to allow for separation of cells for positive and/or negative selection. For example, in some embodiments, the cells and cell populations are separated or isolated using immune-magnetic (or affinity-magnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some aspects, the sample or composition of cells to be separated is incubated with small, magnetizable or magnetically responsive material, such as magnetically responsive particles or microparticles, such as paramagnetic beads (e.g., such as Dynabeads or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some embodiments, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B, which are hereby incorporated by reference. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In certain embodiments, the magnetically responsive particles are coated in primary antibodies or other binding partners, secondary antibodies, lectins, enzymes, or streptavidin. In certain embodiments, the magnetic particles are attached to cells via a coating of primary antibodies specific for one or more markers. In certain embodiments, the cells, rather than the beads, are labeled with a primary antibody or binding partner, and then cell-type specific secondary antibody- or other binding partner (e.g., streptavidin)-coated magnetic particles, are added. In certain embodiments, streptavidin-coated magnetic particles are used in conjunction with biotinylated primary or secondary antibodies.

In some embodiments, the magnetically responsive particles are left attached to the cells that are to be subsequently incubated, cultured and/or engineered; in some aspects, the particles are left attached to the cells for administration to a patient. In some embodiments, the magnetizable or magnetically responsive particles are removed from the cells. Methods for removing magnetizable particles from cells are known and include, e.g., the use of competing non-labeled antibodies, magnetizable particles or antibodies conjugated to cleavable linkers, etc. In some embodiments, the magnetizable particles are biodegradable.

In some embodiments, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain embodiments, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain embodiments, the non-target cells are labelled and depleted from the heterogeneous population of cells.

In certain embodiments, the isolation or separation is carried out using a system, device, or apparatus that carries out one or more of the isolation, cell preparation, separation, processing, incubation, culture, and/or formulation steps of the methods. In some aspects, the system is used to carry out each of these steps in a closed or sterile environment, for example, to minimize error, user handling and/or contamination. In one example, the system is a system as described in International Patent Application, Publication Number WO2009/072003, or US 20110003380 A1.

In some embodiments, the system or apparatus carries out one or more, e.g., all, of the isolation, processing, engineering, and formulation steps in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some aspects, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some embodiments, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some embodiments, the cell populations for use with the methods described herein are unlabeled and are not retained in the column. In some embodiments, the cell populations for use with the methods described herein are labeled and are retained in the column. In some embodiments, the cell populations for use with the methods described herein are eluted from the column after removal of the magnetic field, and are collected within the cell collection bag.

In certain embodiments, separation and/or other steps are carried out using the CliniMACS Prodigy system (Miltenyi Biotec). The CliniMACS Prodigy system in some aspects is equipped with a cell processing unit that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakura et al. (2012) Blood. 1:72-82, and Wang et al. (2012) J Immunother. 35(9):689-701.

In some embodiments, a cell population described herein is collected and enriched (or depleted) via flow cytometry, in which cells stained for multiple cell surface markers are carried in a fluidic stream. In some embodiments, a cell population described herein is collected and enriched (or depleted) via preparative scale (FACS)-sorting. In certain embodiments, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5):355-376. In both cases, cells can be labeled with multiple markers, allowing for the isolation of well-defined T cell subsets at high purity.

In some embodiments, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some embodiments, the preparation methods include steps for freezing, e.g., cryopreserving, the cells, either before or after isolation, incubation, and/or engineering. In some embodiments, the freeze and subsequent thaw step removes granulocytes and, to some extent, monocytes in the cell population. In some embodiments, the cells are suspended in a freezing solution, e.g., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1:1 with media so that the final concentration of DMSO and HSA are 10% and 4%, respectively. The cells are then frozen to -80° C. at a rate of 1° per minute and stored in the vapor phase of a liquid nitrogen storage tank.

### Preparation of Cells, Vectors and Methods of Engineering

In some embodiments, the genetic engineering generally involves introduction of a nucleic acid encoding the recombinant or engineered component into the cell, such as by retroviral transduction, transfection, or transformation. In some embodiments, gene transfer is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

In some embodiments, the cells are incubated and/or cultured prior to or in connection with genetic engineering. The incubation steps can include culture, cultivation, stimulation, activation, and/or propagation. In some embodiments, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor. The incubation and/or engineering may be carried out in a culture vessel, such as a unit, chamber, well, column, tube, tubing set, valve, vial, culture dish, bag, or other container for culture or cultivating cells.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some embodiments, the stimulating conditions or agents include one or more agent, e.g., ligand, which is capable of activating an intracellular signaling domain of a TCR complex. In some aspects, the agent turns on or initiates TCR/CD3 intracellular signaling cascade in a T cell. Such agents can include antibodies, such as those specific for a TCR, e.g. anti-CD3. In some embodiments, the stimulating conditions include one or more agent, e.g. ligand, which is capable of stimulating a costimulatory receptor, e.g., anti-CD28. In some embodiments, such agents and/or ligands may be, bound to solid support such as a bead, and/or one or more cytokines. Optionally, the expansion method may further comprise the step of adding anti-CD3 and/or anti CD28 antibody to the culture medium (e.g., at a concentration of at least about 0.5 ng/ml). In some embodiments, the stimulating agents include IL-2, IL-15 and/or IL-7. In some aspects, the IL-2 concentration is at least about 10 units/mL.

In some aspects, incubation is carried out in accordance with techniques such as those described in US Patent No. 6,040,1 77 to Riddell et al., Klebanoff et al.(2012) J Immunother. 35(9): 651-660, Terakura et al. (2012) Blood. 1:72-82, and/or Wang et al. (2012) J Immunother. 35(9):689-701.

In some embodiments, the T cells are expanded by adding to the composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some aspects, the non-dividing feeder cells can comprise gamma-irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells.

In some embodiments, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some aspects is provided in any suitable amount, such as a ratio of LCL feeder cells to initial T lymphocytes of at least about 10:1.

In some aspects, the cells further are engineered to promote expression of cytokines or other factors.

In some contexts, overexpression of a stimulatory factor (for example, a lymphokine or a cytokine) may be toxic to a subject. Thus, in some contexts, the engineered cells include gene segments that cause the cells to be susceptible to negative selection in vivo, such as upon administration in adoptive immunotherapy. For example in some aspects, the cells are engineered so that they can be eliminated as a result of a change in the in vivo condition of the patient to which they are administered. The negative selectable phenotype may result from the insertion of a gene that confers sensitivity to an administered agent, for example, a compound. Negative selectable genes include the Herpes simplex virus type I thymidine kinase (HSV-I TK) gene (Wigler et al., Cell 2:223, 1977) which confers ganciclovir sensitivity; the cellular hypoxanthine phosphribosyltransferase (HPRT) gene, the cellular adenine phosphoribosyltransferase (APRT) gene, bacterial cytosine deaminase, (Mullen et al., Proc. Natl. Acad. Sci. USA. 89:33 (1992)).

Various methods for the introduction of genetically engineered components, e.g., antigen receptors, e.g., CARs, are well known and may be used with the provided methods and compositions. Exemplary methods include those for transfer of nucleic acids encoding the receptors, including via viral, e.g., retroviral or lentiviral, transduction, transposons, and electroporation.

In some embodiments, recombinant nucleic acids are transferred into cells using recombinant infectious virus particles, such as, e.g., vectors derived from simian virus 40 (SV40), adenoviruses, adeno-associated virus (AAV). In some embodiments, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some embodiments, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some embodiments, the retroviruses include those derived from any avian or mammalian cell source. The retroviruses typically are amphotropic, meaning that they are capable of infecting host cells of several species, including humans. In one embodiment, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637― 1644; Verhoeyen et al. (2009) Methods Mol Biol. 506: 97-114; and Cavalieri et al. (2003) Blood. 102(2): 497-505.

In some embodiments, recombinant nucleic acids are transferred into T cells via electroporation (*see,* e.g., Chicaybam et al, (2013) PLoS ONE 8(3): e60298 and Van Tedeloo et al. (2000) Gene Therapy 7(16): 1431-1437). In some embodiments, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

Among additional nucleic acids, e.g., genes for introduction are those to improve the efficacy of therapy, such as by promoting viability and/or function of transferred cells; genes to provide a genetic marker for selection and/or evaluation of the cells, such as to assess *in vivo* survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. *See,* e.g., Riddell et al., US Patent No. 6,040,177, at columns 14-17.

### V. Compositions, Formulations and Methods of Administration

Also provided are compositions containing the TCR, TCR-like antibody binding molecules or antigen fragments thereof, chimeric receptors (e.g. CARs) and compositions containing the engineered cells, including pharmaceutical compositions and formulations. Also provided are methods of using and uses of the molecules and compositions, such as in the treatment of diseases, conditions, and disorders in which the antigen is expressed, or in detection, diagnostic, and prognostic methods.

### A. Pharmaceutical Compositions and Formulations

Provided are pharmaceutical formulations including TCR or TCR-like binding molecules or antigen-binding fragments, including chimeric receptors (e.g. CARs) and/or the engineered cells expressing the molecules or antigen receptors. For example, in some embodiments, provided are pharmaceutical compositions and formulations including engineered CD4+ and/or CD8+ T cells expressing an antigen receptor or a chimeric antigen receptor, such as a TCR or TCR-like CAR, targeting an MHC-restricted epitope, such as an MHC-class Ia-, MHC-E-. or MHC class II-restricted epitope. Exemplary of such are pharmaceutical compositions and formulations including CD4+ and CD8+ cells that are engineered to express an antigen receptor, e.g. TCR or TCR-like CAR, that target the same MHC class II-restricted epitope.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some aspects, the choice of carrier is determined in part by the particular cell, binding molecule, and/or antibody, and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

Formulations can include lyophilized formulations and aqueous solutions.

The formulation or composition may also contain more than one active ingredient useful for the particular indication, disease, or condition being treated with the binding molecules or cells, preferably those with activities complementary to the binding molecule or cell, where the respective activities do not adversely affect one another. Such active ingredients are suitably present in combination in amounts that are effective for the purpose intended. Thus, in some embodiments, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. In some embodiments, the cells or antibodies are administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and aryl sulphonic acids, for example, p-toluenesulfonic acid.

Active ingredients may be entrapped in microcapsules, in colloidal drug delivery systems (for example, liposomes, albumin microspheres, microemulsions, nano-particles and nanocapsules) or in macroemulsions. In certain embodiments, the pharmaceutical composition is formulated as an inclusion complex, such as cyclodextrin inclusion complex, or as a liposome. Liposomes can serve to target the host cells (e.g., T-cells or NK cells) to a particular tissue. Many methods are available for preparing liposomes, such as those described in, for example, Szoka et al., Ann. Rev. Biophys. Bioeng., 9: 467 (1980), and U.S. Patents 4,235,871, 4,501,728, 4,837,028, and 5,019,369.

The pharmaceutical composition in some aspects can employ time-released, delayed release, and sustained release delivery systems such that the delivery of the composition occurs prior to, and with sufficient time to cause, sensitization of the site to be treated. Many types of release delivery systems are available and known. Such systems can avoid repeated administrations of the composition, thereby increasing convenience to the subject and the physician.

The pharmaceutical composition in some embodiments contains the binding molecules and/or cells in amounts effective to treat or prevent the disease or condition, such as a therapeutically effective or prophylactically effective amount. Therapeutic or prophylactic efficacy in some embodiments is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The cells may be administered using standard administration techniques, formulations, and/or devices. Provided are formulations and devices, such as syringes and vials, for storage and administration of the compositions. Administration of the cells can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some embodiments, the cell populations are administered parenterally. The term "parenteral," as used herein, includes intravenous, intramuscular, subcutaneous, rectal, vaginal, and intraperitoneal administration. In some embodiments, the cell populations are administered to a subject using peripheral systemic delivery by intravenous, intraperitoneal, or subcutaneous injection.

Compositions in some embodiments are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the binding molecule in a solvent, such as in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin.

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for in vivo administration are generally sterile. Sterility may be readily accomplished, e.g., by filtration through sterile filtration membranes.

### B. Methods of Administration and Uses of cells in adoptive cell therapy

Also provided are methods for using and uses of the TCR or TCR-like binding molecules or antigen-binding fragments, including chimeric receptors and/or engineered cells expressing the molecules or antigen receptors. Such methods and uses include therapeutic methods and uses, for example, involving administration of the molecules, cells, or compositions containing the same, to a subject for targeting MHC-restricted peptide epitopes of an antigen associated with a disease, condition, or disorder, including antigens involved in malignancy or transformation of cells (e.g. cancer), an autoimmune or inflammatory disease, or an antigen derived from a viral pathogen or a bacterial pathogen.

In some embodiments, the molecule, cell, and/or composition is administered in an effective amount to effect treatment of the disease or disorder. Uses include uses of the molecules or cells in such methods and treatments, and in the preparation of a medicament in order to carry out such therapeutic methods. In some embodiments, the methods are carried out by administering the molecules or cells, or compositions comprising the same, to the subject having or suspected of having the disease or condition. In some embodiments, the methods thereby treat the disease or condition or disorder in the subject.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some embodiments, the provided molecules and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, an antibody or composition or cell which suppresses tumor growth reduces the rate of growth of the tumor compared to the rate of growth of the tumor in the absence of the antibody or composition or cell.

An "effective amount" of an agent, *e.g.,* a pharmaceutical formulation, binding molecule, antibody, or cells, or composition, in the context of administration, refers to an amount effective, at dosages/amounts and for periods of time necessary, to achieve a desired result, such as a therapeutic or prophylactic result.

A "therapeutically effective amount" of an agent, *e.g.,* a pharmaceutical formulation, antibody, or cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the populations of cells administered. In some embodiments, the provided methods involve administering the molecules, cells, and/or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The disease or condition that is treated can be any in which expression of an antigen is associated with and/or involved in the etiology of a disease condition or disorder, e.g. causes, exacerbates or otherwise is involved in such disease, condition, or disorder. Exemplary diseases and conditions can include diseases or conditions associated with malignancy or transformation of cells (e.g. cancer), autoimmune or inflammatory disease, or an infectious disease, e.g. caused by a bacterial, viral or other pathogen. Exemplary antigens, which include antigens associated with various diseases and conditions that can be treated, are described above. In particular embodiments, the chimeric antigen receptor or transgenic TCR specifically binds to an antigen associated with the disease or condition.

In some embodiments, the methods include adoptive cell therapy, whereby genetically engineered cells expressing the provided molecules targeting an MHC-restricted epitope (e.g., cells expressing a TCR or TCR-like CAR) are administered to subjects. Such administration can promote activation of the cells (e.g., T cell activation) in an antigen -targeted manner, such that the cells of the disease or disorder are targeted for destruction.

Thus, the provided methods and uses include methods and uses for adoptive cell therapy. In some embodiments, the methods include administration of the cells or a composition containing the cells to a subject, tissue, or cell, such as one having, at risk for, or suspected of having the disease, condition or disorder. In some embodiments, the cells, populations, and compositions are administered to a subject having the particular disease or condition to be treated, e.g., via adoptive cell therapy, such as adoptive T cell therapy. In some embodiments, the cells or compositions are administered to the subject, such as a subject having or at risk for the disease or condition. In some aspects, the methods thereby treat, e.g., ameliorate one or more symptom of the disease or condition.

Methods for administration of cells for adoptive cell therapy are known and may be used in connection with the provided methods and compositions. For example, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

In some embodiments, the cell therapy, e.g., adoptive cell therapy, e.g., adoptive T cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

In some embodiments, the cell therapy, e.g., adoptive cell therapy, e.g., adoptive T cell therapy, is carried out by allogeneic transfer, in which the cells are isolated and/or otherwise prepared from a subject other than a subject who is to receive or who ultimately receives the cell therapy, e.g., a first subject. In such embodiments, the cells then are administered to a different subject, e.g., a second subject, of the same species. In some embodiments, the first and second subjects are genetically identical. In some embodiments, the first and second subjects are genetically similar. In some embodiments, the second subject expresses the same HLA class or supertype as the first subject.

In some embodiments, the subject, to whom the cells, cell populations, or compositions are administered, is a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent. In some examples, the patient or subject is a validated animal model for disease, adoptive cell therapy, and/or for assessing toxic outcomes such as cytokine release syndrome (CRS).

The binding molecules, such as TCRs, TCR-like antibodies and chimeric receptors (e.g. CARs) containing the TCR-like antibodies and cells expressing the same, can be administered by any suitable means, for example, by injection, e.g., intravenous or subcutaneous injections, intraocular injection, periocular injection, subretinal injection, intravitreal injection, trans-septal injection, subscleral injection, intrachoroidal injection, intracameral injection, subconjunctival injection, subconjunctival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration. Dosing and administration may depend in part on whether the administration is brief or chronic. Various dosing schedules include but are not limited to single or multiple administrations over various time-points, bolus administration, and pulse infusion.

For the prevention or treatment of disease, the appropriate dosage of the binding molecule or cell may depend on the type of disease to be treated, the type of binding molecule, the severity and course of the disease, whether the binding molecule is administered for preventive or therapeutic purposes, previous therapy, the patient's clinical history and response to the binding molecule, and the discretion of the attending physician. The compositions and molecules and cells are in some embodiments suitably administered to the patient at one time or over a series of treatments.

Depending on the type and severity of the disease, dosages of a binding molecule (e.g. TCR or TCR-like antibody) may include about 1 µg/kg to 15 mg/kg (e.g. 0.1mg/kg-10mg/kg), about 1 µg/kg to 100 mg/kg or more, about 0.05 mg/kg to about 10 mg/kg, 0.5 mg/kg, 2.0 mg/kg, 4.0 mg/kg or 10 mg/kg. Multiple doses may be administered intermittently, e.g. every week or every three weeks. An initial higher loading dose, followed by one or more lower doses may be administered.

In certain embodiments, in the context of genetically engineered cells containing the binding molecules, a subject is administered the range of about one million to about 100 billion cells and/or that amount of cells per kilogram of body weight, such as, e.g., 1 million to about 50 billion cells (e.g., about 5 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some cases about 100 million cells to about 50 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges and/or per kilogram of body weight. Again, dosages may vary depending on attributes particular to the disease or disorder and/or patient and/or other treatments.

In some embodiments, the cells or binding molecules (e.g. TCR or TCR-like antibodies) are administered as part of a combination treatment, such as simultaneously with or sequentially with, in any order, another therapeutic intervention, such as another antibody or engineered cell or receptor or agent, such as a cytotoxic or therapeutic agent.

The cells or binding molecules (e.g. TCR or TCR-like antibodies) in some embodiments are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some embodiments, the cells or binding molecules (e.g. TCR or TCR-like antibodies) are administered prior to the one or more additional therapeutic agents. In some embodiments, the cells or binding molecules (e.g. TCR or TCR-like antibodies) are administered after to the one or more additional therapeutic agents.

Once the cells are administered to a mammal (e.g., a human), the biological activity of the engineered cell populations and/or binding molecules (e.g. TCR or TCR-like antibodies) in some aspects is measured by any of a number of known methods. Parameters to assess include specific binding of an engineered or natural T cell or other immune cell to antigen, in vivo, e.g., by imaging, or ex vivo, e.g., by ELISA or flow cytometry. In certain embodiments, the ability of the engineered cells to destroy target cells can be measured using any suitable method known in the art, such as cytotoxicity assays described in, for example, Kochenderfer et al., J. Immunotherapy, 32(7): 689-702 (2009), and Herman et al. J. Immunological Methods, 285(1): 25-40 (2004). In certain embodiments, the biological activity of the cells also can be measured by assaying expression and/or secretion of certain cytokines, such as CD 107a, IFNγ, IL-2, and TNF. In some aspects the biological activity is measured by assessing clinical outcome, such as reduction in tumor burden or load.

In certain embodiments, engineered cells are modified in any number of ways, such that their therapeutic or prophylactic efficacy is increased. For example, the engineered CAR or TCR expressed by the population can be conjugated either directly or indirectly through a linker to a targeting moiety. The practice of conjugating compounds, e.g., the CAR or TCR, to targeting moieties is known in the art. See, for instance, Wadwa et al., J. Drug Targeting 3: 1 1 1 (1995), and U.S. Patent 5,087,616.

### VI. Definitions

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known to the skilled person in this technical field. Reference to "about" a value or parameter herein includes (and describes) embodiments that are directed to that value or parameter *per se.* For example, description referring to "about X" includes description of "X".

As used herein, "isolated" or "purified with reference to a peptide, protein or polypeptide refers to a molecule which is substantially free of all other polypeptides, contaminants, starting reagents or other materials, or substantially free from chemical precursors or other chemicals when chemically synthesized. Preparations can be determined to be substantially free if they appear free of readily detectable impurities as determined by standard methods of analysis, such as high-performance liquid chromatography (HPLC), thin-layer chromatography (TLC) or capillary electrophoresis (CE), used by those of skill in the art to assess such purity, or sufficiently pure such that further purification would not detectably alter the physical and chemical properties of the substance.

As used herein, the term "recombinant" refers to a cell, microorganism, nucleic acid molecule, or vector that has been modified by introduction of an exogenous, such as heterologous, nucleic acid molecule, or refers to a cell or microorganism that has been altered such that expression of an endogenous nucleic acid molecule or gene is controlled, deregulated or constitutive, where such alterations or modifications may be introduced by genetic engineering. Genetic alterations may include, for example, modifications introducing nucleic acid molecules (which may include an expression control element, such as a promoter) encoding one or more proteins or enzymes, or other nucleic acid molecule additions, deletions, substitutions, or other functional disruption of or addition to a cell's genetic material. Exemplary modifications include those in coding regions or functional fragments thereof of heterologous or homologous polypeptides from a reference or parent molecule.

As used herein, a composition refers to any mixture of two or more products, substances, or compounds, including cells. It may be a solution, a suspension, liquid, powder, a paste, aqueous, non-aqueous or any combination thereof.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

The term "construct" refers to any polynucleotide that contains a recombinant nucleic acid molecule. A construct may be present in a vector (*e.g.,* a bacterial vector, a viral vector) or may be integrated into a genome.

The term "vector," as used herein, refers to a nucleic acid molecule capable of propagating another nucleic acid to which it is linked. Vectors may be, for example, plasmids, cosmids, viruses, a RNA vector or a linear or circular DNA or RNA molecule that may include chromosomal, non-chromosomal, semi-synthetic or synthetic nucleic acid molecules. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Among the vectors are viral vectors, such as CMV vectors, including those having a genome carrying another nucleic acid and capable of inserting into a host genome for propagation thereof.

As used herein "operably linked" refers to the association of components, such as a coding sequence (e.g. a heterologous nucleic acid) and a nucleic acid control sequence (e.g. regulatory sequence(s)), in such a way as to permit gene expression when they are covalently linked in such a way as to place the expression or transcription and/or translation of the coding sequence under the influence or control of the nucleic acid control sequence. Hence, it means that the components described are in a relationship permitting them to function in their intended manner.

The term "expression", as used herein, refers to the process by which a polypeptide is produced based on the encoding sequence of a nucleic acid molecule, such as a gene. The process may include transcription, post-transcriptional control, post-transcriptional modification, translation, post-translational control, post-translational modification, or any combination thereof.

The term "introduced" in the context of inserting a nucleic acid molecule into a cell, means "transfection", or 'transformation" or "transduction" and includes reference to the incorporation of a nucleic acid molecule into a eukaryotic or prokaryotic cell wherein the nucleic acid molecule may be incorporated into the genome of a cell (e.g., chromosome, plasmid, plastid, or mitochondrial DNA), converted into an autonomous replicon, or transiently expressed (e.g., transfected mRNA).

As used herein, a "subject" is a mammal, such as a human or other animal, and typically is human.

As used herein, a control refers to a sample that is substantially identical to the test sample, except that it is not treated with a test parameter, or, if it is a plasma sample, it can be from a normal volunteer not affected with the condition of interest. A control also can be an internal control.

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

All publications, including patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### VII. Exemplary Embodiments

Among the embodiments provided herein are:
1. A method of identifying a peptide epitope, comprising:
   a) contacting an MHC-E molecule with one or more peptides; and
   b) detecting or identifying peptide(s) in the context of the MHC-E molecule.
2. The method of embodiment 1, wherein the one or more peptides comprise peptides of one or more protein antigens.
3. The method of embodiment 2, wherein the one or more peptides in the context of the MHC-E molecule are identified as peptide epitopes of the one or more protein antigens.
4. The method of embodiment 2 or embodiment 3, wherein the antigen is a tumor antigen.
5. The method of embodiments 2 or embodiment 3, wherein the antigen is a pathogenic antigen.
6. The method of embodiment 5, wherein the pathogenic antigen is a bacterial antigen or viral antigen.
7. The method of any of embodiments 1-6, wherein the MHC-E molecule is expressed on the surface of a cell.
8. The method of any of embodiments 1-7, further comprising identifying a peptide binding molecule or antigen-binding fragment thereof that binds to at least one of the one or more peptides in the context of the MHC-E molecule.
9. A method of identifying a peptide binding molecule that binds to one or more peptides in the context of an MHC-E molecule, comprising:
   a) providing a cell comprising one or more peptides in the context of an MHC-E molecule on the surface of the cell; and
   b) identifying a peptide binding molecule or antigen-binding fragment thereof that binds to at least one of the one or more peptides in the context of the MHC-E molecule.
10. The method of embodiment 9, wherein providing a cell comprising one or more peptides in the context of an MHC-E molecule comprises contacting the MHC-E molecule on the surface of the cell with the one or more peptides.
11. The method of embodiment 10, comprising detecting or identifying if the peptide(s) in the context of an MHC-E molecule is present or formed on the surface of the cell prior to providing the cell in a).
12. The method of any of embodiments 9-11, wherein the one or more peptides comprise peptides of a protein antigen.
13. The method of any of embodiments 9-12, wherein the antigen is a tumor antigen or a pathogenic antigen.
14. The method of embodiment 13, wherein the pathogenic antigen is a bacterial antigen or viral antigen.
15. The method of embodiment 6 or embodiment 14, wherein the antigen is a viral antigen and the viral antigen is from hepatitis A, hepatitis B, hepatitis C virus (HCV), human papilloma virus (HPV), hepatitis viral infections, Epstein-Barr virus (EBV), human herpes virus 8 (HHV-8), human T-cell leukemia virus-1 (HTLV-1), human T-cell leukemia virus-2 (HTLV-2), or a cytomegalovirus (CMV).
16. The method of embodiment 15, wherein the antigen is an HPV antigen selected from among HPV-16, HPV-18, HPV-31, HPV-33 and HPV-35.
17. The method of embodiment 15, wherein the viral antigen is an EBV antigen selected from among Epstein-Barr nuclear antigen (EBNA)-1, EBNA-2, EBNA-3A, EBNA-3B, EBNA-3C, EBNA-leader protein (EBNA-LP), latent membrane proteins LMP-1, LMP-2A and LMP-2B, EBV-EA, EBV-MA and EBV-VCA.
18. The method of embodiment 15, wherein the viral antigen is an HTLV-antigen that is TAX.
19. The method of embodiment 15, wherein the viral antigen is an HBV antigen that is a hepatitis B core antigen or a hepatitis B envelope antigen.
20. The method of any of embodiments 9-13, wherein the antigen is a tumor antigen.
21. The method of any of embodiments 4, 7-8 and 20, wherein the tumor antigen is selected from among glioma-associated antigen, β-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, Melanin-A/MART-1, WT-1, S-100, MBP, CD63, MUC1 (e.g. MUC1-8), p53, Ras, cyclin B1, HER-2/neu, carcinoembryonic antigen (CEA), gp100, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A11, MAGE-B1, MAGE-B2, MAGE-B3, MAGE-B4, MAGE-C1, BAGE, GAGE-1, GAGE-2, pl5, tyrosinase (e.g. tyrosinase-related protein 1 (TRP-1) or tyrosinase-related protein 2 (TRP-2)), β-catenin, NY-ESO-1, LAGE-1a, PP1, MDM2, MDM4, EGVFvIII, Tax, SSX2, telomerase, TARP, pp65, CDK4, vimentin, S100, eIF-4A1, IFN-inducible p78, melanotransferrin (p97), Uroplakin II, prostate specific antigen (PSA), human kallikrein (huK2), prostate specific membrane antigen (PSM), and prostatic acid phosphatase (PAP), neutrophil elastase, ephrin B2, BA-46, Bcr-abl, E2A-PRL, H4-RET, IGH-IGK, MYL-RAR, Caspase 8, FRa, CD24, CD44, CD133, CD 166, epCAM, CA-125, HE4, Oval, estrogen receptor, progesterone receptor, uPA, PAI-1, CD19, CD20, CD22, ROR1, CD33/IL3Ra, c-Met, PSMA, Glycolipid F77, GD-2, insulin growth factor (IGF)-I, IGF-II, IGF-I receptor and mesothelin.
22. The method of any of embodiments 1-8 and 11-21, wherein detecting or identifying the one or more peptide(s) in the context of an MHC-E molecule comprises extracting peptides from a lysate of the cell, eluting peptides from the cell surface or isolating the MHC-E molecule or molecules and eluting the one or more peptides from the MHC-E molecule.
23. The method of any of embodiments 1-8 and 11-22, wherein the one or more peptides comprises one or more peptides having a length of from or from about 8 to 20 amino acids or 9 to 15 amino acids.
24. The method of any of embodiments 1-8 and 11-23, wherein the one or more peptides comprises peptides having a length of or about 9 amino acids, about 10 amino acids, about 11 amino acids, about 12 amino acids, about 13 amino acids, about 14 amino acids or about 15 amino acids.
25. The method of any of embodiments 1-8 and 11-24, wherein the one or more peptides comprises overlapping peptides of the antigen or a region of the antigen.
26. The method of embodiment 25, wherein the overlapping peptides, collectively, comprise peptides that represent at least 30%, at least 40%, at least 50%, at least 60%, at least 70%, at least 80%, at least 90% or more of a contiguous sequence of amino acids of the antigen.
27. The method of any of embodiments 1-8 and 11-24, wherein peptides comprised by the one or more peptides are present in orthogonal pools, each of said orthogonal pools comprising at least two or more different peptides, wherein at least one peptide in each orthogonal pool is the same as a peptide present in at least another orthogonal pool.
28. The method of embodiment 27, wherein a peptide in the context of the MHC-E molecule is detected or identified if the peptide is eluted or extracted from the MHC-E molecule or from a cell expressing the MHC-E molecule in at least two orthogonal pools comprising the same peptide.
29. The method of any of embodiments 7-24, wherein the cell comprises one or a combination of antigens heterologous or exogenous to the cell.
30. The method of embodiment 29, wherein the one or combination of antigens present in the cell is processed to one or more peptides, thereby contacting the MHC-E molecule with one or more peptides.
31. The method of embodiment 29 or embodiment 30, wherein the cell comprises one or a combination of synthetic nucleic acid molecules comprising one or more coding sequences encoding the one or combination of antigens.
32. A method of identifying a peptide epitope, comprising:
   a) introducing a synthetic nucleic acid molecule or a combination of synthetic nucleic acid molecules comprising one or more coding sequences encoding an antigen or a combination of antigens into a cell expressing an MHC-E molecule;
   b) incubating the cell under conditions whereby the encoded antigen or antigens are processed to peptides; and
   c) detecting or identifying peptide(s) of the antigen in the context of the MHC-E molecule.
33. The method of embodiment 32, further comprising identifying a peptide binding molecule or antigen-binding fragment thereof that binds to at least one of the one or more peptides of the antigen in the context of the MHC-E molecule.
34. A method of identifying a peptide binding molecule that binds a peptide in the context of an MHC-E molecule, comprising:
   a) introducing a synthetic nucleic acid molecule or a combination of synthetic nucleic acid molecules comprising one or more coding sequences encoding an antigen or combination of antigens into a cell expressing an MHC-E molecule;
   b) incubating the cell under conditions whereby the encoded antigen or combination of antigens is processed to peptides; and
   c) identifying a peptide binding molecule or antigen-binding fragment thereof that binds to at least one of the one or more peptides of the antigen in the context of the MHC-E molecule.
35. The method of any of embodiments 32-34, wherein the antigen is a protein antigen.
36. The method of embodiment 35, wherein the one or more peptides in the context of the MHC-E molecule are identified as peptide epitopes of the one or more protein antigen.
37. The method of embodiment 35 or embodiment 36, wherein the antigen is a tumor antigen or a pathogenic antigen.
38. The method of any of embodiments 31-37, wherein the synthetic nucleic acid is synthetic DNA.
39. The method of embodiment 38, wherein the synthetic DNA is complementary DNA (cDNA).
40. The method of any of embodiments 31-39, wherein the cell comprises a synthetic nucleic acid comprising a coding sequence encoding the antigen.
41. The method of any of embodiments 31-40, wherein the cell comprises a combination of synthetic nucleic acids each individually comprising a coding sequence of one of the combination of antigens.
42. The method of embodiment 41, wherein the combination of synthetic nucleic acids comprises one or more nucleic acid molecules of a cDNA library.
43. The method of embodiment 42, wherein the cDNA library is a tumor-derived cDNA library.
44. The method of embodiment 43, wherein the tumor is a melanoma, sarcoma, breast carcinoma, renal carcinoma, lung carcinoma, ovarian carcinoma, prostate carcinoma, colorectal carcinoma, pancreatic carcinoma, squamous tumor of the head and neck, or squamous carcinoma of the lung.
45. The method of any of embodiments 31-44, wherein the combination of synthetic nucleic acid molecules and/or combination of encoded antigens is present in orthogonal pools, each of said orthogonal pools comprising at least two or more different synthetic nucleic acid molecules and/or encoded antigens, wherein at least one synthetic nucleic acid molecule and/or encoded antigen is the same in at least two orthogonal pools.
46. The method of embodiment 45, wherein a peptide in the context of an MHC-E molecule is detected or identified if the peptide is eluted or extracted from an MHC-E molecule or from a cell expressing an MHC-E molecule in at least two orthogonal pools comprising the same peptide.
47. The method of any of embodiments 1-46, that is performed in an array.
48. The method of embodiment 47, wherein the array is an addressable or spatial array.
49. The method of any of embodiments 1-48, wherein the MHC-E molecule is an HLAE^{∗}01:01 or HLA E^{∗}0103.
50. The method of any of embodiments 7-49, wherein the cell is a primary cell or is a cell line.
51. The method of any of embodiments 7-50, wherein the cell is a human cell.
52. The method of any of embodiments 7-51, wherein the cell is selected from among a fibroblast, a B cell, a dendritic cell and a macrophage.
53. The method of any of embodiments 7-52, wherein the cell is a cell line and the cell line is or is derived from a cell selected from among K562, C1R, KerTr, HCT-15, DLD-1, Daudi, 221, 721.221, BLS-1, BLS-2, JEG-3 and JAR.
54. The method of embodiment 53, wherein the cell is or is derived from a 221 or K562 cell line.
55. The method of any of embodiments 7-54, wherein the cell is an artificial antigen presenting cell.
56. The method of embodiment 55, wherein the artificial antigen presenting cell expresses the MHC-E molecule and one or more of a stimulatory or costimulatory molecule(s), an Fc receptor, an adhesion molecule(s) or a cytokine.
57. The method of any of embodiments 7-56, wherein the cell is genetically or recombinantly engineered to express the MHC-E molecule.
58. The method of any of embodiments 7-32, and 47-57, wherein:
   (1) the cell has been or is incubated with an activating or stimulating agent prior to or simultaneously with contacting the MHC-E molecule with the one or more peptides; or
   (2) the method further comprises incubating the cell with an activating or stimulating agent prior to or simultaneously with contacting the MHC-E molecule with the one or more peptides.
59. The method of any of embodiments 33-57, wherein:
   (1) the cell has been or is incubated with an activating or stimulating agent prior to or simultaneously with introducing the synthetic nucleic acid molecule or combination of synthetic nucleic acid molecules into the cell; or
   (2) the method further comprises incubating the cell with an activating or stimulating agent prior to or simultaneously with introducing the synthetic nucleic acid molecule or combination of synthetic nucleic acid molecules into the cell.
60. The method of embodiment 58 or embodiment 58, wherein the incubating with the activating or stimulating agent increases expression of the MHC-E molecule on the surface of the cell compared to expression of the MHC-E molecule in the absence of said activating or stimulating.
61. The method of embodiment 60, wherein expression of the MHC-E molecule is increased at least 1.2-fold, 1.5-fold, 2-fold, 3-fold, 4-fold, 5-fold, 6-fold, 7-fold, 8-fold, 9-fold or 10-fold.
62. The method of embodiment 60 or embodiment 61, wherein the activating or stimulating is effected in the presence of interferon gamma.
63. The method of embodiment 60 or embodiment 61, wherein the activating or stimulating comprises incubating the cell with a virus or viral particle.
64. The method of embodiment 63, wherein the virus or viral particle is a cytomegalovirus (CMV).
65. The method of any of embodiments 7-64, wherein the cell is repressed and/or disrupted in a gene encoding a classical MHC class I molecule and/or does not express a classical MHC class I molecule.
66. The method of embodiment 65, wherein the repression is effected by an inhibitory nucleic acid molecule.
67. The method of embodiment 66, wherein the inhibitory nucleic acid molecule comprises an RNA interfering agent.
68. The method of embodiment 66 or embodiment 67, wherein the inhibitory nucleic acid is or comprises or encodes a small interfering RNA (siRNA), a microRNA-adapted shRNA, a short hairpin RNA (shRNA), a hairpin siRNA, a microRNA (miRNA-precursor) or a microRNA (miRNA).
69. The method of embodiment 68, wherein disruption of the gene is mediated by a gene editing nuclease, a zinc finger nuclease (ZFN), a clustered regularly interspaced short palindromic nucleic acid (CRISPR) /Cas9, and/or a TAL-effector nuclease (TALEN).
70. The method of any of embodiments 65-69, wherein expression of the classical MHC class I molecule in the cell is reduced by at least 50%, 60%, 70%, 80%, 90%, or 95% as compared to the expression in the cell in the absence of the repression or gene disruption.
71. The method of any of embodiments 65-70, wherein the classical MHC class I molecule is an HLA-A, HLA-B or HLA-C molecule.
72. The method of any of embodiments 32 and 35-71, wherein detecting or identifying a peptide in the context of an MHC-E molecule comprises extracting peptides from a lysate of the cell, eluting peptides from the cell surface or isolating the MHC-E molecule or molecules and eluting the one or more peptides from the MHC-E molecule.
73. The method of embodiment 22 or embodiment 72, wherein isolating the MHC-E molecule or molecules comprising solubilizing the cell and selecting the MHC-E molecule by immunoprecipitation or immunoaffinity chromatography.
74. The method of embodiment 22, embodiment 28 or embodiment 72, wherein eluting peptides from an MHC-E molecule is effected in the presence of a mild acid or a diluted acid.
75. The method of any of embodiments 1-8 and 11-74, comprising fractionating, separating or purifying the identified or detected peptide(s).
76. The method of any of embodiments 1-8 and 11-75, comprising sequencing the identified or detected peptide(s).
77. The method of any of embodiments 1-8 and 11-76, further comprising determining if the identified or detectedpeptide(s) elicit an antigen-specific immune response.
78. The method of embodiment 77, wherein the antigen-specific immune response is a humoral T cell response.
79. The method of embodiment 77, wherein the antigen-specific immune response is a cytotoxic T lymphocyte response.
80. The method of any of embodiments7-79, wherein the cell is a test cell and the method further comprises:
   detecting or identifying peptides in the context of an MHC-E molecule on the surface of a control cell, said control cell having not been contacted with the one or more peptides; and
   identifying peptide(s) in the context of an MHC-E molecule that is unique to the test cell compared to the control cell, thereby identifying the one or more peptides of the antigen in the context of an MHC-E molecule.
81. The method of any of embodiments 1-80 that is performed in vitro.
82. The method of any of embodiments 1-81, wherein:
   the peptide(s) identified in the context of the MHC-E molecule comprise a length of from or from about 8 to 13 amino acids; or
   the peptide(s) identified in the context of the MHC-E molecule comprise a length of or about 8 amino acids, 9 amino acids, 10 amino acids or 11 amino acids.
83. The method of any of embodiments 1-82, wherein the peptide(s) in the context of the MHC-E molecule have a binding affinity with an IC50 for the MHC-E molecule of greater than 200 nM, 300 nM, 400 nM, 500 nM, 600 nM, 700 nM, 800 nM, 900 nM, or 1000.
84. The method of any of embodiments 1-83, wherein the peptide(s) in the context of the MHC-E molecule have a binding affinity with an IC50 for the MHC-E molecule of less than 500 nm, 400 nM, 300 nM, 200 nM, 100 nM, or 50 nM.
85. The method of any of embodiments 1-84, wherein the peptide(s) in the context of the MHC-E molecule are capable of inducing a CD8+ immune response in a subject.
86. The method of embodiment 85, wherein the peptide(s) in the context of the MHC-E molecule are capable of generating a universal immune response in a majority of subjects in a population.
87. The method of embodiment 86, wherein the universal immune response is elicited in greater than 50%, 60%, 70%, 80%, or 90% of subjects in a population.
88. The method of any of embodiments 85-87, wherein the subjects are human subjects.
89. A peptide epitope identified by the methods of any of embodiments 1-8 and 15-88.
90. A stable MHC-E-peptide complex, comprising the peptide epitope of embodiment 89.
91. The stable MHC-E-peptide complex of embodiment 90 that is present on a cell surface.
92. The method of any of embodiments 8-21 and 33-88, wherein identifying the peptide binding molecule or antigen-binding fragment thereof comprises:
   a) assessing binding of a plurality of candidate peptide binding molecules or antigen-binding fragments thereof to the surface of the cell; and
   b) identifying from among the plurality one or more peptide binding molecules that bind to the at least one of the one or more peptides in the context of an MHC-E molecule.
93. A method of identifying a peptide binding molecule or antigen-binding fragment thereof that binds a peptide in the context of an MHC-E molecule, comprising:
   a) assessing binding of a plurality of candidate peptide binding molecules or antigen-binding fragments thereof to the stable MHC-E-peptide complex of embodiment 89 or embodiment 90; and
   b) identifying from among the plurality one or more peptide binding molecules that bind to the peptide in the context of an MHC-E molecule.
94. A method of identifying a peptide binding molecule or antigen-binding fragment thereof that binds an MHC-E-restricted peptide, comprising:
   a) identifying a peptide by the method of any of embodiments 1-8 and 15-88;
   b) assessing binding of a plurality of candidate peptide binding molecules or antigen-binding fragments thereof to an MHC-E molecule comprising the peptide of a) bound thereto; and
   c) identifying from among the plurality one or more peptide binding molecules that bind to the peptide in the context of the MHC-E molecule.
95. The method of any of embodiments 4-31 and 33-94, wherein the plurality of candidate peptide binding molecules comprises one or more T cell receptors (TCRs), antigen-binding fragments of a TCR, antibodies or antigen-binding fragments thereof.
96. The method of any of embodiments 92-95, wherein the plurality of candidate peptide binding molecules comprises at least 2, 5, 10, 100, 10³, 10⁴, 10⁵, 10⁶, 10⁷, 10⁸, 10⁹, or more different molecules.
97. The method of any of embodiments 92-96, wherein the plurality of candidate peptide binding molecules comprise peptide binding molecules comprising one or more mutations compared to a parent or scaffold peptide binding molecule, wherein individual candidate peptide binding molecules comprise one or more different mutations compared to other candidate peptide binding molecules in the plurality.
98. The method of embodiment 97, wherein the one or more amino acid mutations comprise a mutation or mutations in a complementarity determining region (CDR) or CDRs of the molecule.
99. The method of any of embodiments 92-98, wherein the candidate peptide binding molecules are obtained from a sample from a subject or a population of subjects.
100. The method of embodiment 99, wherein the subject or population of subjects comprises normal or healthy subjects or diseased subjects.
101. The method of embodiment 100, wherein the diseased subjects are tumor-bearing subjects.
102. The method of any of embodiments 99-101, wherein the subject has been vaccinated with the peptide epitope of the antigen.
103. The method of any of embodiments 99-102, wherein the subject is a human or rodent.
104. The method of embodiments 99-103, wherein the subject is an HLA-transgenic mouse and/or is a human TCR transgenic mouse.
105. The method of any of embodiments 99-104, wherein the sample comprises T cells.
106. The method of embodiment 105, wherein the sample comprises peripheral blood mononuclear cells (PBMCs) or tumor-infiltrating lymphocytes (TIL).
107. The method of any of embodiments 95-106, wherein the candidate peptide binding molecule comprises a T cell receptor (TCR) or an antigen-binding fragment of a TCR.
108. The method of any of embodiments 95-107, wherein the antigen-binding fragment of a TCR is a single chain TCR (scTCR).
109. The method of any of embodiments 99-103, wherein the sample comprises B cells.
110. The method of embodiment 109, wherein the sample is selected from among blood, bone marrow and spleen and/or the sample comprises PBMCs, splenocytes or bone marrow cells.
111. The method of any of embodiments 95-103, 109 and 110, wherein the candidate peptide binding molecules comprise antibodies or antigen-binding fragments thereof.
112. The method of embodiment 111, wherein the candidate peptide binding molecules comprise IgM-derived antibodies or antigen-binding fragments and/or are naive.
113. The method of embodiment 111 or embodiment 112, wherein the antibodies or antigen-binding fragments thereof are produced by immunizing a host with an immunogen comprising the MHC-peptide complex.
114. The method of any of embodiments 111-113, wherein the candidate peptide binding molecule is a single chain variable fragment (scFv).
115. The method of any of embodiments 92-114, wherein the candidate peptide binding molecules are present in a display library.
116. The method of embodiment 115, wherein the display library is selected from among a cell surface display library, a phage display library, a ribosome display library, an mRNA display library, and a dsDNA display library.
117. The method of any of embodiments 8-21 and 33-116, wherein:
   the identified peptide binding molecule exhibits binding affinity for the peptide in the context of an MHC-E molecule with a dissociation constant (K_{D}) of from or from about 10⁻⁵ M to 10⁻¹³ M, 10⁻⁵ M to 10⁻⁹ or 10⁻⁷ M to 10⁻¹²; or
   the identified peptide binding molecule exhibits binding affinity for the peptide in the context of an MHC-E molecule with a K_{D} of less than or less than about 10⁻⁵ M, 10⁻⁶ M, 10⁻⁷ M, 10⁻⁸ M, 10⁻⁹ M, 10⁻¹⁰ M, 10⁻¹¹ M or less.
118. A peptide binding molecule identified by the method of any of embodiments 8-21 and 33-117.
119. The peptide binding molecule of embodiment 118 that is a TCR or antigen-binding fragment thereof.
120. The peptide binding molecule of embodiment 118 that is an antibody or antigen-binding fragment thereof.
121. A recombinant antigen receptor, comprising the peptide binding molecule of any of embodiments 118-120.
122. The recombinant antigen receptor of embodiment 121 that is a chimeric antigen receptor (CAR).
123. A genetically engineered cell, expressing the peptide binding molecule of any of embodiments 118-120 or a recombinant receptor of embodiment 121 or embodiment 122.
124. The genetically engineered cell of embodiment 123 that is a T cell.
125. The genetically engineered cell of embodiment 124 that is a CD8+ T cell.
126. A CD8+ genetically engineered cell, expressing a peptide binding molecule or a recombinant receptor comprising a peptide binding molecule, wherein the peptide binding molecule specifically binds a peptide epitope in the context of an MHC-E molecule.
127. The CD8+ genetically engineered cell of embodiment 126, wherein the peptide binding molecule is a T cell receptor (TCR), an antigen-binding fragment of a TCR, an antibody or an antigen-binding fragment of an antibody.
128. The CD8+ genetically engineered cell of embodiment 126 or embodiment 127, wherein the recombinant antigen receptor is a chimeric antigen receptor (CAR).
129. A composition, comprising a peptide binding molecule of any of embodiments 118-120, a recombinant receptor of embodiment 121 or embodiment 122 or a genetically engineered cell of any of embodiments 123-128.
130. The composition of embodiment 129, further comprising a pharmaceutically acceptable excipient.
131. A method of treating a disease or condition, comprising administering to a subject a composition of embodiment 129 or embodiment 130.
132. The method of embodiment 131, wherein the peptide binding molecule or recombinant receptor binds to an antigen associated with the disease or condition.
133. The method of embodiment 131 or embodiment 132, wherein the disease or condition is a tumor or a cancer.
134. A pharmaceutical composition of embodiment 129 or embodiment 130 for use in treating a disease or condition.
135. The pharmaceutical composition for use of embodiment 134, wherein the peptide binding molecule or recombinant receptor binds to an antigen associated with the disease or condition.
136. The pharmaceutical composition for use of embodiment 134 or embodiment 135, wherein the disease or condition is a tumor or a cancer.

### SEQUENCE TABLE

| **SEQ ID NO.** | | **Sequence** |
|---|---|---|
| 1 | Protein | |
| | Major histocompatibility complex, class I, E, E*0101 | |
| | Homo sapiens | |
| | GenBank No. AAH02578.1 or UniProt No. P13747.3 | |
| 2 | DNA | CAGAGGCTGGGATCATGGTAGATGGAACCCTCCTTTACTCCTCTCGGAGGCCCTG GCCCTTACCCAGACCTGGGCGGGCTCCCACTCCTTGAAGTATTTCCACACTTCCGT GTCCCGGCCCGGCCGCGGGGAGCCCCGCTTCATCTCTGTGGGCTACGTGGACGAC ACCCAGTTCGTGCGCTTCGACAACGACGCCGCGAGTCCGAGGATGGTGCCGCGGG CGCCGTGGATGGAGCAGGAGGGGTCAGAGTATTGGGACCGGGAGACACGGAGCG CCAGGGACACCGCACAGATTTTCCGAGTGAACCTGCGGACGCTGCGCGGCTACTA CAATCAGAGCGAGGCCGGGTCTCACACCCTGCAGTGGATGCATGGCTGCGAGCTG GGGCCCGACAGGCGCTTCCTCCGCGGGTATGAACAGTTCGCCTACGACGGCAAGG ATTATCTCACCCTGAATGAGGACCTGCGCTCCTGGACCGCGGTGGACACGGCGGC TCAGATCTCCGAGCAAAAGTCAAATGATGCCTCTGAGGCGGAGCACCAGAGAGCC TACCTGGAAGACACATGCGTGGAGTGGCTCCACAAATACCTGGAGAAGGGGAAG GAGACGCTGCTTCACCTGGAGCCCC CAAAGACACACGTGACTCACCACCCCATCTCTGACCATGAGGCCACCCTGAGGTG CTGGGCCCTGGGCTTCTACCCTGCGGAGATCACACTGACCTGGCAGCAGGATGGG GAGGGCCATACCCAGGACACGGAGCTCGTGGAGACCAGGCCTGCAGGGGATGGA ACCTTCCAGAAGTGGGCAGCTGTGGTGGTGCCTTCTGGAGAGGAGCAGAGATACA CGTGCCATGTGCAGCATGAGGGGCTACCCGAGCCCGTCACCCTGAGATGGAAGCC GGCTTCCCAGCCCACCATCCCCATCGTGGGCATCATTGCTGGCCTGGTTCTCCTTG GATCTGTGGTCTCTGGAGCTGTGGTTGCTGCTGTGATATGGAGGAAGAAGAGCTC AGGTGGAAAAGGAGGGAGCTACTCTAAGGCTGAGTGGAGCGACAGTGCCCAGGG GTCTGAGTCTCACAGCTTGTAAAGCCTGAGACAGCTGCCTTGTGTGCGACTGAGAT GCACAGCTGCCTTGTGTGCGACTGAGATGCAGGATTTCCTCACGCCTCCCCTATGT GTCTTAGGGGACTCTGGCTTCTCTTTTTGCAAGGGCCTCTGAATCTGTCTGTGTCCC TGTTAGCACAATGTGAGGAGGT AGAGAAACAGTCCACCTCTGTGTCTACCATGACCCCCTTCCTCACACTGACCTGTG TTCCTTCCCTGTTCTCTTTTCTATTAAAAATAAGAACCTGGGCAGAGTGCGGCAGC TCATGCCTGTAATCCCAGCACTTAGGGAGGCCGAGGAGGGCAGATCACGAGGTCA GGAGATCGAAACCATCCTGGCTAACACGGTGAAACCCCGTCTCTACTAAAAATA CAAAAAATTAGCTGGGCGCAGAGGCACGGGCCTGTAGTCCCAGCTACTCAGGAGG CGGAGGCAGGAGAATGGCGTCAACCCGGGAGGCGGAGGTTGCAGTGAGCCAGGA TTGTGCGACTGCACTCCAGCCTGGGTGACAGGGTGAAACGCCATCTCAAAAAATA |
| | Major histocompatibility complex, class I, E | |
| | Homo sapiens | |
| | GenBank No. BC002578 | |
| | Coding DNA sequence (CDS) 15-1091 | |
| | | AAAATTAAAAAAAAAAAAAAAA |
| 3 | Protein | MVDGTLLLLLSEALALTQTWAGSHSLKYFHTSVSRPGRGEPRFISVGYVDDTQFVRFD NDAASPRMVPRAPWMEQEGSEYWDRETRSARDTAQIFRVNLRTLRGYYNQSEAGSH TLQWMHGCELGPDGRFLRGYEQFAYDGKDYLTLNEDLRSWTAVDTAAQISEQKSND ASEAEHQRAYLEDTCVEWLHKYLEKGKETLLHLEPPKTHVTHHPISDHEATLRCWAL GFYPAEITLTWQQDGEGHTQDTELVETRPAGDGTFQKWAAVVVPSGEEQRYTCHVQ HEGLPEPVTLRWKPASQPTIPIVGIIAGLVLLGSVVSGAVVAAVIWRKKSSGGKGGSYS KAEWSDSAQGSESHSL |
| | Major histocompatibility complex, class I, E, E*0103 | |
| | Homo sapiens | |
| | GenBank No. NP_005507 | |
| 4 | DNA | GCAGACTCAGTTCTCATTCCCAATGGGTGTCGGGTTTCTAGAGAAGCCAATCAGCG TCGCCACGACTCCCGACTATAAAGTCCCCATCCGGACTCAAGAAGTTCTCAGGACT CAGAGGCTGGGATCATGGTAGATGGAACCCTCCTTTTACTCCTCTCGGAGGCCCTG GCCCTTACCCAGACCTGGGCGGGCTCCCACTCCTTGAAGTATTTCCACACTTCCGT GTCCCGGCCCGGCCGCGGGGAGCCCCGCTTCATCTCTGTGGGCTACGTGGACGAC ACCCAGTTCGTGCGCTTCGACAACGACGCCGCGAGTCCGAGGATGGTGCCGCGGG CGCCGTGGATGGAGCAGGAGGGGTCAGAGTATTGGGACCGGGAGACACGGAGCG CCAGGGACACCGCACAGATTTTCCGAGTGAATCTGCGGACGCTGCGCGGCTACTA CAATCAGAGCGAGGCCGGGTCTCACACCCTGCAGTGGATGCATGGCTGCGAGCTG GGGCCCGACGGGCGCTTCCTCCGCGGGTATGAACAGTTCGCCTACGACGGCAAGG ATTATCTCACCCTGAATGAGGACCTGCGCTCCTGGACCGCGGTGGACACGGCGGC TCAGATCTCCGAGCAAAAGTCA AATGATGCCTCTGAGGCGGAGCACCAGAGAGCCTACCTGGAAGACACATGCGTGG AGTGGCTCCACAAATACCTGGAGAAGGGGAAGGAGACGCTGCTTCACCTGGAGCC CCCAAAGACACACGTGACTCACCACCCCATCTCTGACCATGAGGCCACCCTGAGG TGCTGGGCCCTGGGCTTCTACCCTGCGGAGATCACACTGACCTGGCAGCAGGATG GGGAGGGCCATACCCAGGACACGGAGCTCGTGGAGACCAGGCCTGCAGGGGATG GAACCTTCCAGAAGTGGGCAGCTGTGGTGGTGCCTTCTGGAGAGGAGCAGAGATA CACGTGCCATGTGCAGCATGAGGGGCTACCCGAGCCCGTCACCCTGAGATGGAAG CCGGCTTCCCAGCCCACCATCCCCATCGTGGGCATCATTGCTGGCCTGGTTCTCCT TGGATCTGTGGTCTCTGGAGCTGTGGTTGCTGCTGTGATATGGAGGAAGAAGAGC TCAGGTGGAAAAGGAGGGAGCTACTCTAAGGCTGAGTGGAGCGACAGTGCCCAG GGGTCTGAGTCTCACAGCTTGTAAAGCCTGAGACAGCTGCCTTGTGTGCGACTGA GATGCACAGCTGCCTTGTGTGCGACT GAGATGCAGGATTTCCTCACGCCTCCCCTATGTGTCTTAGGGGACTCTGGCTTCTC TTTTTGCAAGGGCCTCTGAATCTGTCTGTGTCCCTGTTAGCACAATGTGAGGAGGT AGAGAAACAGTCCACCTCTGTGTCTACCATGACCCCCTTCCTCACACTGACCTGTG TTCCTTCCCTGTTCTCTTTTCTATTAAAAATAAGAACCTGGGCAGAGTGCGGCAGC TCATGCCTGTAATCCCAGCACTTAGGGAGGCCGAGGAGGGCAGATCACGAGGTCA GGAGATCGAAACCATCCTGGCTAACACGGTGAAACCCCGTCTCTACTAAAAAATA CAAAAAATTAGCTGGGCGCAGAGGCACGGGCCTGTAGTCCCAGCTACTCAGGAGG CGGAGGCAGGAGAATGGCGTCAACCCGGGAGGCGGAGGTTGCAGTGAGCCAGGA TTGTGCGACTGCACTCCAGCCTGGGTGACAGGGTGAAACGCCATCTCAAAAAATA AAAATTGAAAAATAAAAAAAGAACCTGGATCTCAATTTAATTTTTCATATTCTTGC AATGAAATGGACTTGAGGAAGCTAAGATCATAGCTAGAAATACAGATAATTCCAC AGCACATCTCTAGCAAATTTA GCCTATTCCTATTCTCTAGCCTATTCCTTACCACCTGTAATCTTGACCATATACCTT |
| | Major histocompatibility complex, class I, E, E*0103 | |
| | Homo sapiens | |
| | GenBank No. NM_005516.5 | |
| | CDS 127-1203 | |
| | | GGAGTTGAATATTGTTTTCATACTGCTGTGGTTTGAATGTTCCCTCCAACACTCATG TTGAGACTTAATCCCTAATGTGGCAATACTGAAAGGTGGGGCCTTTGAGATGTGAT TGGATCGTAAGGCTGTGCCTTCATTCATGGGTTAATGGATTAATGGGTTATCACAG GAATGGGACTGGTGGCTTTATAAGAAGAGGAAAAGAGAACTGAGCTAGCATGCC CAGCCCACAGAGAGCCTCCACTAGAGTGATGCTAAGTGGAAATGTGAGGTGCAGC TGCCACAGAGGGCCCCCACCAGGGAAATGTCTAGTGTCTAGTGGATCCAGGCCAC AGGAGAGAGTGCCTTGTGGAGCGCTGGGAGCAGGACCTGACCACCACCAGGACC CCAGAACTGTGGAGTCAGTGGCAGCATGCAGCGCCCCCTTGGGAAAGCTTTAGGC ACCAGCCTGCAACCCATTCGAGCAGCCACGTAGGCTGCACCCAGCAAAGCCACAG GCACGGGGCTACCTGAGGCCTTGGGGGCCCAATCCCTGCTCCAGTGTGTCCGTGA GGCAGCACACGAAGTCAAAAG AGATTATTCTCTTCCCACAGATACCTTTTCTCTCCCATGACCCTTTAACAGCATCTG CTTCATTCCCCTCACCTTCCCAGGCTGATCTGAGGTAAACTTTGAAGTAAAATAAA AGCTGTGTTTGAGCATCATTTGTATTTCAAAAAAAAAAAAAAAAA |
| 5 | Residues 3-11 of A*0101 leader And A*0301 And A*3601 And Cw*1502 | VMAPRTLLL |
| 6 | Residues 3-11 of A*0201 And A*0211 And A*2403 And A*2501 | VMAPRTLVL |
| 7 | Residues 3-11 of A*0702 and B*06501 And A*0801 | VMAPRTVLL |
| 8 | Residues 3- 11 of G | VMAPRTLFL |
| 9 | Residues 3-11 of Cw*0401 | VMAPRTLIL |
| 10 | HLA-C CRISPR guide RNA 1 | AGCGACGCCGCGAGTCCGAG |
| 11 | HLA-C CRISPR guide RNA 2 | GGTCGCAGCCAGACATCCTC |
| 12 | HLA-C CRISPR guide RNA 3 | GACACAGAAGTACAAGCGCC |
| 13 | HLA-C CRISPR guide RNA 4 | TCACCGCTATGATGTGTAGG |
| 14 | HLA-C CRISPR guide RNA 5 | CTCTCAGCTGCTCCGCCGCA |
| 15 | HLA-C CRISPR guide RNA 6 | CTTCCTCCTACACATCATAG |
| 16 | HLA-A CRISPR guide RNA 1 | CGTCCTGCCGGTACCCGCGG |
| 17 | HLA-A CRISPR guide RNA 2 | TACCGGCAGGACGCCTACGA |
| 18 | HLA-A CRISPR guide RNA 3 | ACAGCGACGCCGCGAGCCAG |
| 19 | HLA-A CRISPR guide RNA 4 | CCAGTCACAGACTGACCGAG |
| 20 | HLA-A CRISPR guide RNA 5 | TCCCTCCTTACCCCATCTCA |
| 21 | HLA-A CRISPR guide RNA 6 | CCACCCCATCTCTGACCATG |
| 22 | HLA-B CRISPR guide RNA 1 | CGTCGCAGCCGTACATGCTC |
| 23 | HLA-B CRISPR guide RNA 2 | CGCTGTCGAACCTCACGAAC |
| 24 | HLA-B CRISPR guide RNA 3 | GGATGGCGAGGACCAAACTC |
| 25 | HLA-B CRISPR guide RNA 4 | CCTGGCTGTCCTAGCAGTTG |
| 26 | HLA-B CRISPR guide RNA 5 | CGTACTGGTCATGCCCGCGG |
| 27 | HLA-B CRISPR guide RNA 6 | CTCCGATGACCACAACTGCT |
| 28 | A2-1 siRNA | GGATTACATCGCCCTGAAAG |
| 29 | A2-2 siRNA | GCAGGAGGGTCCGGAGTATT |
| 30 | A2-3 siRNA | GGACGGGGAGACACGGAAAG |
| 31 | A2-4 siRNA | GAAAGTGAAGGCCCACTCA |
| 32 | ABC-1 siRNA | GATACCTGGAGAACGGGAAG |
| 33 | ABC-2 siRNA | GCTGTGGTGGTGCCTTCTGG |
| 34 | ABC-3 siRNA | GCTACTACAACCAGAGCGAG |
| 35 | ABC-4 siRNA | GTGGCTCCGCAGATACCTG |
| 36 | HLA-A-specific shRNA | CACCUGCCAUGUGCAUGAUUUGUGUAGUCAUGCUGCACAUGGC AGGUGUUUUU GGAGAUCACACUGACCUGGCAUUUGUGUAGUGCCAGGUCAGU GUGAUCUCCUUUUUU |
| 37 | HLA ABC-specific shRNA | |
| 38 | spacer (IgG4hinge) (aa) | ESKYGPPCPPCP |
| | homo sapiens | |
| 39 | spacer (IgG4hinge) (nt) | GAATCTAAGTACGGACCGCCCTGCCCCCCTTGCCCT |
| | homo sapiens | |
| 40 | Hinge-CH3 spacer | |
| | Homo sapiens | |
| 41 | Hinge-CH2-CH3 spacer | ESKYGPPCPPCPGQPREPQVYTLPPSQEEMTKNQVSLTCLVKGFYPS DIAVEWESNGQPENNYKTTPPVLDSDGSFFLYSRLTVDKSRWQEGN VFSCSVMHEALHNHYTQKSLSLSLGK ESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQ DWLNGKEYKCKVSNKGLPSSIEKTISKAKGQPREPQVYTLPPSQEE MTKNQVSLTCLVKGFYPSDIAVEWESNGQPENNYKTTPPVLDSDGS FFLYSRLTVDKSRWQEGNVFSCSVMHEALHNHYTQKSLSLSLGK RWPESPKAQASSVPTAQPQAEGSLAKATTAPATTRNTGRGGEEKK KEKEKEEQEERETKTPECPSHTQPLGVYLLTPAVQDLWLRDKATFT CFVVGSDLKDAHLTWEVAGKVPTGGVEEGLLERHSNGSQSQHSRL TLPRSL |
| | Homo sapiens | |
| 42 | IgD-hinge-Fc | RWPESPKAQASSVPTAQPQAEGSLAKATTAPATTRNTGRGGEEKK KEKEKEEQEERETKTPECPSHTQPLGVYLLTPAVQDLWLRDKATFT CFVVGSDLKDAHLTWEVAGKVPTGGVEEGLLERHSNGSQSQHSRL TLPRSL |
| | Homo sapiens | |
| | | WNAGTSVTCTLNHPSLPPQRLMALREPAAQAPVKLSLNLLASSDPP EAASWLLCEVSGFSPPNILLMWLEDQREVNTSGFAPARPPPQPGSTT FWAWSVLRVPAPPSPQPATYTCVVSHEDSRTLLNASRSLEVSYVTD H |
| 43 | tEGFR | MLLLVTSLLLCELPHPAFLLIPRKVCNGIGIGEFKDSLSINATNIKHFK NCTSISGDLHILPVAFRGDSFTHTPPLDPQELDILKTVKEITGFLLIQA WPENRTDLHAFENLEIIRGRTKQHGQFSLAVVSLNITSLGLRSLKEIS DGDVIISGNKNLCYANTINWKKLFGTSGQKTKIISNRGENSCKATGQ VCHALCSPEGCWGPEPRDCVSCRNVSRGRECVDKCNLLEGEPREFV ENSECIQCHPECLPQAMNITCTGRGPDNCIQCAHYIDGPHCVKTCPA GVMGENNTLVWKYADAGHVCHLCHPNCTYGCTGPGLEGCPTNGP KIPSIATGMVGALLLLLVVALGIGLFM |
| | artificial | |
| 44 | T2A | LEGGGEGRGSLLTCGDVEENPGPR |
| | artificial | |
| 45 | CD28 (amino acids 153-179 of Accession No. P10747) | FWVLVVVGGVLACYSLLVTVAFIIFWV |
| | Homo sapiens | |
| 46 | CD28 (amino acids 114-179 of Accession No. P10747) | IEVMYPPPYLDNEKSNGTIIHVKGKHLCPSPLFPGPSKP FWVLVVVGGVLACYSLLVTVAFIIFWV |
| | Homo sapiens | |
| 47 | CD28 (amino acids 180-220 of P10747) | RSKRSRLLHSDYMNMTPRRPGPTRKHYQPYAPPRDFAAYRS |
| | Homo sapiens | |
| 48 | CD28 (LL to GG) Homo sapiens | RSKRSRGGHSDYMNMTPRRPGPTRKHYQPYAPPRDFAAYRS |
| 49 | 4-1BB (amino acids 214-255 of Q07011.1) | KRGRKKLLYIFKQPFMRPVQTTQEEDGCSCRFPEEEEGGCEL |
| | Homo sapiens | |
| 50 | CD3 zeta | RVKFSRSADAPAYQQGQNQLYNELNLGRREEYDVLDKRRGRDPE MGGKPRRKNPQEGLYN ELQKDKMAEA YSEIGMKGER RRGKGHDGLY QGLSTATKDTYDALHMQALP PR |
| | Homo sapiens | |
| 51 | CD3 zeta | RVKFSRSAEPPAYQQGQNQLYNELNLGRREEYDVLDKRRGRDPEM GGKPRRKNPQEGLYN ELQKDKMAEA YSEIGMKGER RRGKGHDGLY QGLSTATKDTYDALHMQALP PR |
| | Homo sapiens | |
| 52 | CD3 zeta | RVKFSRSADAPAYKQGQNQLYNELNLGRREEYDVLDKRRGRDPE MGGKPRRKNPQEGLYN ELQKDKMAEA YSEIGMKGER RRGKGHDGLY QGLSTATKDTYDALHMQALP PR |
| | Homo sapiens | |
| 53 | Linker | GSADDAKKDAAKKDGKS |
| 54 | Linker | -PGGG-(SGGGG)₅-P- wherein P is proline, G is glycine and S is serine |
| 55 | Linker | -PGGG-(SGGGG)₆-P- wherein P is proline, G is glycine and S is serine |
| 56 | T2A | EGRGSLLTCG DVEENPGP |
| 57 | P2A | GSGATNFSLL KQAGDVEENP GP |
| 58 | P2A | ATNFSLLKQA GDVEENPGP |
| 59 | E2A | QCTNYALLKL AGDVESNPGP |
| 60 | F2A | VKQTLNFDLL KLAGDVESNP GP |
| 61 | tEGFR | RKVCNGIGIGEFKDSLSINATNIKHFKNCTSISGDLHILPVAFRGDSFT HTPPLDPQELDILKTVKEITGFLLIQAWPENRTDLHAFENLEIIRGRT KQHGQFSLAVVSLNITSLGLRSLKEISDGDVIISGNKNLCYANTINW KKLFGTSGQKTKIISNRGENSCKATGQVCHALCSPEGCWGPEPRDC VSCRNVSRGRECVDKCNLLEGEPREFVENSECIQCHPECLPQAMNIT CTGRGPDNCIQCAHYIDGPHCVKTCPAGVMGENNTLVWKYADAG HVCHLCHPNCTYGCTGPGLEGCPTNGPKIPSIATGMVGALLLLLVV ALGIGLFM |

Further aspects of the invention are defined by the following clauses:
1. A method of identifying a peptide epitope, comprising:
   a) contacting an MHC-E molecule with one or more peptides; and
   b) detecting or identifying peptide(s) in the context of the MHC-E molecule.
2. The method of clause 1, wherein the one or more peptides comprise peptides of one or more protein antigens.
3. The method of clause 2, wherein the one or more peptides in the context of the MHC-E molecule are identified as peptide epitopes of the one or more protein antigens.
4. The method of clause 2 or clause 3, wherein the antigen is a tumor antigen.
5. The method of clauses 2 or clause 3, wherein the antigen is a pathogenic antigen.
6. The method of clause 5, wherein the pathogenic antigen is a bacterial antigen or viral antigen.
7. The method of any of clauses 1-6, wherein the MHC-E molecule is expressed on the surface of a cell.
8. The method of any of clauses 1-7, further comprising identifying a peptide binding molecule or antigen-binding fragment thereof that binds to at least one of the one or more peptides in the context of the MHC-E molecule.
9. A method of identifying a peptide binding molecule that binds to one or more peptides in the context of an MHC-E molecule, comprising:
   a) providing a cell comprising one or more peptides in the context of an MHC-E molecule on the surface of the cell; and
   b) identifying a peptide binding molecule or antigen-binding fragment thereof that binds to at least one of the one or more peptides in the context of the MHC-E molecule.
10. The method of clause 9, wherein providing a cell comprising one or more peptides in the context of an MHC-E molecule comprises contacting the MHC-E molecule on the surface of the cell with the one or more peptides.
11. The method of clause 10, comprising detecting or identifying if the peptide(s) in the context of an MHC-E molecule is present or formed on the surface of the cell prior to providing the cell in a).
12. The method of any of clauses 9-11, wherein the one or more peptides comprise peptides of a protein antigen.
13. The method of any of clauses 9-12, wherein the antigen is a tumor antigen or a pathogenic antigen.
14. The method of clause 13, wherein the pathogenic antigen is a bacterial antigen or viral antigen.
15. The method of clause 6 or clause 14, wherein the antigen is a viral antigen and the viral antigen is from hepatitis A, hepatitis B, hepatitis C virus (HCV), human papilloma virus (HPV), hepatitis viral infections, Epstein-Barr virus (EBV), human herpes virus 8 (HHV-8), human T-cell leukemia virus-1 (HTLV-1), human T-cell leukemia virus-2 (HTLV-2), or a cytomegalovirus (CMV).
16. The method of clause 15, wherein the antigen is an HPV antigen selected from among HPV-16, HPV-18, HPV-31, HPV-33 and HPV-35.
17. The method of clause 15, wherein the viral antigen is an EBV antigen selected from among Epstein-Barr nuclear antigen (EBNA)-1, EBNA-2, EBNA-3A, EBNA-3B, EBNA-3C, EBNA-leader protein (EBNA-LP), latent membrane proteins LMP-1, LMP-2A and LMP-2B, EBV-EA, EBV-MA and EBV-VCA.
18. The method of clause 15, wherein the viral antigen is an HTLV-antigen that is TAX
19. The method of clause 15, wherein the viral antigen is an HBV antigen that is a hepatitis B core antigen or a hepatitis B envelope antigen.
20. The method of any of clauses 9-13, wherein the antigen is a tumor antigen.
21. The method of any of clauses 4, 7-8 and 20, wherein the tumor antigen is selected from among glioma-associated antigen, β-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, Melanin-A/MART-1, WT-1, S-100, MBP, CD63, MUC1 (e.g. MUC1-8), p53, Ras, cyclin B1, HER-2/neu, carcinoembryonic antigen (CEA), gp100, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A11, MAGE-B1, MAGE-B2, MAGE-B3, MAGE-B4, MAGE-C1, BAGE, GAGE-1, GAGE-2, pl5, tyrosinase (e.g. tyrosinase-related protein 1 (TRP-1) or tyrosinase-related protein 2 (TRP-2)), β-catenin, NY-ESO-1, LAGE-la, PP1, MDM2, MDM4, EGVFvIII, Tax, SSX2, telomerase, TARP, pp65, CDK4, vimentin, S100, eIF-4A1, IFN-inducible p78, melanotransferrin (p97), Uroplakin II, prostate specific antigen (PSA), human kallikrein (huK2), prostate specific membrane antigen (PSM), and prostatic acid phosphatase (PAP), neutrophil elastase, ephrin B2, BA-46, Bcr-abl, E2A-PRL, H4-RET, IGH-IGK, MYL-RAR, Caspase 8, FRa, CD24, CD44, CD133, CD 166, epCAM, CA-125, HE4, Oval, estrogen receptor, progesterone receptor, uPA, PAI-1, CD19, CD20, CD22, ROR1, CD33/IL3Ra, c-Met, PSMA, Glycolipid F77, GD-2, insulin growth factor (IGF)-I, IGF-II, IGF-I receptor and mesothelin.
22. The method of any of clauses 1-8 and 11-21, wherein detecting or identifying the one or more peptide(s) in the context of an MHC-E molecule comprises extracting peptides from a lysate of the cell, eluting peptides from the cell surface or isolating the MHC-E molecule or molecules and eluting the one or more peptides from the MHC-E molecule.
23. The method of any of clauses 1-8 and 11-22, wherein the one or more peptides comprises one or more peptides having a length of from or from about 8 to 20 amino acids or 9 to 15 amino acids.
24. The method of any of clauses 1-8 and 11-23, wherein the one or more peptides comprises peptides having a length of or about 9 amino acids, about 10 amino acids, about 11 amino acids, about 12 amino acids, about 13 amino acids, about 14 amino acids or about 15 amino acids.
25. The method of any of clauses 1-8 and 11-24, wherein the one or more peptides comprises overlapping peptides of the antigen or a region of the antigen.
26. The method of clause 25, wherein the overlapping peptides, collectively, comprise peptides that represent at least 30%, at least 40%, at least 50%, at least 60%, at least 70%, at least 80%, at least 90% or more of a contiguous sequence of amino acids of the antigen.
27. The method of any of clauses 1-8 and 11-24, wherein peptides comprised by the one or more peptides are present in orthogonal pools, each of said orthogonal pools comprising at least two or more different peptides, wherein at least one peptide in each orthogonal pool is the same as a peptide present in at least another orthogonal pool.
28. The method of clause 27, wherein a peptide in the context of the MHC-E molecule is detected or identified if the peptide is eluted or extracted from the MHC-E molecule or from a cell expressing the MHC-E molecule in at least two orthogonal pools comprising the same peptide.
29. The method of any of clauses 7-24, wherein the cell comprises one or a combination of antigens heterologous or exogenous to the cell.
30. The method of clause 29, wherein the one or combination of antigens present in the cell is processed to one or more peptides, thereby contacting the MHC-E molecule with one or more peptides.
31. The method of clause 29 or clause 30, wherein the cell comprises one or a combination of synthetic nucleic acid molecules comprising one or more coding sequences encoding the one or combination of antigens.
32. A method of identifying a peptide epitope, comprising:
   a) introducing a synthetic nucleic acid molecule or a combination of synthetic nucleic acid molecules comprising one or more coding sequences encoding an antigen or a combination of antigens into a cell expressing an MHC-E molecule;
   b) incubating the cell under conditions whereby the encoded antigen or antigens are processed to peptides; and
   c) detecting or identifying peptide(s) of the antigen in the context of the MHC-E molecule.
33. The method of clause 32, further comprising identifying a peptide binding molecule or antigen-binding fragment thereof that binds to at least one of the one or more peptides of the antigen in the context of the MHC-E molecule.
34. A method of identifying a peptide binding molecule that binds a peptide in the context of an MHC-E molecule, comprising:
   a) introducing a synthetic nucleic acid molecule or a combination of synthetic nucleic acid molecules comprising one or more coding sequences encoding an antigen or combination of antigens into a cell expressing an MHC-E molecule;
   b) incubating the cell under conditions whereby the encoded antigen or combination of antigens is processed to peptides; and
   c) identifying a peptide binding molecule or antigen-binding fragment thereof that binds to at least one of the one or more peptides of the antigen in the context of the MHC-E molecule.
35. The method of any of clauses 32-34, wherein the antigen is a protein antigen.
36. The method of clause 35, wherein the one or more peptides in the context of the MHC-E molecule are identified as peptide epitopes of the one or more protein antigen.
37. The method of clause 35 or clause 36, wherein the antigen is a tumor antigen or a pathogenic antigen.
38. The method of any of clauses 31-37, wherein the synthetic nucleic acid is synthetic DNA.
39. The method of clause 38, wherein the synthetic DNA is complementary DNA (cDNA).
40. The method of any of clauses 31-39, wherein the cell comprises a synthetic nucleic acid comprising a coding sequence encoding the antigen.
41. The method of any of clauses 31-40, wherein the cell comprises a combination of synthetic nucleic acids each individually comprising a coding sequence of one of the combination of antigens.
42. The method of clause 41, wherein the combination of synthetic nucleic acids comprises one or more nucleic acid molecules of a cDNA library.
43. The method of clause 42, wherein the cDNA library is a tumor-derived cDNA library.
44. The method of clause 43, wherein the tumor is a melanoma, sarcoma, breast carcinoma, renal carcinoma, lung carcinoma, ovarian carcinoma, prostate carcinoma, colorectal carcinoma, pancreatic carcinoma, squamous tumor of the head and neck, or squamous carcinoma of the lung.
45. The method of any of clauses 31-44, wherein the combination of synthetic nucleic acid molecules and/or combination of encoded antigens is present in orthogonal pools, each of said orthogonal pools comprising at least two or more different synthetic nucleic acid molecules and/or encoded antigens, wherein at least one synthetic nucleic acid molecule and/or encoded antigen is the same in at least two orthogonal pools.
46. The method of clause 45, wherein a peptide in the context of an MHC-E molecule is detected or identified if the peptide is eluted or extracted from an MHC-E molecule or from a cell expressing an MHC-E molecule in at least two orthogonal pools comprising the same peptide.
47. The method of any of clauses 1-46, that is performed in an array.
48. The method of clause 47, wherein the array is an addressable or spatial array.
49. The method of any of clauses 1-48, wherein the MHC-E molecule is an HLAE^{∗}01:01 or HLA E^{∗}0103.
50. The method of any of clauses 7-49, wherein the cell is a primary cell or is a cell line.
51. The method of any of clauses 7-50, wherein the cell is a human cell.
52. The method of any of clauses 7-51, wherein the cell is selected from among a fibroblast, a B cell, a dendritic cell and a macrophage.
53. The method of any of clauses 7-52, wherein the cell is a cell line and the cell line is or is derived from a cell selected from among K562, C1R, KerTr, HCT-15, DLD-1, Daudi, 221, 721.221, BLS-1, BLS-2, JEG-3 and JAR.
54. The method of clause 53, wherein the cell is or is derived from a 221 or K562 cell line.
55. The method of any of clauses 7-54, wherein the cell is an artificial antigen presenting cell.
56. The method of clause 55, wherein the artificial antigen presenting cell expresses the MHC-E molecule and one or more of a stimulatory or costimulatory molecule(s), an Fc receptor, an adhesion molecule(s) or a cytokine.
57. The method of any of clauses 7-56, wherein the cell is genetically or recombinantly engineered to express the MHC-E molecule.
58. The method of any of clauses 7-32, and 47-57, wherein:
   (1) the cell has been or is incubated with an activating or stimulating agent prior to or simultaneously with contacting the MHC-E molecule with the one or more peptides; or
   (2) the method further comprises incubating the cell with an activating or stimulating agent prior to or simultaneously with contacting the MHC-E molecule with the one or more peptides.
59. The method of any of clauses 33-57, wherein:
   (1) the cell has been or is incubated with an activating or stimulating agent prior to or simultaneously with introducing the synthetic nucleic acid molecule or combination of synthetic nucleic acid molecules into the cell; or
   (2) the method further comprises incubating the cell with an activating or stimulating agent prior to or simultaneously with introducing the synthetic nucleic acid molecule or combination of synthetic nucleic acid molecules into the cell.
60. The method of clause 58 or clause 59, wherein the incubating with the activating or stimulating agent increases expression of the MHC-E molecule on the surface of the cell compared to expression of the MHC-E molecule in the absence of said activating or stimulating.
61. The method of clause 60, wherein expression of the MHC-E molecule is increased at least 1.2-fold, 1.5-fold, 2-fold, 3-fold, 4-fold, 5-fold, 6-fold, 7-fold, 8-fold, 9-fold or 10-fold.
62. The method of clause 60 or clause 61, wherein the activating or stimulating is effected in the presence of interferon gamma.
63. The method of clause 60 or clause 61, wherein the activating or stimulating comprises incubating the cell with a virus or viral particle.
64. The method of clause 63, wherein the virus or viral particle is a cytomegalovirus (CMV).
65. The method of any of clauses 7-64, wherein the cell is repressed and/or disrupted in a gene encoding a classical MHC class I molecule and/or does not express a classical MHC class I molecule.
66. The method of clause 65, wherein the repression is effected by an inhibitory nucleic acid molecule.
67. The method of clause 66, wherein the inhibitory nucleic acid molecule comprises an RNA interfering agent.
68. The method of clause 66 or clause 67, wherein the inhibitory nucleic acid is or comprises or encodes a small interfering RNA (siRNA), a microRNA-adapted shRNA, a short hairpin RNA (shRNA), a hairpin siRNA, a microRNA (miRNA-precursor) or a microRNA (miRNA).
69. The method of clause 68, wherein disruption of the gene is mediated by a gene editing nuclease, a zinc finger nuclease (ZFN), a clustered regularly interspaced short palindromic nucleic acid (CRISPR) /Cas9, and/or a TAL-effector nuclease (TALEN).
70. The method of any of clauses 65-69, wherein expression of the classical MHC class I molecule in the cell is reduced by at least 50%, 60%, 70%, 80%, 90%, or 95% as compared to the expression in the cell in the absence of the repression or gene disruption.
71. The method of any of clauses 65-70, wherein the classical MHC class I molecule is an HLA-A, HLA-B or HLA-C molecule.
72. The method of any of clauses 32 and 35-71, wherein detecting or identifying a peptide in the context of an MHC-E molecule comprises extracting peptides from a lysate of the cell, eluting peptides from the cell surface or isolating the MHC-E molecule or molecules and eluting the one or more peptides from the MHC-E molecule.
73. The method of clause 22 or clause 72, wherein isolating the MHC-E molecule or molecules comprising solubilizing the cell and selecting the MHC-E molecule by immunoprecipitation or immunoaffinity chromatography.
74. The method of clause 22, clause 28 or clause 72, wherein eluting peptides from an MHC-E molecule is effected in the presence of a mild acid or a diluted acid.
75. The method of any of clauses 1-8 and 11-74, comprising fractionating, separating or purifying the identified or detected peptide(s).
76. The method of any of clauses 1-8 and 11-75, comprising sequencing the identified or detected peptide(s).
77. The method of any of clauses 1-8 and 11-76, further comprising determining if the identified or detected peptide(s) elicit an antigen-specific immune response.
78. The method of clause 77, wherein the antigen-specific immune response is a humoral T cell response.
79. The method of clause 77, wherein the antigen-specific immune response is a cytotoxic T lymphocyte response.
80. The method of any of clauses7-79, wherein the cell is a test cell and the method further comprises:
   detecting or identifying peptides in the context of an MHC-E molecule on the surface of a control cell, said control cell having not been contacted with the one or more peptides; and
   identifying peptide(s) in the context of an MHC-E molecule that is unique to the test cell compared to the control cell, thereby identifying the one or more peptides of the antigen in the context of an MHC-E molecule.
81. The method of any of clauses 1-80 that is performed in vitro.
82. The method of any of clauses 1-81, wherein:
   the peptide(s) identified in the context of the MHC-E molecule comprise a length of from or from about 8 to 13 amino acids; or
   the peptide(s) identified in the context of the MHC-E molecule comprise a length of or about 8 amino acids, 9 amino acids, 10 amino acids or 11 amino acids.
83. The method of any of clauses 1-82, wherein the peptide(s) in the context of the MHC-E molecule have a binding affinity with an IC50 for the MHC-E molecule of greater than 200 nM, 300 nM, 400 nM, 500 nM, 600 nM, 700 nM, 800 nM, 900 nM, or 1000.
84. The method of any of clauses 1-83, wherein the peptide(s) in the context of the MHC-E molecule have a binding affinity with an IC50 for the MHC-E molecule of less than 500 nm, 400 nM, 300 nM, 200 nM, 100 nM, or 50 nM.
85. The method of any of clauses 1-84, wherein the peptide(s) in the context of the MHC-E molecule are capable of inducing a CD8+ immune response in a subject.
86. The method of clause 85, wherein the peptide(s) in the context of the MHC-E molecule are capable of generating a universal immune response in a majority of subjects in a population.
87. The method of clause 86, wherein the universal immune response is elicited in greater than 50%, 60%, 70%, 80%, or 90% of subjects in a population.
88. The method of any of clauses 85-87, wherein the subjects are human subjects.
89. A peptide epitope identified by the methods of any of clauses 1-8 and 15-88.
90. A stable MHC-E-peptide complex, comprising the peptide epitope of clause 89.
91. The stable MHC-E-peptide complex of clause 90 that is present on a cell surface.
92. The method of any of clauses 8-21 and 33-88, wherein identifying the peptide binding molecule or antigen-binding fragment thereof comprises:
   a) assessing binding of a plurality of candidate peptide binding molecules or antigen-binding fragments thereof to the surface of the cell; and
   b) identifying from among the plurality one or more peptide binding molecules that bind to the at least one of the one or more peptides in the context of an MHC-E molecule.
93. A method of identifying a peptide binding molecule or antigen-binding fragment thereof that binds a peptide in the context of an MHC-E molecule, comprising:
   a) assessing binding of a plurality of candidate peptide binding molecules or antigen-binding fragments thereof to the stable MHC-E-peptide complex of clause 89 or clause 90; and
   b) identifying from among the plurality one or more peptide binding molecules that bind to the peptide in the context of an MHC-E molecule.
94. A method of identifying a peptide binding molecule or antigen-binding fragment thereof that binds an MHC-E-restricted peptide, comprising:
   a) identifying a peptide by the method of any of clauses 1-8 and 15-88;
   b) assessing binding of a plurality of candidate peptide binding molecules or antigen-binding fragments thereof to an MHC-E molecule comprising the peptide of a) bound thereto; and
   c) identifying from among the plurality one or more peptide binding molecules that bind to the peptide in the context of the MHC-E molecule.
95. The method of any of clauses 92-94, wherein the plurality of candidate peptide binding molecules comprises one or more T cell receptors (TCRs), antigen-binding fragments of a TCR, antibodies or antigen-binding fragments thereof.
96. The method of any of clauses 92-95, wherein the plurality of candidate peptide binding molecules comprises at least 2, 5, 10, 100, 10³, 10⁴, 10⁵, 10⁶, 10⁷, 10⁸, 10⁹, or more different molecules.
97. The method of any of clauses 92-96, wherein the plurality of candidate peptide binding molecules comprise peptide binding molecules comprising one or more mutations compared to a parent or scaffold peptide binding molecule, wherein individual candidate peptide binding molecules comprise one or more different mutations compared to other candidate peptide binding molecules in the plurality.
98. The method of clause 97, wherein the one or more amino acid mutations comprise a mutation or mutations in a complementarity determining region (CDR) or CDRs of the molecule.
99. The method of any of clauses 92-98, wherein the candidate peptide binding molecules are obtained from a sample from a subject or a population of subjects.
100. The method of clause 99, wherein the subject or population of subjects comprises normal or healthy subjects or diseased subjects.
101. The method of clause 100, wherein the diseased subjects are tumor-bearing subj ects.
102. The method of any of clauses 99-101, wherein the subject has been vaccinated with the peptide epitope of the antigen.
103. The method of any of clauses 99-102, wherein the subject is a human or rodent.
104. The method of clauses 99-103, wherein the subject is an HLA-transgenic mouse and/or is a human TCR transgenic mouse.
105. The method of any of clauses 99-104, wherein the sample comprises T cells.
106. The method of clause 105, wherein the sample comprises peripheral blood mononuclear cells (PBMCs) or tumor-infiltrating lymphocytes (TIL).
107. The method of any of clauses 95-106, wherein the candidate peptide binding molecule comprises a T cell receptor (TCR) or an antigen-binding fragment of a TCR.
108. The method of any of clauses 95-107, wherein the antigen-binding fragment of a TCR is a single chain TCR (scTCR).
109. The method of any of clauses 99-103, wherein the sample comprises B cells.
110. The method of clause 109, wherein the sample is selected from among blood, bone marrow and spleen and/or the sample comprises PBMCs, splenocytes or bone marrow cells.
111. The method of any of clauses 95-103, 109 and 110, wherein the candidate peptide binding molecules comprise antibodies or antigen-binding fragments thereof.
112. The method of clause 111, wherein the candidate peptide binding molecules comprise IgM-derived antibodies or antigen-binding fragments and/or are naive.
113. The method of clause 111 or clause 112, wherein the antibodies or antigen-binding fragments thereof are produced by immunizing a host with an immunogen comprising the MHC-peptide complex.
114. The method of any of clauses 111-113, wherein the candidate peptide binding molecule is a single chain variable fragment (scFv).
115. The method of any of clauses 92-114, wherein the candidate peptide binding molecules are present in a display library.
116. The method of clause 115, wherein the display library is selected from among a cell surface display library, a phage display library, a ribosome display library, an mRNA display library, and a dsDNA display library.
117. The method of any of clauses 8-21 and 33-116, wherein:
   the identified peptide binding molecule exhibits binding affinity for the peptide in the context of an MHC-E molecule with a dissociation constant (K_{D}) of from or from about 10⁻⁵ M to 10⁻¹³ M, 10⁻⁵ M to 10⁻⁹ or 10⁻⁷ M to 10⁻¹²; or
   the identified peptide binding molecule exhibits binding affinity for the peptide in the context of an MHC-E molecule with a K_{D} of less than or less than about 10⁻⁵ M, 10⁻⁶ M, 10⁻⁷ M, 10⁻⁸ M, 10⁻⁹ M, 10⁻¹⁰ M, 10⁻¹¹ M or less.
118. A peptide binding molecule identified by the method of any of clauses 8-21 and 33-117.
119. The peptide binding molecule of clause 118 that is a TCR or antigen-binding fragment thereof.
120. The peptide binding molecule of clause 118 that is an antibody or antigen-binding fragment thereof.
121. A recombinant antigen receptor, comprising the peptide binding molecule of any of clauses 118-120.
122. The recombinant antigen receptor of clause 121 that is a chimeric antigen receptor (CAR).
123. A genetically engineered cell, expressing the peptide binding molecule of any of clauses 118-120 or a recombinant receptor of clause 121 or clause 122.
124. The genetically engineered cell of clause 123 that is a T cell.
125. The genetically engineered cell of clause 124 that is a CD8+ T cell.
126. A CD8+ genetically engineered cell, expressing a peptide binding molecule or a recombinant receptor comprising a peptide binding molecule, wherein the peptide binding molecule specifically binds a peptide epitope in the context of an MHC-E molecule.
127. The CD8+ genetically engineered cell of clause 126, wherein the peptide binding molecule is a T cell receptor (TCR), an antigen-binding fragment of a TCR, an antibody or an antigen-binding fragment of an antibody.
128. The CD8+ genetically engineered cell of clause 126 or clause 127, wherein the recombinant antigen receptor is a chimeric antigen receptor (CAR).
129. A composition, comprising a peptide binding molecule of any of clauses 118-120, a recombinant receptor of clause 121 or clause 122 or a genetically engineered cell of any of clauses 123-128.
130. The composition of clause 129, further comprising a pharmaceutically acceptable excipient.
131. A method of treating a disease or condition, comprising administering to a subject a composition of clause 129 or clause 130.
132. The method of clause 131, wherein the peptide binding molecule or recombinant receptor binds to an antigen associated with the disease or condition.
133. The method of clause 131 or clause 132, wherein the disease or condition is a tumor or a cancer.
134. A pharmaceutical composition of clause 129 or clause 130 for use in treating a disease or condition.
135. The pharmaceutical composition for use of clause 134, wherein the peptide binding molecule or recombinant receptor binds to an antigen associated with the disease or condition.
136. The pharmaceutical composition for use of clause 134 or clause 135, wherein the disease or condition is a tumor or a cancer.

## Claims

1. An in vitro method of identifying a peptide binding molecule that binds a peptide in the context of an MHC-E molecule, comprising:
a) introducing a combination of synthetic nucleic acid molecules into a cell expressing an MHC-E molecule, wherein the combination of synthetic nucleic acid molecules comprises nucleic acids each individually comprising a coding sequence encoding a protein antigen of one of a combination of protein antigens;
b) incubating the cell under conditions whereby the encoded protein antigen or combination of protein antigens is processed to peptides; and
c) identifying a peptide binding molecule or antigen-binding fragment thereof that binds to at least one of the one or more peptide(s) of the protein antigen in the context of the MHC-E molecule.

2. The method of claim 1, wherein the combination of synthetic nucleic acids comprises one or more nucleic acid molecules of a cDNA library, optionally
wherein the cDNA library is a tumor-derived cDNA library, further optionally
wherein the tumor is a melanoma, sarcoma, breast carcinoma, renal carcinoma, lung carcinoma, ovarian carcinoma, prostate carcinoma, colorectal carcinoma, pancreatic carcinoma, squamous tumor of the head and neck, or squamous carcinoma of the lung.

3. The method of claim 1 or claim 2, wherein the combination of synthetic nucleic acid molecules and/or the combination of encoded protein antigens is present in orthogonal pools, each of said orthogonal pools comprising at least two or more different synthetic nucleic acid molecules and/or encoded protein antigens, wherein at least one synthetic nucleic acid molecule and/or encoded protein antigen is the same in at least two orthogonal pools.

4. The method of any of claims 1-3, wherein the method is performed in an array, optionally
wherein the array is an addressable or spatial array.

5. The method of any of claims 1-4, wherein:
i) the cell is a primary cell or is a cell line; and/or
ii) the cell is a human cell; and/or
iii) the cell is selected from among a fibroblast, a B cell, a dendritic cell and a macrophage; and/or
iv) the cell is an artificial antigen presenting cell.

6. The method of any of claims 1-5, wherein the cell is genetically or recombinantly engineered to express the MHC-E molecule.

7. The method of any of claims 1-6, wherein:
(1) the cell has been or is incubated with an activating or stimulating agent prior to or simultaneously with introducing the combination of synthetic nucleic acid molecules into the cell; or
(2) the method further comprises incubating the cell with an activating or stimulating agent prior to or simultaneously with introducing the combination of synthetic nucleic acid molecules into the cell, optionally
wherein the incubating with the activating or stimulating agent increases expression of the MHC-E molecule on the surface of the cell compared to expression of the MHC-E molecule in the absence of said activating or stimulating, further optionally
wherein the activating or stimulating is effected in the presence of interferon gamma.

8. The method of any of claims 1-7, wherein the cell is repressed and/or disrupted in a gene encoding a classical MHC class I molecule and/or does not express a classical MHC class I molecule.

9. The method of any of claims 1-8, wherein identifying the peptide binding molecule or antigen-binding fragment thereof comprises:
a) assessing binding of a plurality of candidate peptide binding molecules or antigen-binding fragments thereof to the surface of the cell; and
b) identifying from among the plurality of candidate peptide binding molecules or antigen binding fragments thereof one or more peptide binding molecule that binds to the at least one of the one or more peptide(s) of the protein antigen in the context of an MHC-E molecule.

10. The method of claim 9, wherein the plurality of candidate peptide binding molecules comprises one or more T cell receptors (TCRs), antigen-binding fragments of a TCR, antibodies or antigen-binding fragments thereof.

11. The method of claim 9 or claim 10, wherein the plurality of candidate peptide binding molecules comprises at least 2, 5, 10, 100, 10³, 10⁴, 10⁵, 10⁶, 10⁷, 10⁸, 10⁹, or more different molecules.

12. The method of any of claims 9-11, wherein the plurality of candidate peptide binding molecules comprise peptide binding molecules comprising one or more mutations compared to a parent or scaffold peptide binding molecule, wherein individual candidate peptide binding molecules comprise one or more different mutations compared to other candidate peptide binding molecules in the plurality, optionally
wherein the one or more amino acid mutations comprise a mutation or mutations in a complementarity determining region (CDR) or CDRs of the molecule.

13. The method of any of claims 9-12, wherein the candidate peptide binding molecules are obtained from a sample from a subject or a population of subjects, optionally
wherein the subject has been vaccinated with the peptide epitope of the antigen.

14. The method of any of claims 1-13, wherein:
the identified peptide binding molecule exhibits binding affinity for the peptide in the context of an MHC-E molecule with a dissociation constant (K_{D}) of from or from about 10⁻⁵ M to 10⁻¹³ M, 10⁻⁵ M to 10⁻⁹ or 10⁻⁷ M to 10⁻¹²; or
the identified peptide binding molecule exhibits binding affinity for the peptide in the context of an MHC-E molecule with a K_{D} of less than or less than about 10⁻⁵ M, 10⁻⁶ M, 10⁻⁷ M, 10⁻⁸ M, 10⁻⁹ M, 10⁻¹⁰ M, 10⁻¹¹ M or less.

15. A peptide binding molecule identified by the method of any of claims 1-14.


SEQUENCE LISTING

<110>  Juno Therapeutics, Inc.
       Tareen, Semih U.
       Sissons, James
       Frohlich, Mark
       Ebens, Allen

<120>  MHC-E RESTRICTED EPITOPES, BINDING MOLECULES AND RELATED METHODS
       AND USES

<130>  735042002240

<140> Not Yet Assigned
<141> Concurrently Herewith

<150>  US 62/355,236
<151>  2016-06-27

<160>  61

<170>  PatentIn version 3.5

<210>  1
<211>  358
<212>  PRT
<213>  Homo sapiens


<220>
<221>  misc_feature
<223>  Major histocompatibility complex, class I, E, E*0101

<400>  1

<210> 2
<211> 1670
<212> DNA
<213> Homo sapiens

<220>
<221> misc_feature
<223> Major histocompatibility complex, class I, E (CDS for amino acids
15-1091)

<400> 2
cagaggctgg gatcatggta gatggaaccc tccttttact cctctcggag gccctggccc 60

ttacccagac ctgggcgggc tcccactcct tgaagtattt ccacacttcc gtgtcccggc 120

ccggccgcgg ggagccccgc ttcatctctg tgggctacgt ggacgacacc cagttcgtgc 180

gcttcgacaa cgacgccgcg agtccgagga tggtgccgcg ggcgccgtgg atggagcagg 240

aggggtcaga gtattgggac cgggagacac ggagcgccag ggacaccgca cagattttcc 300

gagtgaacct gcggacgctg cgcggctact acaatcagag cgaggccggg tctcacaccc 360

tgcagtggat gcatggctgc gagctggggc ccgacaggcg cttcctccgc gggtatgaac 420

agttcgccta cgacggcaag gattatctca ccctgaatga ggacctgcgc tcctggaccg 480

cggtggacac ggcggctcag atctccgagc aaaagtcaaa tgatgcctct gaggcggagc 540

accagagagc ctacctggaa gacacatgcg tggagtggct ccacaaatac ctggagaagg 600

ggaaggagac gctgcttcac ctggagcccc caaagacaca cgtgactcac caccccatct 660

ctgaccatga ggccaccctg aggtgctggg ccctgggctt ctaccctgcg gagatcacac 720

tgacctggca gcaggatggg gagggccata cccaggacac ggagctcgtg gagaccaggc 780

ctgcagggga tggaaccttc cagaagtggg cagctgtggt ggtgccttct ggagaggagc 840

agagatacac gtgccatgtg cagcatgagg ggctacccga gcccgtcacc ctgagatgga 900

agccggcttc ccagcccacc atccccatcg tgggcatcat tgctggcctg gttctccttg 960

gatctgtggt ctctggagct gtggttgctg ctgtgatatg gaggaagaag agctcaggtg 1020

gaaaaggagg gagctactct aaggctgagt ggagcgacag tgcccagggg tctgagtctc 1080

acagcttgta aagcctgaga cagctgcctt gtgtgcgact gagatgcaca gctgccttgt 1140

gtgcgactga gatgcaggat ttcctcacgc ctcccctatg tgtcttaggg gactctggct 1200

tctctttttg caagggcctc tgaatctgtc tgtgtccctg ttagcacaat gtgaggaggt 1260

agagaaacag tccacctctg tgtctaccat gacccccttc ctcacactga cctgtgttcc 1320

ttccctgttc tcttttctat taaaaataag aacctgggca gagtgcggca gctcatgcct 1380

gtaatcccag cacttaggga ggccgaggag ggcagatcac gaggtcagga gatcgaaacc 1440

atcctggcta acacggtgaa accccgtctc tactaaaaaa tacaaaaaat tagctgggcg 1500

cagaggcacg ggcctgtagt cccagctact caggaggcgg aggcaggaga atggcgtcaa 1560

cccgggaggc ggaggttgca gtgagccagg attgtgcgac tgcactccag cctgggtgac 1620

agggtgaaac gccatctcaa aaaataaaaa ttaaaaaaaa aaaaaaaaaa 1670

<210> 3
<211> 358
<212> PRT
<213> Homo sapiens

<220>
<221> misc_feature
<223> Major histocompatibility complex, class I, E, E*0103

<400> 3

Met Val Asp Gly Thr Leu Leu Leu Leu Leu Ser Glu Ala Leu Ala Leu
1               5                   10                  15

Thr Gln Thr Trp Ala Gly Ser His Ser Leu Lys Tyr Phe His Thr Ser
            20                  25                  30

Val Ser Arg Pro Gly Arg Gly Glu Pro Arg Phe Ile Ser Val Gly Tyr
        35                  40                  45

Val Asp Asp Thr Gln Phe Val Arg Phe Asp Asn Asp Ala Ala Ser Pro
    50                  55                  60

Arg Met Val Pro Arg Ala Pro Trp Met Glu Gln Glu Gly Ser Glu Tyr
65                  70                  75                  80

Trp Asp Arg Glu Thr Arg Ser Ala Arg Asp Thr Ala Gln Ile Phe Arg
                85                  90                  95

Val Asn Leu Arg Thr Leu Arg Gly Tyr Tyr Asn Gln Ser Glu Ala Gly
            100                 105                 110

Ser His Thr Leu Gln Trp Met His Gly Cys Glu Leu Gly Pro Asp Gly
        115                 120                 125

Arg Phe Leu Arg Gly Tyr Glu Gln Phe Ala Tyr Asp Gly Lys Asp Tyr
    130                 135                 140

Leu Thr Leu Asn Glu Asp Leu Arg Ser Trp Thr Ala Val Asp Thr Ala
145                 150                 155                 160

Ala Gln Ile Ser Glu Gln Lys Ser Asn Asp Ala Ser Glu Ala Glu His
                165                 170                 175

Gln Arg Ala Tyr Leu Glu Asp Thr Cys Val Glu Trp Leu His Lys Tyr
            180                 185                 190

Leu Glu Lys Gly Lys Glu Thr Leu Leu His Leu Glu Pro Pro Lys Thr
        195                 200                 205

His Val Thr His His Pro Ile Ser Asp His Glu Ala Thr Leu Arg Cys
    210                 215                 220

Trp Ala Leu Gly Phe Tyr Pro Ala Glu Ile Thr Leu Thr Trp Gln Gln
225                 230                 235                 240

Asp Gly Glu Gly His Thr Gln Asp Thr Glu Leu Val Glu Thr Arg Pro
                245                 250                 255

Ala Gly Asp Gly Thr Phe Gln Lys Trp Ala Ala Val Val Val Pro Ser
            260                 265                 270

Gly Glu Glu Gln Arg Tyr Thr Cys His Val Gln His Glu Gly Leu Pro
        275                 280                 285

Glu Pro Val Thr Leu Arg Trp Lys Pro Ala Ser Gln Pro Thr Ile Pro
    290                 295                 300

Ile Val Gly Ile Ile Ala Gly Leu Val Leu Leu Gly Ser Val Val Ser
305                 310                 315                 320

Gly Ala Val Val Ala Ala Val Ile Trp Arg Lys Lys Ser Ser Gly Gly
                325                 330                 335

Lys Gly Gly Ser Tyr Ser Lys Ala Glu Trp Ser Asp Ser Ala Gln Gly
            340                 345                 350

Ser Glu Ser His Ser Leu
        355

<210>  4
<211>  2679
<212>  DNA
<213>  Homo sapiens

<220>
<221>  misc_feature
<223>  Major histocompatibility complex, class I, E, E*0103 (CDS for
       amino acids 127-1203)

<400>  4
gcagactcag ttctcattcc caatgggtgt cgggtttcta gagaagccaa tcagcgtcgc      60

cacgactccc gactataaag tccccatccg gactcaagaa gttctcagga ctcagaggct     120

gggatcatgg tagatggaac cctcctttta ctcctctcgg aggccctggc ccttacccag 180

acctgggcgg gctcccactc cttgaagtat ttccacactt ccgtgtcccg gcccggccgc 240

ggggagcccc gcttcatctc tgtgggctac gtggacgaca cccagttcgt gcgcttcgac 300

aacgacgccg cgagtccgag gatggtgccg cgggcgccgt ggatggagca ggaggggtca 360

gagtattggg accgggagac acggagcgcc agggacaccg cacagatttt ccgagtgaat 420

ctgcggacgc tgcgcggcta ctacaatcag agcgaggccg ggtctcacac cctgcagtgg 480

atgcatggct gcgagctggg gcccgacggg cgcttcctcc gcgggtatga acagttcgcc 540

tacgacggca aggattatct caccctgaat gaggacctgc gctcctggac cgcggtggac 600

acggcggctc agatctccga gcaaaagtca aatgatgcct ctgaggcgga gcaccagaga 660

gcctacctgg aagacacatg cgtggagtgg ctccacaaat acctggagaa ggggaaggag 720

acgctgcttc acctggagcc cccaaagaca cacgtgactc accaccccat ctctgaccat 780

gaggccaccc tgaggtgctg ggccctgggc ttctaccctg cggagatcac actgacctgg 840

cagcaggatg gggagggcca tacccaggac acggagctcg tggagaccag gcctgcaggg 900

gatggaacct tccagaagtg ggcagctgtg gtggtgcctt ctggagagga gcagagatac 960

acgtgccatg tgcagcatga ggggctaccc gagcccgtca ccctgagatg gaagccggct 1020

tcccagccca ccatccccat cgtgggcatc attgctggcc tggttctcct tggatctgtg 1080

gtctctggag ctgtggttgc tgctgtgata tggaggaaga agagctcagg tggaaaagga 1140

gggagctact ctaaggctga gtggagcgac agtgcccagg ggtctgagtc tcacagcttg 1200

taaagcctga gacagctgcc ttgtgtgcga ctgagatgca cagctgcctt gtgtgcgact 1260

gagatgcagg atttcctcac gcctccccta tgtgtcttag gggactctgg cttctctttt 1320

tgcaagggcc tctgaatctg tctgtgtccc tgttagcaca atgtgaggag gtagagaaac 1380

agtccacctc tgtgtctacc atgaccccct tcctcacact gacctgtgtt ccttccctgt 1440

tctcttttct attaaaaata agaacctggg cagagtgcgg cagctcatgc ctgtaatccc 1500

agcacttagg gaggccgagg agggcagatc acgaggtcag gagatcgaaa ccatcctggc 1560

taacacggtg aaaccccgtc tctactaaaa aatacaaaaa attagctggg cgcagaggca 1620

cgggcctgta gtcccagcta ctcaggaggc ggaggcagga gaatggcgtc aacccgggag 1680

gcggaggttg cagtgagcca ggattgtgcg actgcactcc agcctgggtg acagggtgaa 1740

acgccatctc aaaaaataaa aattgaaaaa taaaaaaaga acctggatct caatttaatt 1800

tttcatattc ttgcaatgaa atggacttga ggaagctaag atcatagcta gaaatacaga 1860

taattccaca gcacatctct agcaaattta gcctattcct attctctagc ctattcctta 1920

ccacctgtaa tcttgaccat ataccttgga gttgaatatt gttttcatac tgctgtggtt 1980

tgaatgttcc ctccaacact catgttgaga cttaatccct aatgtggcaa tactgaaagg 2040

tggggccttt gagatgtgat tggatcgtaa ggctgtgcct tcattcatgg gttaatggat 2100

taatgggtta tcacaggaat gggactggtg gctttataag aagaggaaaa gagaactgag 2160

ctagcatgcc cagcccacag agagcctcca ctagagtgat gctaagtgga aatgtgaggt 2220

gcagctgcca cagagggccc ccaccaggga aatgtctagt gtctagtgga tccaggccac 2280

aggagagagt gccttgtgga gcgctgggag caggacctga ccaccaccag gaccccagaa 2340

ctgtggagtc agtggcagca tgcagcgccc ccttgggaaa gctttaggca ccagcctgca 2400

acccattcga gcagccacgt aggctgcacc cagcaaagcc acaggcacgg ggctacctga 2460

ggccttgggg gcccaatccc tgctccagtg tgtccgtgag gcagcacacg aagtcaaaag 2520

agattattct cttcccacag ataccttttc tctcccatga ccctttaaca gcatctgctt 2580

cattcccctc accttcccag gctgatctga ggtaaacttt gaagtaaaat aaaagctgtg 2640

tttgagcatc atttgtattt caaaaaaaaa aaaaaaaaa 2679

<210> 5
<211> 9
<212> PRT
<213> Artificial Sequence

<220>
<223> leader sequence (residues 3-11 of A0101, A0301, A3601, and
Cw1502)

<400> 5

Val Met Ala Pro Arg Thr Leu Leu Leu
1               5

<210> 6
<211> 9
<212> PRT
<213> Artificial Sequence

<220>
<223> leader sequence (residues 3-11 of A0201, A0211, A2403, and
A2501)

<400> 6

Val Met Ala Pro Arg Thr Leu Val Leu
1               5

<210> 7
<211> 9
<212> PRT
<213> Artificial Sequence

<220>
<223> leader sequence (residues 3-11 of A0702, B06501, and A0801)

<400>  7

Val Met Ala Pro Arg Thr Val Leu Leu
1               5


<210>  8
<211>  9
<212>  PRT
<213>  Artificial Sequence

<220>
<223>  leader sequence (Residues 3-11 of G)

<400>  8

Val Met Ala Pro Arg Thr Leu Phe Leu
1               5


<210>  9
<211>  9
<212>  PRT
<213>  Artificial Sequence

<220>
<223>  leader sequence (residues 3-11 of Cw0401)

<400>  9

Val Met Ala Pro Arg Thr Leu Ile Leu
1               5


<210>  10
<211>  20
<212>  DNA
<213>  Artificial Sequence

<220>
<223>  HLA-C CRISPR guide RNA 1

<400>  10
agcgacgccg cgagtccgag                                                  20


<210>  11
<211>  20
<212>  DNA
<213>  Artificial Sequence

<220>
<223>  HLA-C CRISPR guide RNA 2

<400>  11
ggtcgcagcc agacatcctc                                                  20


<210>  12
<211>  20
<212>  DNA
<213>  Artificial Sequence

<220>
<223>  HLA-C CRISPR guide RNA 3

<400>  12
gacacagaag tacaagcgcc                                                   20


<210>  13
<211>  20
<212>  DNA
<213>  Artificial Sequence

<220>
<223>  HLA-C CRISPR guide RNA 4

<400>  13
tcaccgctat gatgtgtagg                                                   20


<210>  14
<211>  20
<212>  DNA
<213>  Artificial Sequence

<220>
<223>  HLA-C CRISPR guide RNA 5

<400>  14
ctctcagctg ctccgccgca                                                   20


<210>  15
<211>  20
<212>  DNA
<213>  Artificial Sequence

<220>
<223>  HLA-C CRISPR guide RNA 6

<400>  15
cttcctccta cacatcatag                                                   20


<210>  16
<211>  20
<212>  DNA
<213>  Artificial Sequence

<220>
<223>  HLA-A CRISPR guide RNA 1

<400>  16
cgtcctgccg gtacccgcgg                                                   20


<210>  17
<211>  20
<212>  DNA
<213>  Artificial Sequence

<220>
<223>  HLA-A CRISPR guide RNA 2

<400>  17
taccggcagg acgcctacga                                                   20


<210>  18
<211>  20
<212>  DNA
<213>  Artificial Sequence

<220>
<223>  HLA-A CRISPR guide RNA 3

<400>  18
acagcgacgc cgcgagccag                                                   20


<210>  19
<211>  20
<212>  DNA
<213>  Artificial Sequence

<220>
<223>  HLA-A CRISPR guide RNA 4

<400>  19
ccagtcacag actgaccgag                                                   20


<210>  20
<211>  20
<212>  DNA
<213>  Artificial Sequence

<220>
<223>  HLA-A CRISPR guide RNA 5

<400>  20
tccctcctta ccccatctca                                                   20


<210>  21
<211>  20
<212>  DNA
<213>  Artificial Sequence

<220>
<223>  HLA-A CRISPR guide RNA 6

<400>  21
ccaccccatc tctgaccatg                                                   20


<210>  22
<211>  20
<212>  DNA
<213>  Artificial Sequence

<220>
<223>  HLA-B CRISPR guide RNA 1

<400>  22
cgtcgcagcc gtacatgctc                                                   20

<210>  23
<211>  20
<212>  DNA
<213>  Artificial Sequence

<220>
<223>  HLA-B CRISPR guide RNA 2

<400>  23
cgctgtcgaa cctcacgaac                                                   20


<210>  24
<211>  20
<212>  DNA
<213>  Artificial Sequence

<220>
<223>  HLA-B CRISPR guide RNA 3

<400>  24
ggatggcgag gaccaaactc                                                   20


<210>  25
<211>  20
<212>  DNA
<213>  Artificial Sequence

<220>
<223>  HLA-B CRISPR guide RNA 4

<400>  25
cctggctgtc ctagcagttg                                                   20


<210>  26
<211>  20
<212>  DNA
<213>  Artificial Sequence

<220>
<223>  HLA-B CRISPR guide RNA 5

<400>  26
cgtactggtc atgcccgcgg                                                   20


<210>  27
<211>  20
<212>  DNA
<213>  Artificial Sequence

<220>
<223>  HLA-B CRISPR guide RNA 6

<400>  27
ctccgatgac cacaactgct                                                   20


<210>  28
<211>  20

<212>  DNA
<213>  Artificial Sequence

<220>
<223>  A2-1 siRNA

<400>  28
ggattacatc gccctgaaag                                                    20


<210>  29
<211>  20
<212>  DNA
<213>  Artificial Sequence

<220>
<223>  A2-2 siRNA

<400>  29
gcaggagggt ccggagtatt                                                    20


<210>  30
<211>  20
<212>  DNA
<213>  Artificial Sequence

<220>
<223>  A2-3 siRNA

<400>  30
ggacggggag acacggaaag                                                    20


<210>  31
<211>  19
<212>  DNA
<213>  Artificial Sequence

<220>
<223>  A2-4 siRNA

<400>  31
gaaagtgaag gcccactca                                                     19


<210>  32
<211>  20
<212>  DNA
<213>  Artificial Sequence

<220>
<223>  ABC-1 siRNA

<400>  32
gatacctgga gaacgggaag                                                    20


<210>  33
<211>  20
<212>  DNA
<213>  Artificial Sequence

<220>
<223>  ABC-2 siRNA

<400>  33
gctgtggtgg tgccttctgg                                                    20


<210>  34
<211>  20
<212>  DNA
<213>  Artificial Sequence

<220>
<223>  ABC-3 siRNA

<400>  34
gctactacaa ccagagcgag                                                    20


<210>  35
<211>  19
<212>  DNA
<213>  Artificial Sequence

<220>
<223>  ABC-4 siRNA

<400>  35
gtggctccgc agatacctg                                                     19


<210>  36
<211>  54
<212>  RNA
<213>  Artificial Sequence

<220>
<223>  HLA-A-specific shRNA

<400>  36
caccugccau gugcaugauu uguguaguca ugcugcacau ggcagguguu uuuu              54


<210>  37
<211>  57
<212>  RNA
<213>  Artificial Sequence

<220>
<223>  HLA ABC-specific shRNA

<400>  37
ggagaucaca cugaccuggc auuuguguag ugccagguca gugugaucuc cuuuuuu           57


<210>  38
<211>  12
<212>  PRT
<213>  Artificial Sequence

<220>
<223>  spacer (human IgG4hinge) (aa)

<400>  38

Glu Ser Lys Tyr Gly Pro Pro Cys Pro Pro Cys Pro
1               5                   10


<210>  39
<211>  36
<212>  DNA
<213>  Artificial Sequence

<220>
<223>  spacer (human IgG4hinge) (nt)

<400>  39
gaatctaagt acggaccgcc ctgcccccct tgccct                                36


<210>  40
<211>  119
<212>  PRT
<213>  Artificial Sequence

<220>
<223>  Hinge-CH3 spacer (Homo sapiens)

<400>  40

Glu Ser Lys Tyr Gly Pro Pro Cys Pro Pro Cys Pro Gly Gln Pro Arg
1               5                   10                  15


Glu Pro Gln Val Tyr Thr Leu Pro Pro Ser Gln Glu Glu Met Thr Lys
            20                  25                  30


Asn Gln Val Ser Leu Thr Cys Leu Val Lys Gly Phe Tyr Pro Ser Asp
        35                  40                  45


Ile Ala Val Glu Trp Glu Ser Asn Gly Gln Pro Glu Asn Asn Tyr Lys
    50                  55                  60


Thr Thr Pro Pro Val Leu Asp Ser Asp Gly Ser Phe Phe Leu Tyr Ser
65                  70                  75                  80


Arg Leu Thr Val Asp Lys Ser Arg Trp Gln Glu Gly Asn Val Phe Ser
                85                  90                  95


Cys Ser Val Met His Glu Ala Leu His Asn His Tyr Thr Gln Lys Ser
            100                 105                 110


Leu Ser Leu Ser Leu Gly Lys
        115


<210>  41
<211>  229
<212>  PRT

<213>  Artificial Sequence

<220>
<223>  Hinge-CH2-CH3 spacer (Homo sapiens)

<400>  41

Glu Ser Lys Tyr Gly Pro Pro Cys Pro Pro Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210> 42
<211> 282
<212> PRT
<213> Artificial Sequence

<220>
<223> IgD-hinge-Fc (Homo sapiens)

<400> 42

Arg Trp Pro Glu Ser Pro Lys Ala Gln Ala Ser Ser Val Pro Thr Ala
1               5                   10                  15

Gln Pro Gln Ala Glu Gly Ser Leu Ala Lys Ala Thr Thr Ala Pro Ala
            20                  25                  30

Thr Thr Arg Asn Thr Gly Arg Gly Gly Glu Glu Lys Lys Lys Glu Lys
        35                  40                  45

Glu Lys Glu Glu Gln Glu Glu Arg Glu Thr Lys Thr Pro Glu Cys Pro
    50                  55                  60

Ser His Thr Gln Pro Leu Gly Val Tyr Leu Leu Thr Pro Ala Val Gln
65                  70                  75                  80

Asp Leu Trp Leu Arg Asp Lys Ala Thr Phe Thr Cys Phe Val Val Gly
                85                  90                  95

Ser Asp Leu Lys Asp Ala His Leu Thr Trp Glu Val Ala Gly Lys Val
            100                 105                 110

Pro Thr Gly Gly Val Glu Glu Gly Leu Leu Glu Arg His Ser Asn Gly
        115                 120                 125

Ser Gln Ser Gln His Ser Arg Leu Thr Leu Pro Arg Ser Leu Trp Asn
    130                 135                 140

Ala Gly Thr Ser Val Thr Cys Thr Leu Asn His Pro Ser Leu Pro Pro
145                 150                 155                 160

Gln Arg Leu Met Ala Leu Arg Glu Pro Ala Ala Gln Ala Pro Val Lys
                165                 170                 175

Leu Ser Leu Asn Leu Leu Ala Ser Ser Asp Pro Pro Glu Ala Ala Ser
            180                 185                 190

Trp Leu Leu Cys Glu Val Ser Gly Phe Ser Pro Pro Asn Ile Leu Leu

195                 200                 205

Met Trp Leu Glu Asp Gln Arg Glu Val Asn Thr Ser Gly Phe Ala Pro
    210                 215                 220

Ala Arg Pro Pro Pro Gln Pro Gly Ser Thr Thr Phe Trp Ala Trp Ser
225                 230                 235                 240

Val Leu Arg Val Pro Ala Pro Pro Ser Pro Gln Pro Ala Thr Tyr Thr
                245                 250                 255

Cys Val Val Ser His Glu Asp Ser Arg Thr Leu Leu Asn Ala Ser Arg
            260                 265                 270

Ser Leu Glu Val Ser Tyr Val Thr Asp His
        275                 280

<210>  43
<211>  357
<212>  PRT
<213>  Artificial Sequence

<220>
<223>  tEGFR

<400>  43

Met Leu Leu Leu Val Thr Ser Leu Leu Leu Cys Glu Leu Pro His Pro
1               5                   10                  15

Ala Phe Leu Leu Ile Pro Arg Lys Val Cys Asn Gly Ile Gly Ile Gly
            20                  25                  30

Glu Phe Lys Asp Ser Leu Ser Ile Asn Ala Thr Asn Ile Lys His Phe
        35                  40                  45

Lys Asn Cys Thr Ser Ile Ser Gly Asp Leu His Ile Leu Pro Val Ala
    50                  55                  60

Phe Arg Gly Asp Ser Phe Thr His Thr Pro Pro Leu Asp Pro Gln Glu
65                  70                  75                  80

Leu Asp Ile Leu Lys Thr Val Lys Glu Ile Thr Gly Phe Leu Leu Ile
                85                  90                  95

Gln Ala Trp Pro Glu Asn Arg Thr Asp Leu His Ala Phe Glu Asn Leu
            100                 105                 110

Glu Ile Ile Arg Gly Arg Thr Lys Gln His Gly Gln Phe Ser Leu Ala
        115                 120                 125

Val Val Ser Leu Asn Ile Thr Ser Leu Gly Leu Arg Ser Leu Lys Glu
    130                 135                 140

Ile Ser Asp Gly Asp Val Ile Ile Ser Gly Asn Lys Asn Leu Cys Tyr
145                 150                 155                 160

Ala Asn Thr Ile Asn Trp Lys Lys Leu Phe Gly Thr Ser Gly Gln Lys
                165                 170                 175

Thr Lys Ile Ile Ser Asn Arg Gly Glu Asn Ser Cys Lys Ala Thr Gly
            180                 185                 190

Gln Val Cys His Ala Leu Cys Ser Pro Glu Gly Cys Trp Gly Pro Glu
        195                 200                 205

Pro Arg Asp Cys Val Ser Cys Arg Asn Val Ser Arg Gly Arg Glu Cys
    210                 215                 220

Val Asp Lys Cys Asn Leu Leu Glu Gly Glu Pro Arg Glu Phe Val Glu
225                 230                 235                 240

Asn Ser Glu Cys Ile Gln Cys His Pro Glu Cys Leu Pro Gln Ala Met
                245                 250                 255

Asn Ile Thr Cys Thr Gly Arg Gly Pro Asp Asn Cys Ile Gln Cys Ala
            260                 265                 270

His Tyr Ile Asp Gly Pro His Cys Val Lys Thr Cys Pro Ala Gly Val
        275                 280                 285

Met Gly Glu Asn Asn Thr Leu Val Trp Lys Tyr Ala Asp Ala Gly His
    290                 295                 300

Val Cys His Leu Cys His Pro Asn Cys Thr Tyr Gly Cys Thr Gly Pro
305                 310                 315                 320

Gly Leu Glu Gly Cys Pro Thr Asn Gly Pro Lys Ile Pro Ser Ile Ala
                325                 330                 335

Thr Gly Met Val Gly Ala Leu Leu Leu Leu Leu Val Val Ala Leu Gly
            340                 345                 350

Ile Gly Leu Phe Met
        355

<210>  44

<211>  24
<212>  PRT
<213>  Artificial Sequence

<220>
<223>  T2A

<400>  44

Leu Glu Gly Gly Gly Glu Gly Arg Gly Ser Leu Leu Thr Cys Gly Asp
1               5                   10                  15

Val Glu Glu Asn Pro Gly Pro Arg
            20

<210>  45
<211>  27
<212>  PRT
<213>  Homo sapiens

<220>
<221>  misc_feature
<223>  CD28 (amino acids amino acids 153-179 of Accession No. P10747)

<400>  45

Phe Trp Val Leu Val Val Val Gly Gly Val Leu Ala Cys Tyr Ser Leu
1               5                   10                  15

Leu Val Thr Val Ala Phe Ile Ile Phe Trp Val
            20                  25

<210>  46
<211>  66
<212>  PRT
<213>  Homo sapiens

<220>
<221>  misc_feature
<223>  CD28 (amino acids 114-179 of Accession No. P10747)

<400>  46

Ile Glu Val Met Tyr Pro Pro Pro Tyr Leu Asp Asn Glu Lys Ser Asn
1               5                   10                  15

Gly Thr Ile Ile His Val Lys Gly Lys His Leu Cys Pro Ser Pro Leu
            20                  25                  30

Phe Pro Gly Pro Ser Lys Pro Phe Trp Val Leu Val Val Val Gly Gly
        35                  40                  45

Val Leu Ala Cys Tyr Ser Leu Leu Val Thr Val Ala Phe Ile Ile Phe
    50                  55                  60

Trp Val
65

<210> 47
<211> 41
<212> PRT
<213> Homo sapiens

<220>
<221> misc_feature
<223> CD28 (amino acids 180-220 of P10747)

<400> 47

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210> 48
<211> 41
<212> PRT
<213> Homo sapiens

<220>
<221> misc_feature
<223> CD28 (LL to GG)

<400> 48

Arg Ser Lys Arg Ser Arg Gly Gly His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210> 49
<211> 42
<212> PRT
<213> Homo sapiens

<220>
<221> misc_feature
<223> 4-1BB (amino acids 214-255 of Q07011.1)

<400>  49

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210>  50
<211>  112
<212>  PRT
<213>  Homo sapiens

<220>
<221>  misc_feature
<223>  CD3 zeta

<400>  50

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  51
<211>  112
<212>  PRT
<213>  Homo sapiens

<220>
<221>  misc_feature

<223>  CD3 zeta

<400>  51

Arg Val Lys Phe Ser Arg Ser Ala Glu Pro Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  52
<211>  112
<212>  PRT
<213>  Homo sapiens

<220>
<221>  misc_feature
<223>  CD3 zeta

<400>  52

Arg Val Lys Phe Ser Arg Ser Ala Asp Ala Pro Ala Tyr Lys Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  53
<211>  17
<212>  PRT
<213>  Artificial Sequence

<220>
<223>  Linker 2

<400>  53

Gly Ser Ala Asp Asp Ala Lys Lys Asp Ala Ala Lys Lys Asp Gly Lys
1               5                   10                  15

Ser

<210>  54
<211>  30
<212>  PRT
<213>  Artificial Sequence

<220>
<223>  Linker 1

<400>  54

Pro Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Pro
            20                  25                  30

<210>  55
<211>  35
<212>  PRT
<213>  Artificial Sequence

<220>
<223>  Linker

<400>  55

Pro Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

Gly Gly Pro
        35

<210>  56
<211>  18
<212>  PRT
<213>  Artificial Sequence

<220>
<223>  T2A

<400>  56

Glu Gly Arg Gly Ser Leu Leu Thr Cys Gly Asp Val Glu Glu Asn Pro
1               5                   10                  15

Gly Pro

<210>  57
<211>  22
<212>  PRT
<213>  Artificial Sequence

<220>
<223>  P2A

<400>  57

Gly Ser Gly Ala Thr Asn Phe Ser Leu Leu Lys Gln Ala Gly Asp Val
1               5                   10                  15

Glu Glu Asn Pro Gly Pro
            20

<210>  58
<211>  19
<212>  PRT
<213>  Artificial Sequence

<220>
<223>  P2A

<400>  58

Ala Thr Asn Phe Ser Leu Leu Lys Gln Ala Gly Asp Val Glu Glu Asn
1               5                   10                  15

Pro Gly Pro

<210>  59
<211>  20
<212>  PRT
<213>  Artificial Sequence

<220>
<223>  E2A

<400>  59

Gln Cys Thr Asn Tyr Ala Leu Leu Lys Leu Ala Gly Asp Val Glu Ser
1               5                   10                  15

Asn Pro Gly Pro
            20

<210>  60
<211>  22
<212>  PRT
<213>  Artificial Sequence

<220>
<223>  F2A

<400>  60

Val Lys Gln Thr Leu Asn Phe Asp Leu Leu Lys Leu Ala Gly Asp Val
1               5                   10                  15

Glu Ser Asn Pro Gly Pro
            20

<210>  61
<211>  335
<212>  PRT
<213>  Artificial Sequence

<220>
<223>  tEGFR

<400>  61

Arg Lys Val Cys Asn Gly Ile Gly Ile Gly Glu Phe Lys Asp Ser Leu
1               5                   10                  15

Ser Ile Asn Ala Thr Asn Ile Lys His Phe Lys Asn Cys Thr Ser Ile
            20                  25                  30

Ser Gly Asp Leu His Ile Leu Pro Val Ala Phe Arg Gly Asp Ser Phe
        35                  40                  45

Thr His Thr Pro Pro Leu Asp Pro Gln Glu Leu Asp Ile Leu Lys Thr
    50                  55                  60

Val Lys Glu Ile Thr Gly Phe Leu Leu Ile Gln Ala Trp Pro Glu Asn
65                  70                  75                  80

Arg Thr Asp Leu His Ala Phe Glu Asn Leu Glu Ile Ile Arg Gly Arg
                85                  90                  95

Thr Lys Gln His Gly Gln Phe Ser Leu Ala Val Val Ser Leu Asn Ile
            100                 105                 110

Thr Ser Leu Gly Leu Arg Ser Leu Lys Glu Ile Ser Asp Gly Asp Val
        115                 120                 125

Ile Ile Ser Gly Asn Lys Asn Leu Cys Tyr Ala Asn Thr Ile Asn Trp
    130                 135                 140

Lys Lys Leu Phe Gly Thr Ser Gly Gln Lys Thr Lys Ile Ile Ser Asn
145                 150                 155                 160

Arg Gly Glu Asn Ser Cys Lys Ala Thr Gly Gln Val Cys His Ala Leu
                165                 170                 175

Cys Ser Pro Glu Gly Cys Trp Gly Pro Glu Pro Arg Asp Cys Val Ser
            180                 185                 190

Cys Arg Asn Val Ser Arg Gly Arg Glu Cys Val Asp Lys Cys Asn Leu
        195                 200                 205

Leu Glu Gly Glu Pro Arg Glu Phe Val Glu Asn Ser Glu Cys Ile Gln
    210                 215                 220

Cys His Pro Glu Cys Leu Pro Gln Ala Met Asn Ile Thr Cys Thr Gly
225                 230                 235                 240

Arg Gly Pro Asp Asn Cys Ile Gln Cys Ala His Tyr Ile Asp Gly Pro
                245                 250                 255

His Cys Val Lys Thr Cys Pro Ala Gly Val Met Gly Glu Asn Asn Thr
            260                 265                 270

Leu Val Trp Lys Tyr Ala Asp Ala Gly His Val Cys His Leu Cys His
        275                 280                 285

Pro Asn Cys Thr Tyr Gly Cys Thr Gly Pro Gly Leu Glu Gly Cys Pro
    290                 295                 300

Thr Asn Gly Pro Lys Ile Pro Ser Ile Ala Thr Gly Met Val Gly Ala
305                 310                 315                 320

Leu Leu Leu Leu Leu Val Val Ala Leu Gly Ile Gly Leu Phe Met
                325                 330                 335